# EUROPEAN PATENT SPECIFICATION

(11) **EP 3 931 187 B1**
(45) Date of publication and mention of the grant of the patent: **18.10.2023**
(21) Application number: 20708470.8
(22) Date of filing: 28.02.2020
(51) Int. Cl.: C07D 401/04, C07D 213/86, A01N 43/54, C07C 59/147

(54) **PESTICIDALLY ACTIVE HETEROCYCLIC DERIVATIVES WITH SULFUR CONTAINING SUBSTITUENTS**
PESTIZIDWIRKSAME HETEROCYCLISCHE DERIVATE MIT SCHWEFELHALTIGEN SUBSTITUENTEN
DÉRIVÉS HÉTÉROCYCLIQUES ACTIFS SUR LE PLAN PESTICIDE AVEC DES SUBSTITUANTS CONTENANT DU SOUFRE

(30) Priority: 28.02.2019 IN 201911007909; 19.06.2019 EP 19181188
(43) Date of publication of application: 05.01.2022
(73) Proprietor: Syngenta Crop Protection AG, 4058 Basel (CH)
(72) Inventor: SIKERVAR, Vikas, Ilhas Goa 403 110 (IN); SEN, Indira, Ilhas Goa 403110 (IN); MUEHLEBACH, Michel, 4332 Stein (CH); RENDLER, Sebastian, 4058 Basel (CH); STOLLER, André, 4332 Stein (CH); EMERY, Daniel, 4332 Stein (CH); BUCHHOLZ, Anke, 4332 Stein (CH)
(86) International application number: PCT/EP2020/055347
(87) International publication number: WO 2020/174096

(56) References cited:
- EP-A1- 3 202 762
- WO-A1-2018/008727
- DATABASE REGISTRY [Online] CHEMICAL ABSTRACTS SERVICE, COLUMBUS, OHIO, US; 31 December 2013 (2013-12-31), XP055680993, Database accession no. 1507771-62-1
- DATABASE REGISTRY [Online] CHEMICAL ABSTRACTS SERVICE, COLUMBUS, OHIO, US; 20 December 2013 (2013-12-20), XP055680999, Database accession no. 1499347-09-9
- DATABASE REGISTRY [Online] CHEMICAL ABSTRACTS SERVICE, COLUMBUS, OHIO, US; 10 February 2014 (2014-02-10), XP055681018, Database accession no. 1540338-03-1

## Description

The present invention relates to pesticidally active, in particular insecticidally active heterocyclic derivatives containing sulfur substituents, to processes for their preparation, to compositions comprising those compounds, and to their use for controlling animal pests, including arthropods and in particular insects or representatives of the order *Acarina.*

Some pyrimidinone compounds with insecticidal action are known and described, for example, in WO 2016/052247, WO 2017/090654, WO 2018/008727 and JP 2018/076354. Other pyrimidinone compounds with herbicidal action were disclosed, for example, in US 6107485, EP 663396 and US 5300477. Yet different pyrimidinones are known from WO 2005/060654 as mitotic kinesin inhibitors useful for the treatment of cancer.

It has now surprisingly been found that certain novel pesticidally active pyrimidinone derivatives, featuring an N-alkyl group in position 3, a haloalkoxy or haloalkenyloxy substitution in position 5, no substituent in position 6, and with sulfur containing substitutents in radical Q have favourable properties as pesticides.

The present invention therefore provides compounds of formula I, 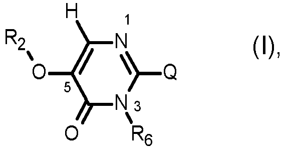 wherein
R₂ is C₁-C₆haloalkyl, C₂-C₆haloalkenyl or C₁-C₄haloalkylsulfonyl;
R₆ is C₁-C₆alkyl;
Q is a radical selected from the group consisting of formula Qa and Qb 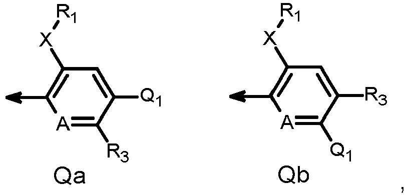
wherein the arrow denotes the point of attachment to the pyrimidinone ring;
and wherein A represents CH or N;
X is S, SO, SOz or SO(NH);
R₁ is C₁-C₄alkyl or C₃-C₆cycloalkyl-C₁-C₄alkyl;
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆cyanoalkoxy, C₁-C₆haloalkoxy, -N(R₄)₂, -N(R₄)COR₅, -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono- or polysubstituted by substituents selected from the group consisting of halogen, cyano, C₁-C₄alkyl, C₁-C₄haloalkyl, C₁-C₄alkoxy, C₁-C₄haloalkoxy, C₁-C₄alkylsulfanyl, C₁-C₄alkylsulfinyl and C₁-C₄alkylsulfonyl; and said ring system can contain 1, 2 or 3 ring heteroatoms selected from the group consisting of nitrogen, oxygen and sulphur, where said ring system may not contain more than one ring oxygen atom and not more than one ring sulfur atom; or
Q₁ is a five-membered aromatic ring system linked via a ring nitrogen atom to the ring which contains the substituent A, said ring system is unsubstituted or is mono- or polysubstituted by substituents selected from the group consisting of halogen, cyano, C₁-C₄alkyl, C₁-C₄haloalkyl, C₁-C₄alkoxy, C₁-C₄haloalkoxy, C₁-C₄alkylsulfanyl, C₁-C₄alkylsulfinyl and C₁-C₄alkylsulfonyl; and said ring system contains 1, 2 or 3 ring heteroatoms selected from the group consisting of nitrogen, oxygen and sulphur, where said ring system contains at least one ring nitrogen atom and may not contain more than one ring oxygen atom and not more than one ring sulfur atom;
R₃ is hydrogen, halogen or C₁-C₄alkyl;
each R₄ is independently hydrogen, C₁-C₄alkyl or C₃-C₆cycloalkyl; and
R₅ is C₁-C₆alkyl, C₁-C₆haloalkyl or C₃-C₆cycloalkyl.

The present invention also provides agrochemically acceptable salts, stereoisomers, enantiomers, tautomers and N-oxides of the compounds of formula I.

Compounds of formula I which have at least one basic centre can form, for example, acid addition salts, for example with strong inorganic acids such as mineral acids, for example perchloric acid, sulfuric acid, nitric acid, nitrous acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds of formula I which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

In each case, the compounds of formula (I) according to the invention are in free form, in oxidized form as a N-oxide or in salt form, e.g. an agronomically usable salt form.

N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991.

The compounds of formula I according to the invention also include hydrates which may be formed during the salt formation.

Where substituents are indicated as being itself further substituted, this means that they carry one or more identical or different substituents, e.g. one to four substituents. Normally not more than three such optional substituents are present at the same time. Preferably not more than two such substituents are present at the same time (i.e. the group is substituted by one or two of the substituents indicated). Where the additional substituent group is a larger group, such as cycloalkyl or phenyl, it is most preferred that only one such optional substituent is present. Where a group is indicated as being substituted, e.g. alkyl, this includes those groups that are part of other groups, e.g. the alkyl in alkylthio.

The term "C₁-Cₙalkyl" as used herein refers to a saturated straight-chain or branched hydrocarbon radical attached via any of the carbon atoms having 1 to n carbon atoms, for example, any one of the radicals methyl, ethyl, n-propyl, 1-methylbutyl, 2-methylbutyl, 3-methylbutyl, 2, 2-dimethylpropyl, 1-ethylpropyl, n-hexyl, n-pentyl, 1, 1-dimethylpropyl, 1, 2-dimethylpropyl, 1- methylpentyl, 2-methylpentyl, 3-methylpentyl, 4-methylpentyl, 1, 1-dimethylbutyl, 1,2- dimethylbutyl, 1,3-dimethylbutyl, 2, 2-dimethylbutyl, 2, 3-dimethylbutyl, 3, 3-dimethylbutyl, 1-ethylbutyl, 2-ethylbutyl, 1,1, 2-trimethylpropyl, 1,2, 2-trimethylpropyl, 1-ethyl-1- methylpropyl, or 1-ethyl-2-methylpropyl.

The term "C₂-Cₙ-alkenyl" as used herein refers to a straight-chain or branched unsaturated alkyl radical, for example, vinyl, allyl, homoallyl, but-1-eneyl, and but-2-eneyl. Where appropriate, the alkeneyl chains can be of either the (E)- or (Z)-configuration.

The term "C₁-Cₙhaloalkyl" as used herein refers to a straight-chain or branched saturated alkyl radical attached via any of the carbon atoms having 1 to n carbon atoms (as mentioned above), where some or all of the hydrogen atoms in these radicals may be replaced by fluorine, chlorine, bromine and/or iodine, i.e., for example, any one of chloromethyl, dichloromethyl, trichloromethyl, fluoromethyl, difluoromethyl, trifluoromethyl, chlorofluoromethyl, dichlorofluoromethyl, chlorodifluoromethyl, 2-fluoroethyl, 2-chloroethyl, 2-bromoethyl, 2-iodoethyl, 2, 2-difluoroethyl, 2,2, 2-trifluoroethyl, 2-chloro-2-fluoroethyl, 2-chloro-2, 2-difluoroethyl, 2, 2-dichloro-2-fluoroethyl, 2,2, 2-trichloroethyl, pentafluoroethyl, 2-fluoropropyl, 3-fluoropropyl, 2,2- difluoropropyl, 2, 3-difluoropropyl, 2-chloropropyl, 3-chloropropyl, 2, 3-dichloropropyl, 2- bromopropyl, 3-bromopropyl, 3,3, 3-trifluoropropyl, 3,3, 3-trichloropropyl, 2,2, 3,3, 3-pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. Accordingly, a term "C₁-C₂-fluoroalkyl" would refer to a C₁-C₂-alkyl radical which carries 1,2, 3,4, or 5 fluorine atoms, for example, any one of difluoromethyl, trifluoromethyl, 1-fluoroethyl, 2-fluoroethyl, 2, 2-difluoroethyl, 2,2, 2-trifluoroethyl, 1,1, 2, 2-tetrafluoroethyl or penta- fluoroethyl.

The term "C₁-Cₙalkoxy" as used herein refers to a straight-chain or branched saturated alkyl radical having 1 to n carbon atoms (as mentioned above) which is attached via an oxygen atom, i.e., for example, any one of methoxy, ethoxy, n-propoxy, 1-methylethoxy, n-butoxy, 1-methylpropoxy, 2-methylpropoxy or 1, 1-dimethylethoxy.

The term "C₁-Cₙhaloalkoxy" as used herein refers to a C₁-Cₙalkoxy radical as mentioned above which is partially or fully substituted by fluorine, chlorine, bromine and/or iodine, i.e., for example, any one of chloromethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 2-fluoroethoxy, 2-chloroethoxy, 2-bromoethoxy, 2-iodoethoxy, 2, 2-difluoroethoxy, 2,2, 2-trifluoroethoxy, 2-chloro-2-fluoroethoxy, 2-chloro-2, 2-difluoroethoxy, 2, 2-dichloro-2-fluoroethoxy, 2,2, 2-trichloroethoxy, pentafluoroeth- oxy, 2-fluoropropoxy, 3-fluoropropoxy, 2, 2-difluoropropoxy, 2, 3-difluoropropoxy, 2-chloropropoxy, 3-chloropropoxy, 2, 3-dichloropropoxy, 2-bromopropoxy, 3- bromopropoxy, 3,3, 3-trifluoropropoxy, 3,3, 3-trichloropropoxy, 2,2, 3,3, 3- pentafluoropropoxy, heptafluoropropoxy, 1-(fluoromethyl)-2-fluoroethoxy, 1- (chloromethyl)-2-chloroethoxy, 1-(bromomethyl)-2-bromoethoxy, 4-fluorobutoxy, 4- chlorobutoxy, or 4-bromobutoxy.

The term "C₁-Cₙ-alkylsulfanyl" as used herein refers to a straight chain or branched saturated alkyl radical having 1 to n carbon atoms (as mentioned above) which is attached via a sulfur atom, i.e., for example, any one of methylthio, ethylthio, n-propylthio, 1-methylethylthio, butylthio, 1-methylpropylthio, 2- methylpropylthio or 1, 1-dimethylethylthio.

The term "C₁-Cₙalkylsulfinyl" as used herein refers to a straight chain or branched saturated alkyl radical having 1 to n carbon atoms (as mentioned above) which is attached *via* the sulfur atom of the sulfinyl group, i.e., for example, any one of methylsulfinyl, ethylsulfinyl, n-propylsulfinyl, 1-methylethyl-sulfinyl, n-butylsulfinyl, 1-methylpropylsulfinyl, 2-methylpropylsulfinyl, 1, 1-dimethyl-ethylsulfinyl, n-pentylsulfinyl, 1-methylbutylsulfinyl, 2-methylbutylsulfinyl, 3-methyl- butylsulfinyl, 1, 1-dimethylpropylsulfinyl, 1, 2-dimethylpropylsulfinyl, 2,2- dimethylpropylsulfinyl or 1-ethylpropylsulfinyl.

The term "C₁-Cₙalkylsulfonyl" as used herein refers to a straight chain or branched saturated alkyl radical having 1 to n carbon atoms (as mentioned above) which is attached *via* the sulfur atom of the sulfonyl group, i.e., for example, any one of methylsulfonyl, ethylsulfonyl, n-propylsulfonyl, isopropylsulfonyl, n-butylsulfonyl, 1-methylpropylsulfonyl, 2-methylpropylsulfonyl or t-butylsulphonyl.

The term "C₁-Cₙhaloalkylsulfanyl" as used herein refers to a C1-Cₙalkylthio radical as mentioned above which is partially or fully substituted by fluorine, chlorine, bromine and/or iodine, i.e., for example, any one of fluoromethylthio, difluoromethylthio, trifluoromethylthio, chlorodifluoromethylthio, bromodifluoromethylthio, 2-fluoroethylthio, 2-chloroethylthio, 2-bromoethylthio, 2-iodoethylthio, 2, 2-difluoroethylthio, 2,2,2-trifluoroethylthio, 2,2, 2-trichloroethylthio, 2-chloro-2-fluoroethylthio, 2-chloro-2,2-difluoroethylthio, 2, 2-dichloro-2-fluoroethylthio, pentafluoroethylthio, 2-fluoropropylthio, 3-fluoropropylthio, 2-chloropropylthio, 3-chloropropylthio, 2-bromopropylthio, 3-bromopropylthio, 2,2-difluoropropylthio, 2,3-difluoropropylthio, 2, 3-dichloropropylthio, 3,3, 3- trifluoropropylthio, 3,3, 3-trichloropropylthio, 2,2, 3,3, 3-pentafluoropropylthio, heptafluoropropylthio, 1- (fluoromethyl)-2-fluoroethylthio, 1- (chloromethyl)-2-chloroethylthio, 1- (bromomethyl)-2-bromoethylthio, 4-fluorobutylthio, 4-chlorobutylthio, or 4- bromobutylthio.

The term "C₁-Cₙhaloalkylsulfinyl" and "C₁-Cₙhaloalkylsulfonyl" refers to the groups above but with the sulfur in oxidations state 1 or 2 respectively.

The term "C₁-Cₙcyanoalkyl" as used herein refers to a straight chain or branched saturated alkyl radicals having 1 to n carbon atoms (as mentioned above) which is substituted by a cyano group, for example cyanomethylene, cyanoethylene, 1,1-dimethylcyanomethyl, cyanomethyl, cyanoethyl, and 1-dimethylcyanomethyl.

The term "C₁-Cₙcyanoalkoxy" refers to the groups above but which is attached *via* an oxygen atom.

The suffix "-C₁-Cₙalkyl" after terms such as "C₃-Cₙcycloalkyl", wherein n is an integer from 1-6, as used herein refers to a straight chain or branched saturated alkyl radicals which is substituted by C₃-Cₙcycloalkyl. An example of C₃-Cₙcycloalkyl-C₁-Cₙalkyl is for example, cyclopropylmethyl.

The term "C₃-C₆cycloalkyl" as used herein refers to 3-6 membered cycloylkyl groups such as cyclopropane, cyclobutane, cyclopropane, cyclopentane and cyclohexane.

Halogen is generally fluorine, chlorine, bromine or iodine. This also applies, correspondingly, to halogen in combination with other meanings, such as haloalkyl.

In the context of this invention "mono- or polysubstituted" in the definition of the Q₁ substituents, means typically, depending on the chemical structure of the substituents, monosubstituted to five-times substituted, more preferably mono-, double- or triple-substituted.

In the context of the this invention, the phrase "Q₁ is a five- to six-membered aromatic ring system, linked via a ring carbon atom ..." and the phrase "Q₁ is a five-membered aromatic ring system linked via a ring nitrogen atom ...", as the case may be, refer to the manner of attachment of particular embodmients of the substituent Q₁ to the radical Q (wherein Q also is substituted by X-R₁ and R₃ as described above) as represented by either formula Qa or formula Qb, which can be pyridyl or phenyl when A represents N or CH, respectively, as the case may be.

In the context of this invention, examples of "Q₁ is a five- to six-membered aromatic ring system, linked via a ring carbon atom ...; and said ring system can contain 1, 2 or 3 heteroatoms ..." are, but not limited to, phenyl, pyrazolyl, triazolyl, pyridinyl and pyrimidinyl; preferably phenyl, 2-pyridyl, 3-pyridyl, 4-pyridyl, pyrimidin-2-yl, pyrimidin-4-yl, and pyrimidin-5-yl..

In the context of this invention, examples of "Q₁ is a five-membered aromatic ring system linked via a ring nitrogen atom ...; and said ring system contains 1, 2 or 3 heteroatoms ..." are, but not limited to, pyrazolyl, pyrrolyl, imidazolyl and triazolyl; preferably pyrrol-1-yl, pyrazol-1-yl, triazol-2-yl, 1,2,4-triazol-1-yl, triazol-1-yl, and imidazol-1-yl.

Certain embodiments according to the invention are provided as set out below.

Embodiment 1 provides compounds of formula I, or an agrochemically acceptable salt, stereoisomer, enantiomer, tautomer or N-oxide thereof, as defined above.

Embodiment 2 provides compounds, or an agrochemically acceptable salt, stereoisomer, enantiomer, tautomer or N-oxide thereof, according to embodiment 1 wherein Q is Qa and having preferred values of R₂, A, X, R₁, Q₁, R₄, R₅, R₆ and R₃ as set out below.

Embodiment 3 provides compounds, or an agrochemically acceptable salt, stereoisomer, enantiomer, tautomer or N-oxide thereof, according to embodiment 1 wherein Q is Qb and having preferred values of R₂, A, X, R₁, Q₁, R₄, R₅, R₆ and R₃ as set out below.

With respect to embodiments 1 - 3, preferred values of R₂, A, X, R₁, Q₁, R₄, R₅, R₆ and R₃ are, in any combination thereof, as set out below:
Preferably R₂ is C₁-C₆haloalkyl or C₁-C₄haloalkylsulfonyl.

More preferably R₂ is C₁-C₆haloalkyl, even more preferably C₁-C₆fluoroalkyl.

Most preferably R₂ is -CH₂CF₂CHF₂ or -CH₂CF₂CF₃.

Preferably A is N.

Preferably X is S or SO₂.

Most preferably X is SOz.

Preferably R₁ is C₁-C₄alkyl or cyclopropyl-C₁-C₄alkyl.

More preferably R₁ is ethyl or cyclopropylmethyl.

Most preferably R₁ is ethyl.
Preferably Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the ring which contains the substituent A, said ring system is unsubstituted or is mono-substituted by substituents selected from the group consisting of halogen, cyano and C₁-C₄haloalkyl; and said ring system contains 2 or 3 ring nitrogen atoms.

More preferably Q₁ is hydrogen, halogen, trifluoromethyl, cyclopropyl, cyanocyclopropyl, cyanoisopropyl, trifluoroethoxy, -N(R₄)₂, -N(R₄)COR₅, or-N(R₄)CON(R₄)₂, in each of which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl.

Most preferably Q₁ is hydrogen, chlorine, bromine, trifluoromethyl, cyclopropyl, 1-cyanocyclopropyl, 1-cyano-1-methyl-ethyl, 2,2,2-trifluoroethoxy, -NH(CH₃), -N(CH₃)COCH₃, -N(CH₃)CO(cyclopropyl), - N(H)CONH(CH₃), -N(CH₃)CONH(CH₃), (oxazolidin-2-one)-3-yl, 2-pyridyloxy, pyrazol-1-yl, 3-chloro-pyrazol-1-yl, 3-cyano-pyrazol-1-yl, 3-trifluoromethyl-pyrazol-1-yl, 1 ,2,4-triazol-1-yl or pyrimidin-2-yl. Preferably each R₄ is independently hydrogen or C₁-C₄alkyl.

Most preferably each R₄ is independently hydrogen or methyl.

Preferably R₅ is C₁-C₆alkyl or C₃-C₆cycloalkyl.

More preferably R₅ is methyl or cyclopropyl.

Most preferably R₅ is methyl.

Preferably R₆ is C₁-C₃alkyl.

Most preferably R₆ is methyl.

Preferably R₃ is hydrogen or C₁-C₄alkyl.

More preferably R₃ is hydrogen or methyl.

Most preferably R₃ is hydrogen.

One group of compounds according to the invention are those of formula I-1 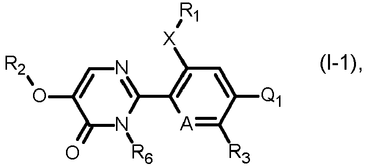
wherein A, X, R₁, R₂ and R₆ are as defined for compounds of formula I (above), or an agrochemically acceptable salt, stereoisomer, enantiomer, tautomer or N-oxide thereof, and wherein Q₁ is preferably hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the ring which contains the substituent A, said ring system is unsubstituted or is mono-substituted by substituents selected from the group consisting of halogen, cyano or C₁-C₄haloalkyl; and said ring system contains 2 or 3 ring nitrogen atoms;
R₃ is preferably hydrogen or C₁-C₄alkyl;
each R₄ is independently preferably hydrogen or C₁-C₄alkyl; and
R₅ is preferably C₁-C₆alkyl or C₃-C₆cycloalkyl.

Preferred definitions of A, X, R₁, R₂ and R₆ are as defined for compounds of formula I (above), and more preferably Q₁ is hydrogen, halogen, trifluoromethyl, cyclopropyl, cyanocyclopropyl, cyanoisopropyl, trifluoroethoxy, -N(R₄)₂, -N(R₄)COR₅, or -N(R₄)CON(R₄)₂, in which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl; and R₃ is hydrogen or methyl, preferably hydrogen.

One group of compounds according to this embodiment are compounds of formula (I-1a) which are compounds of formula (I-1) wherein A is N.

Another group of compounds according to this embodiment are compounds of formula (I-1b) which are compounds of formula (I-1) wherein A is CH.

Another group of compounds according to the invention are those of formula I-2 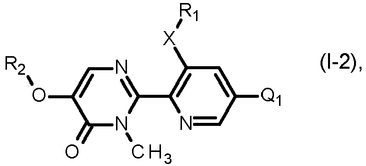
wherein X, R₁ and R₂ are as defined for compounds of formula I (above), or an agrochemically acceptable salt, stereoisomer, enantiomer, tautomer or N-oxide thereof, and wherein Q₁ is preferably hydrogen, halogen, C₁-C₆haloalkyl, C₃-C₆cycloalkyl, C₃-C₆cycloalkyl monosubstituted by cyano,
C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the pyridyl ring substituted by X-R₁, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the pyridyl ring substituted by X-R₁, said ring system is unsubstituted or is mono-substituted by substituents selected from the group consisting of halogen, cyano and C₁-C₄haloalkyl; and said ring system contains 2 or 3 ring nitrogen atoms;
each R₄ is independently preferably hydrogen or C₁-C₄alkyl; and
R₅ is preferably C₁-C₆alkyl or C₃-C₆cycloalkyl.

Preferred definitions of X, R₁ and R₂ are as defined for compounds of formula I (above), and more preferably Q₁ is hydrogen, halogen, trifluoromethyl, cyclopropyl, cyanocyclopropyl, cyanoisopropyl, trifluoroethoxy, -N(R₄)₂, -N(R₄)COR₅, or -N(R₄)CON(R₄)₂, in which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl.

One group of compounds according to this embodiment are compounds of formula (I-2a) which are compounds of formula (I-2) wherein X is S or SOz, preferably SOz.

Another group of compounds according to this embodiment are compounds of formula (I-2b-1) which are compounds of formula (I-2) wherein R₁ is C₁-C₄alkyl or cyclopropyl-C₁-C₄alkyl, preferably ethyl or cyclopropylmethyl.

Another group of compounds according to this embodiment are compounds of formula (I-2b-2) which are compounds of formula (I-2) wherein R₁ is C₁-C₄alkyl, preferably ethyl.

Another group of compounds according to this embodiment are compounds of formula (I-2c) which are compounds of formula (I-2) wherein R₂ is C₁-C₆haloalkyl, more preferably C₁-C₆fluoroalkyl, most preferably -CH₂CF₂CHF₂ or -CH₂CF₂CF₃.

Another group of compounds according to the invention are those of formula I-3 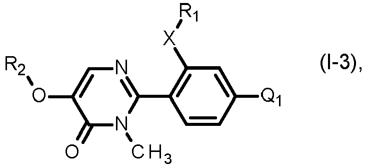
wherein X, R₁ and R₂ are as defined for compounds of formula I (above), or an agrochemically acceptable salt, stereoisomer, enantiomer, tautomer or N-oxide thereof, and wherein Q₁ is preferably hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the phenyl ring substituted by X-R₁, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the phenyl ring substituted by X-R₁, said ring system is unsubstituted or is mono-substituted by substituents selected from the group consisting of halogen, cyano and C₁-C₄haloalkyl; and said ring system contains 2 or 3 ring nitrogen atoms;
each R₄ is independently preferably hydrogen or C₁-C₄alkyl; and
R is preferably C₁-C₆alkyl or C₃-C₆cycloalkyl.

Preferred definitions of X, R₁ and R₂ are as defined for compounds of formula I (above), and more preferably Q₁ is hydrogen, halogen, trifluoromethyl, cyclopropyl, cyanocyclopropyl, cyanoisopropyl, trifluoroethoxy, -N(R₄)₂, -N(R₄)COR₅, or -N(R₄)CON(R₄)₂, in which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl.

One group of compounds according to this embodiment are compounds of formula (I-3a) which are compounds of formula (I-3) wherein X is S or SOz, preferably SOz.

Another group of compounds according to this embodiment are compounds of formula (I-3b-1) which are compounds of formula (I-3) wherein R₁ is C₁-C₄alkyl or cyclopropyl-C₁-C₄alkyl, preferably ethyl or cyclopropylmethyl.

Another group of compounds according to this embodiment are compounds of formula (I-3b-2) which are compounds of formula (I-3) wherein R₁ is C₁-C₄alkyl, preferably ethyl.

Another group of compounds according to this embodiment are compounds of formula (I-3c) which are compounds of formula (I-3) wherein R₂ is C₁-C₆haloalkyl, more preferably C₁-C₆fluoroalkyl, most preferably -CH₂CF₂CHF₂ or -CH₂CF₂CF₃.

Another group of compounds according to the invention are those of formula I-4 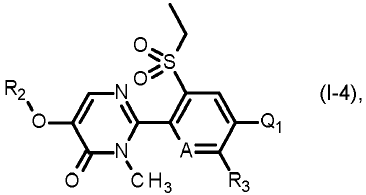 wherein
A is CH or N, preferably N;
R₂ is C₁-C₆haloalkyl, preferably -CH₂CF₂CHF₂ or-CH₂CF₂CF₃;
R₃ is hydrogen or C₁-C₄alkyl, preferably hydrogen or methyl;
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the ring which contains the substituent A, said ring system is unsubstituted or is mono-substituted by substituents selected from the group consisting of halogen, cyano and C₁-C₄haloalkyl; and said ring system contains 2 or 3 ring nitrogen atoms;
each R₄ is independently hydrogen or C₁-C₄alkyl, preferably hydrogen or methyl; and R₅ is C₁-C₆alkyl or C₃-C₆cycloalkyl, preferably methyl or cyclopropyl; or
an agrochemically acceptable salt, stereoisomer, enantiomer, tautomer or N-oxide thereof.

One further preferred group of compounds according to this embodiment are compounds of formula (I-4a) which are compounds of formula (I-4) wherein A is N.

Another preferred group of compounds according to this embodiment are compounds of formula (I-4b) which are compounds of formula (I-4) wherein A is CH.

One further preferred group of compounds according to this embodiment are compounds of formula (I-4c) which are compounds of formula (I-4) wherein R₃ is hydrogen.

Another preferred group of compounds according to this embodiment are compounds of formula (I-4d) which are compounds of formula (I-4) wherein R₃ is C₁-C₄alkyl, preferably methyl.

One further preferred group of compounds according to this embodiment are compounds of formula (I-4e) which are compounds of formula (I-4) wherein A is N and R₃ is hydrogen.

One further preferred group of compounds according to this embodiment are compounds of formula (I-4f) which are compounds of formula (I-4) wherein Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂ or-N(R₄)COR₅, both in which R₄ is independently hydrogen or C₁-C₄alkyl (preferably hydrogen or methyl) and R₅ is C₁-C₆alkyl (preferably methyl), or Q₁ is 2-pyridyloxy; preferably Q₁ is hydrogen, halogen, trifluoromethyl, cyclopropyl, cyanocyclopropyl, cyanoisopropyl, trifluoroethoxy, -N(R₄)₂ or -N(R₄)COCH₃, both in which R₄ is independently hydrogen or methyl, or Q₁ is 2-pyridyloxy; or Q₁ is a five-membered aromatic ring system linked via a ring nitrogen atom to the ring which contains the substituent A, said ring system can be mono-substituted by substituents selected from the group consisting of halogen, cyano or C₁-C₄haloalkyl; and said ring system contains 2 nitrogen atoms; preferably Q₁ is N-linked pyrazolyl which can be mono-substituted by chloro, cyano or trifluoromethyl.

One further preferred group of compounds according to this embodiment are compounds of formula (I-4g) which are compounds of formula (I-4) wherein Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂ or-N(R₄)COR₅, both in which R₄ is independently hydrogen or C₁-C₄alkyl (preferably hydrogen or methyl) and R₅ is C₁-C₆alkyl (preferably methyl), 2-pyridyloxy or N-linked pyrazolyl which can be mono-substituted by chloro, cyano or trifluoromethyl; preferably Q₁ is hydrogen, chlorine, bromine, trifluoromethyl, cyclopropyl, 1-cyanocyclopropyl, 1-cyano-1-methyl-ethyl, 2,2,2-trifluoroethoxy, -NH₂, - NH(CH₃), -N(CH₃)₂, -NHCOCH₃, -N(CH₃)COCH₃, 2-pyridyloxy, pyrazol-1-yl, 3-chloro-pyrazol-1-yl, 3-cyano-pyrazol-1-yl or 3-trifluoromethyl-pyrazol-1-yl.

One further preferred group of compounds according to this embodiment are compounds of formula (I-4h) which are compounds of formula (I-4) wherein
A is N;
R₂ is C₁-C₆haloalkyl, preferably -CH₂CF₂CHF₂ or -CH₂CF₂CF₃;
R₃ is hydrogen or C₁-C₄alkyl, preferably hydrogen or methyl; and
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂ or -N(R₄)COR₅, both in which R₄ is independently hydrogen or C₁-C₄alkyl (preferably hydrogen or methyl) and R₅ is C₁-C₆alkyl (preferably methyl), or Q₁ is 2-pyridyloxy; preferably Q₁ is hydrogen, halogen, trifluoromethyl, cyclopropyl, cyanocyclopropyl, cyanoisopropyl, trifluoroethoxy, -N(R₄)₂ or -N(R₄)COCH₃, both in which R₄ is independently hydrogen or methyl, or Q₁ is 2-pyridyloxy; or
Q₁ is a five-membered aromatic ring system linked via a ring nitrogen atom to the ring which contains the substituent A, said ring system is unsubstituted or is mono-substituted by substituents selected from the group consisting of halogen, cyano and C₁-C₄haloalkyl; and said ring system contains 2 ring nitrogen atoms; preferably Q₁ is N-linked pyrazolyl which can be mono-substituted by chloro, cyano or trifluoromethyl.

Another preferred group of compounds according to this embodiment are compounds of formula (I-4i) which are compounds of formula (I-4) wherein
A is N;
R₂ is C₁-C₆haloalkyl, preferably -CH₂CF₂CHF₂ or-CH₂CF₂CF₃;
R₃ is hydrogen; and
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂ or -N(R₄)COR₅, both in which R₄ is independently hydrogen or C₁-C₄alkyl (preferably hydrogen or methyl) and R₅ is C₁-C₆alkyl (preferably methyl), 2-pyridyloxy or N-linked pyrazolyl which can be mono-substituted by chloro, cyano or trifluoromethyl; preferably Q₁ is hydrogen, chlorine, bromine, trifluoromethyl, cyclopropyl, 1-cyanocyclopropyl, 1-cyano-1-methyl-ethyl, 2,2,2-trifluoroethoxy, -NH₂, -NH(CH₃), -N(CH₃)₂, -NHCOCH₃, -N(CH₃)COCH₃, 2-pyridyloxy, pyrazol-1-yl, 3-chloro-pyrazol-1-yl, 3-cyano-pyrazol-1-yl or 3-trifluoromethyl-pyrazol-1-yl.

Another preferred group of compounds according to this embodiment are compounds of formula (I-4j) which are compounds of formula (I-4) wherein
A is N;
R₂ is C₁-C₆haloalkyl, preferably -CH₂CF₂CHF₂ or-CH₂CF₂CF₃;
R₃ is hydrogen; and
Q₁ is hydrogen, halogen, C₃-C₆cycloalkyl, C₁-C₆cyanoalkyl, or -N(R₄)COR₅ in which R₄ is independently hydrogen or C₁-C₄alkyl (preferably hydrogen or methyl) and R₅ is C₁-C₆alkyl (preferably methyl); preferably Q₁ is hydrogen, chlorine, bromine, cyclopropyl, 1-cyano-1-methyl-ethyl, or-N(CH₃)COCH₃.

Another preferred group of compounds according to this embodiment are compounds of formula (I-4k) which are compounds of formula (I-4) wherein
A is N;
R₂ is C₁-C₆haloalkyl, preferably -CH₂CF₂CHF₂ or-CH₂CF₂CF₃;
R₃ is C₁-C₄alkyl, preferably methyl; and
Q₁ is hydrogen, halogen, C₃-C₆cycloalkyl, C₁-C₆cyanoalkyl, or -N(R₄)COR₅ in which R₄ is hydrogen or C₁-C₄alkyl (preferably hydrogen or methyl) and R₅ is C₁-C₆alkyl (preferably methyl); preferably Q₁ is hydrogen, chlorine, bromine, cyclopropyl, 1-cyano-1-methyl-ethyl, or -N(CH₃)COCH₃.

One group of compounds according to the invention are those of formula I-5 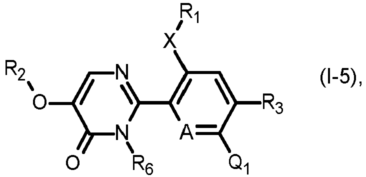
wherein A, X, R₁, R₂ and R₆ are as defined for compounds of formula I (above), or an agrochemically acceptable salt, stereoisomer, enantiomer, tautomer or N-oxide thereof, and wherein Q₁ is preferably hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the ring which contains the substituent A, said ring system is unsubstituted or is mono-substituted by substituents selected from the group consisting of halogen, cyano and C₁-C₄haloalkyl; and said ring system contains 2 or 3 ring nitrogen atoms;
R₃ is preferably hydrogen or C₁-C₄alkyl;
R₄ is independently preferably hydrogen or C₁-C₄alkyl; and
R₅ is preferably C₁-C₆alkyl or C₃-C₆cycloalkyl.

Preferred definitions of A, X, R₁, R₂ and R₆ are as defined for compounds of formula I (above), and more preferably Q₁ is hydrogen, halogen, trifluoromethyl, cyclopropyl, cyanocyclopropyl, cyanoisopropyl, trifluoroethoxy, -N(R₄)₂, -N(R₄)COR₅, or -N(R₄)CON(R₄)₂, in which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl; and R₃ is hydrogen or methyl, preferably hydrogen.

One group of compounds according to this embodiment are compounds of formula (I-5a) which are compounds of formula (I-5) wherein A is N.

Another group of compounds according to this embodiment are compounds of formula (I-5b) which are compounds of formula (I-5) wherein A is CH.

Another group of compounds according to the invention are those of formula I-6 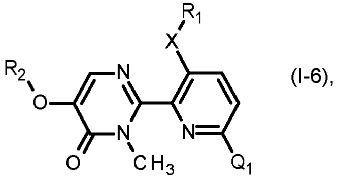
wherein X, R₁ and R₂ are as defined for compounds of formula I (above), or an agrochemically acceptable salt, stereoisomer, enantiomer, tautomer or N-oxide thereof, and wherein Q₁ is preferably hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the pyridyl ring substituted by X-R₁, said ring system is unsubstituted or is mono-substituted by substituents selected from the group consisting of halogen, cyano and C₁-C₄haloalkyl; and said ring system can contain 1 or 2 nitrogen atoms; or
Q₁ is a five-membered aromatic ring system linked via a ring nitrogen atom to the pyridyl ring substituted by X-R₁, said ring system is unsubstituted or is mono-substituted by substituents selected from the group consisting of halogen, cyano and C₁-C₄haloalkyl; and said ring system contains 2 or 3 nitrogen atoms;
R₄ is independently preferably hydrogen or C₁-C₄alkyl; and
R₅ is preferably C₁-C₆alkyl or C₃-C₆cycloalkyl.

Preferred definitions of X, R₁ and R₂ are as defined for compounds of formula I (above), and more preferably Q₁ is hydrogen, halogen, trifluoromethyl, cyclopropyl, cyanocyclopropyl, cyanoisopropyl, trifluoroethoxy, -N(R₄)₂, -N(R₄)COR₅, or -N(R₄)CON(R₄)₂, in which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl.

One group of compounds according to this embodiment are compounds of formula (I-6a) which are compounds of formula (I-6) wherein X is S or SOz, preferably SOz.

Another group of compounds according to this embodiment are compounds of formula (I-6b-1) which are compounds of formula (I-6) wherein R₁ is C₁-C₄alkyl or cyclopropyl-C₁-C₄alkyl, preferably ethyl or cyclopropylmethyl.

Another group of compounds according to this embodiment are compounds of formula (I-6b-2) which are compounds of formula (I-6) wherein R₁ is C₁-C₄alkyl, preferably ethyl.

Another group of compounds according to this embodiment are compounds of formula (I-6c) which are compounds of formula (I-6) wherein R₂ is C₁-C₆haloalkyl, more preferably C₁-C₆fluoroalkyl, most preferably -CH₂CF₂CHF₂ or -CH₂CF₂CF₃.

Another group of compounds according to the invention are those of formula I-7 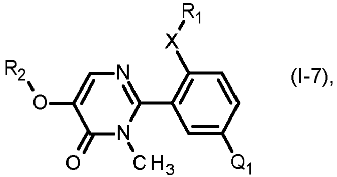
wherein X, R₁ and R₂ are as defined for compounds of formula I (above), or an agrochemically acceptable salt, stereoisomer, enantiomer, tautomer or N-oxide thereof, and wherein Q₁ is preferably hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the phenyl ring substituted by X-R₁, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the phenyl ring substituted by X-R₁, said ring system is unsubstituted or is mono-substituted by substituents selected from the group consisting of halogen, cyano and C₁-C₄haloalkyl; and said ring system contains 2 or 3 ring nitrogen atoms;
R₄ is independently preferably hydrogen or C₁-C₄alkyl; and
R₅ is preferably C₁-C₆alkyl or C₃-C₆cycloalkyl.

Preferred definitions of X, R₁ and R₂ are as defined for compounds of formula I (above), and more preferably Q₁ is hydrogen, halogen, trifluoromethyl, cyclopropyl, cyanocyclopropyl, cyanoisopropyl, trifluoroethoxy, -N(R₄)₂, -N(R₄)COR₅, or -N(R₄)CON(R₄)₂, in which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl.

One group of compounds according to this embodiment are compounds of formula (I-7a) which are compounds of formula (I-7) wherein X is S or SOz, preferably SOz.

Another group of compounds according to this embodiment are compounds of formula (I-7b-1) which are compounds of formula (I-7) wherein R₁ is C₁-C₄alkyl or cyclopropyl-C₁-C₄alkyl, preferably ethyl or cyclopropylmethyl.

Another group of compounds according to this embodiment are compounds of formula (I-7b-2) which are compounds of formula (I-7) wherein R₁ is C₁-C₄alkyl, preferably ethyl.

Another group of compounds according to this embodiment are compounds of formula (I-7c) which are compounds of formula (I-7) wherein R₂ is C₁-C₆haloalkyl, more preferably C₁-C₆fluoroalkyl, most preferably -CH₂CF₂CHF₂ or -CH₂CF₂CF₃.

Another group of compounds according to the invention are those of formula I-8 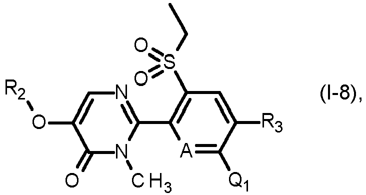 wherein
A is CH or N, preferably N;
R₂ is C₁-C₆haloalkyl, preferably -CH₂CF₂CHF₂ or-CH₂CF₂CF₃;
R₃ is hydrogen or C₁-C₄alkyl, preferably hydrogen or methyl;
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the ring which contains the substituent A, said ring system can be mono-substituted by substituents selected from the group consisting of halogen, cyano or C₁-C₄haloalkyl; and said ring system contains 2 or 3 ring nitrogen atoms;
R₄ is independently hydrogen or C₁-C₄alkyl, preferably hydrogen or methyl; and
R₅ is C₁-C₆alkyl or C₃-C₆cycloalkyl, preferably methyl or cyclopropyl; or
an agrochemically acceptable salt, stereoisomer, enantiomer, tautomer or N-oxide thereof.

One further preferred group of compounds according to this embodiment are compounds of formula (I-8a) which are compounds of formula (I-8) wherein A is N.

Another preferred group of compounds according to this embodiment are compounds of formula (I-8b) which are compounds of formula (I-8) wherein A is CH.

One further preferred group of compounds according to this embodiment are compounds of formula (I-8c) which are compounds of formula (I-8) wherein R₃ is hydrogen.

Another preferred group of compounds according to this embodiment are compounds of formula (I-8d) which are compounds of formula (I-8) wherein R₃ is C₁-C₄alkyl, preferably methyl.

One further preferred group of compounds according to this embodiment are compounds of formula (I-8e) which are compounds of formula (I-8) wherein A is N and R₃ is hydrogen.

One further preferred group of compounds according to this embodiment are compounds of formula (I-8f) which are compounds of formula (I-8) wherein Q₁ is hydrogen, halogen, C₃-C₆cycloalkyl, -N(R₄)₂, - N(R₄)COR₅ or -N(R₄)CON(R₄)₂, in each of which R₄ is independently either hydrogen or C₁-C₄alkyl (preferably hydrogen or methyl) and R₅ is C₁-C₆alkyl or C₃-C₆cycloalkyl (preferably methyl or cyclopropyl), or Q₁ is (oxazolidin-2-one)-3-yl; preferably Q₁ is hydrogen, halogen, cyclopropyl, -N(R₄)₂, - N(R₄)COR₅ or -N(R₄)CON(R₄)₂, in each of which R₄ is independently either hydrogen or methyl and R₅ is methyl or cyclopropyl, or Q₁ is (oxazolidin-2-one)-3-yl; or
Q₁ is a six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system contains 2 ring nitrogen atoms; preferably Q₁ is pyrimidinyl which can be mono-substituted by chloro, cyano or trifluoromethyl; or Q₁ is a five-membered aromatic ring system linked via a ring nitrogen atom to the ring which contains the substituent A, said ring system is unsubstituted or is mono-substituted by substituents selected from the group consisting of halogen, cyano and C₁-C₄haloalkyl; and said ring system contains 3 ring nitrogen atoms; preferably Q₁ is N-linked triazolyl which can be mono-substituted by chloro, cyano or trifluoromethyl.

One further preferred group of compounds according to this embodiment are compounds of formula (I-8g) which are compounds of formula (I-8) wherein Q₁ is hydrogen, halogen, C₃-C₆cycloalkyl, -N(R₄)₂, - N(R₄)COR₅ or -N(R₄)CON(R₄)₂, in each of which R₄ is independently either hydrogen or C₁-C₄alkyl (preferably hydrogen or methyl) and R₅ is C₁-C₆alkyl or C₃-C₆cycloalkyl (preferably methyl or cyclopropyl), (oxazolidin-2-one)-3-yl, pyrimidinyl or N-linked triazolyl; preferably Q₁ is hydrogen, chlorine, bromine, cyclopropyl, -NH₂, -NH(CH₃), -NHCOCH₃, -N(CH₃)COCH₃, -NHCO(cyclopropyl), - N(CH₃)CO(cyclopropyl), -N(H)CONH₂, -N(H)CONH(CH₃), -N(H)CON(CH₃)₂, -N(CH₃)CONH₂, - N(CH₃)CONH(CH₃), -N(CH₃)CON(CH₃)₂, (oxazolidin-2-one)-3-yl, pyrimidin-2-yl or 1,2,4-triazol-1-yl.

One further preferred group of compounds according to this embodiment are compounds of formula (I-8h) which are compounds of formula (I-8) wherein
A is N;
R₂ is C₁-C₆haloalkyl, preferably -CH₂CF₂CHF₂ or-CH₂CF₂CF₃;
R₃ is hydrogen or C₁-C₄alkyl, preferably hydrogen or methyl; and
Q₁ is hydrogen, halogen, C₃-C₆cycloalkyl, -N(R₄)₂, -N(R₄)COR₅ or -N(R₄)CON(R₄)₂, in each of which R₄ is independently either hydrogen or C₁-C₄alkyl (preferably hydrogen or methyl) and R₅ is C₁-C₆alkyl or C₃-C₆cycloalkyl (preferably methyl or cyclopropyl), or Q₁ is (oxazolidin-2-one)-3-yl; preferably Q₁ is hydrogen, halogen, cyclopropyl, -N(R₄)₂, -N(R₄)COR₅ or-N(R₄)CON(R₄)₂, in each of which R₄ is independently either hydrogen or methyl and R₅ is methyl or cyclopropyl, or Q₁ is (oxazolidin-2-one)-3-yl; or
Q₁ is a six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system contains 2 ring nitrogen atoms; preferably Q₁ is pyrimidinyl which can be mono-substituted by chloro, cyano or trifluoromethyl; or Q₁ is a five-membered aromatic ring system linked via a ring nitrogen atom to the ring which contains the substituent A, said ring system is unsubstituted or is mono-substituted by substituents selected from the group consisting of halogen, cyano and C₁-C₄haloalkyl; and said ring system contains 3 ring nitrogen atoms; preferably Q₁ is N-linked triazolyl which can be mono-substituted by chloro, cyano or trifluoromethyl.

Another preferred group of compounds according to this embodiment are compounds of formula (I-8i) which are compounds of formula (I-8) wherein
A is N;
R₂ is C₁-C₆haloalkyl, preferably -CH₂CF₂CHF₂ or-CH₂CF₂CF₃;
R₃ is hydrogen; and
Q₁ is hydrogen, halogen, C₃-C₆cycloalkyl, -N(R₄)₂, -N(R₄)COR₅ or -N(R₄)CON(R₄)₂, in each of which R₄ is independently either hydrogen or C₁-C₄alkyl (preferably hydrogen or methyl) and R₅ is C₁-C₆alkyl or C₃-C₆cycloalkyl (preferably methyl or cyclopropyl), (oxazolidin-2-one)-3-yl, pyrimidinyl or N-linked triazolyl; preferably Q₁ is hydrogen, chlorine, bromine, cyclopropyl, -NH₂, -NH(CH₃), -NHCOCH₃, - N(CH₃)COCH₃, -NHCO(cyclopropyl), -N(CH₃)CO(cyclopropyl), -N(H)CONH₂, -N(H)CONH(CH₃), - N(H)CON(CH₃)₂, -N(CH₃)CONH₂, -N(CH₃)CONH(CH₃), -N(CH₃)CON(CH₃)₂, (oxazolidin-2-one)-3-yl, pyrimidin-2-yl or 1,2,4-triazol-1-yl.

Another preferred group of compounds according to this embodiment are compounds of formula (I-8j) which are compounds of formula (I-8) wherein
A is N;
R₂ is C₁-C₆haloalkyl, preferably -CH₂CF₂CHF₂ or -CH₂CF₂CF₃;
R₃ is hydrogen; and
Q₁ is hydrogen, halogen, -N(R₄)CON(R₄)₂, in which R₄ is independently either hydrogen or C₁-C₄alkyl (preferably hydrogen or methyl) or 1,2,4-triazol-1-yl; preferably Q₁ is hydrogen, chlorine, bromine, - N(H)CONH(CH₃), -N(CH₃)CONH(CH₃) or 1,2,4-triazol-1-yl.

An outstanding group of compounds according to the invention are those of formula I-9 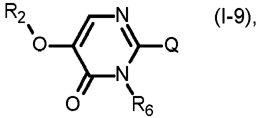 wherein
R₂ is C₁-C₆haloalkyl, preferably -CH₂CF₂CHF₂ or-CH₂CF₂CF₃;
R₆ is C₁-C₃alkyl, preferably methyl;
Q is a radical selected from the group consisting of formula Qa1 and Qb1 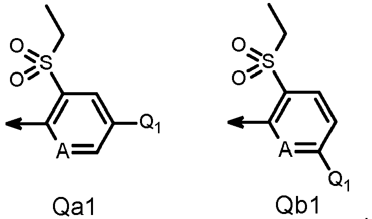
wherein the arrow denotes the point of attachment to the pyrimidinone ring;
and wherein
A is CH or N, preferably N; and
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅ or -N(R₄)CON(R₄)₂, in each of which R₄ is independently either hydrogen or C₁-C₄alkyl (preferably hydrogen or methyl) and R₅ is C₁-C₆alkyl or C₃-C₆cycloalkyl (preferably methyl or cyclopropyl), (oxazolidin-2-one)-3-yl, 2-pyridyloxy, N-linked pyrazolyl which can be mono-substituted by chloro, cyano or trifluoromethyl; or Q₁ is pyrimidinyl or N-linked triazolyl; preferably Q₁ is hydrogen, chlorine, bromine, trifluoromethyl, cyclopropyl, 1-cyanocyclopropyl, 1-cyano-1-methyl-ethyl, 2,2,2-trifluoroethoxy, -NH₂, -NH(CH₃), -N(CH₃)₂, -NHCOCH₃, -N(CH₃)COCH₃, - NHCO(cyclopropyl), -N(CH₃)CO(cyclopropyl), -N(H)CONH₂, -N(H)CONH(CH₃), -N(H)CON(CH₃)₂, - N(CH₃)CONH₂, -N(CH₃)CONH(CH₃), -N(CH₃)CON(CH₃)₂, (oxazolidin-2-one)-3-yl, 2-pyridyloxy, pyrazol-1-yl, 3-chloro-pyrazol-1-yl, 3-cyano-pyrazol-1-yl, 3-trifluoromethyl-pyrazol-1-yl, pyrimidin-2-yl or 1,2,4-triazol-1-yl.

One further outstanding group of compounds according to this embodiment are compounds of formula (I-9a) which are compounds of formula (I-9) wherein
R₂ is C₁-C₆haloalkyl, preferably -CH₂CF₂CHF₂ or -CH₂CF₂CF₃;
R₆ is C₁-C₃alkyl, preferably methyl;
Q is a radical selected from the group consisting of formula Qa1 and Qb1, wherein A is N; and
Q₁ is hydrogen, halogen, C₃-C₆cycloalkyl, C₁-C₆cyanoalkyl, -N(R₄)COR₅ or -N(R₄)CON(R₄)₂, both in which R₄ is independently either hydrogen or C₁-C₄alkyl (preferably hydrogen or methyl) and R₅ is C₁-C₆alkyl (preferably methyl) or 1,2,4-triazol-1-yl; preferably Q₁ is hydrogen, chlorine, bromine, cyclopropyl, 1-cyano-1-methyl-ethyl, -N(CH₃)COCH₃, -N(H)CONH(CH₃), -N(CH₃)CONH(CH₃) or 1,2,4-triazol-1-yl.

One further outstanding group of compounds according to this embodiment are compounds of formula (I-9b) which are compounds of formula (I-9) wherein
R₂ is C₁-C₆haloalkyl, preferably -CH₂CF₂CHF₂ or -CH₂CF₂CF₃;
R₆ is methyl;
Q is a radical selected from the group consisting of formula Qa1 and Qb1, wherein A is N; and
Q₁ is hydrogen, chlorine, bromine, cyclopropyl, 1-cyano-1-methyl-ethyl, -N(CH₃)COCH₃, - N(H)CONH(CH₃), -N(CH₃)CONH(CH₃) or 1,2,4-triazol-1-yl.

One further outstanding group of compounds according to this embodiment are compounds of formula (I-9c) which are compounds of formula (I-9) wherein
R₂ is C₁-C₆haloalkyl, preferably -CH₂CF₂CHF₂ or-CH₂CF₂CF₃;
R₆ is methyl;
Q is a radical selected from the group consisting of formula Qa1 and Qb1, wherein A is N; and
Q₁ is hydrogen, bromine, cyclopropyl, 1-cyano-1-methyl-ethyl or-N(CH₃)COCH₃ when Q is Qa1; or
Q₁ is hydrogen, chlorine, -N(H)CONH(CH₃), -N(CH₃)CONH(CH₃) or 1,2,4-triazol-1-yl, when Q is Qb1.

One further outstanding group of compounds according to this embodiment are compounds of formula (I-9d) which are compounds of formula (I-9) wherein
R₂ is C₁-C₆haloalkyl, preferably -CH₂CF₂CHF₂ or-CH₂CF₂CF₃;
R₆ is C₁-C₃alkyl, preferably methyl;
Q is a radical selected from the group consisting of formula Qa1 and Qb1, wherein A is N; and
Q₁ is hydrogen, halogen, C₃-C₆cycloalkyl, C₃-C₆cycloalkyl monosubstituted by cyano, C₁-C₆cyanoalkyl, - N(R₄)₂, -N(R₄)COR₅ or -N(R₄)CON(R₄)₂, all in which R₄ is independently either hydrogen or C₁-C₄alkyl (preferably hydrogen or methyl) and R₅ is C₁-C₆alkyl or C₃-C₆cycloalkyl (preferably methyl or cyclopropyl), 2-pyridyloxy, or 1,2,4-triazol-1-yl; preferably Q₁ is hydrogen, chlorine, bromine, cyclopropyl, 1-cyanocyclopropyl, 1-cyano-1-methyl-ethyl, -NH(CH₃), -N(CH₃)COCH₃, -N(CH₃)CO(cyclopropyl), - N(CH₃)CONH(CH₃), 2-pyridyloxy or 1,2,4-triazol-1-yl.

One further outstanding group of compounds according to this embodiment are compounds of formula (I-9e) which are compounds of formula (I-9) wherein
R₂ is C₁-C₆haloalkyl, preferably -CH₂CF₂CHF₂ or-CH₂CF₂CF₃;
R₆ is methyl;
Q is a radical selected from the group consisting of formula Qa1 and Qb1, wherein A is N; and
Q₁ is hydrogen, chlorine, bromine, cyclopropyl, 1-cyanocyclopropyl, 1-cyano-1-methyl-ethyl, -NH(CH₃), - N(CH₃)COCH₃, -N(CH₃)CO(cyclopropyl), -N(CH₃)CONH(CH₃), 2-pyridyloxy or 1,2,4-triazol-1-yl.

One further outstanding group of compounds according to this embodiment are compounds of formula (I-9f) which are compounds of formula (I-9) wherein
R₂ is C₁-C₆haloalkyl, preferably -CH₂CF₂CHF₂ or-CH₂CF₂CF₃;
R₆ is methyl;
Q is a radical selected from the group consisting of formula Qa1 and Qb1, wherein A is N; and
Q₁ is hydrogen, halogen, C₃-C₆cycloalkyl, C₃-C₆cycloalkyl monosubstituted by cyano, C₁-C₆cyanoalkyl, - N(R₄)COR₅ in which R₄ is C₁-C₄alkyl (preferably methyl) and R₅ is C₁-C₆alkyl (preferably methyl), or 2-pyridyloxy when Q is Qa1; preferably Q₁ is hydrogen, bromine, cyclopropyl, 1-cyanocyclopropyl, 1-cyano-1-methyl-ethyl, 2-pyridyloxy or -N(CH₃)COCH₃ when Q is Qa1; or
Q₁ is hydrogen, halogen, -N(R₄)₂, -N(R₄)COR₅ or -N(R₄)CON(R₄)₂, all in which R₄ is independently either hydrogen or C₁-C₄alkyl (preferably hydrogen or methyl) and R₅ is C₁-C₆alkyl or C₃-C₆cycloalkyl (preferably methyl or cyclopropyl), or 1,2,4-triazol-1-yl, when Q is Qb1; preferably Q₁ is hydrogen, chlorine, -NH(CH₃), -N(CH₃)COCH₃, -N(CH₃)CO(cyclopropyl), -N(CH₃)CONH(CH₃) or 1,2,4-triazol-1-yl, when Q is Qb1.

One further outstanding group of compounds according to this embodiment are compounds of formula (I-9g) which are compounds of formula (I-9) wherein
R₂ is C₁-C₆haloalkyl, preferably -CH₂CF₂CHF₂ or-CH₂CF₂CF₃;
R₆ is methyl;
Q is a radical selected from the group consisting of formula Qa1 and Qb1, wherein A is N; and
Q₁ is hydrogen, bromine, cyclopropyl, 1-cyanocyclopropyl, 1-cyano-1-methyl-ethyl, 2-pyridyloxy or-N(CH₃)COCH₃ when Q is Qa1; or
Q₁ is hydrogen, chlorine, -NH(CH₃), -N(CH₃)COCH₃, -N(CH₃)CO(cyclopropyl), -N(CH₃)CONH(CH₃) or
1,2,4-triazol-1-yl, when Q is Qb1.

Compounds according to the invention may possess any number of benefits including, inter alia, advantageous levels of biological activity for protecting plants against insects or superior properties for use as agrochemical active ingredients (for example, greater biological activity, an advantageous spectrum of activity, an increased safety profile, improved physico-chemical properties, or increased biodegradability or environmental profile). In particular, it has been surprisingly found that certain compounds of formula (I) may show an advantageous safety profile with respect to non-target arthropods, in particular pollinators such as honey bees, solitary bees, and bumble bees. Most particularly, Apis mellifera.

In another aspect the present invention provides a composition comprising an insecticidally, acaricidally, nematicidally or molluscicidally effective amount of a compound of formula (I), or an agrochemically acceptable salt, stereoisomer, enantiomer, tautomer or N-oxide thereof, as defined in any of the embodiments under compounds of formula (I-1), (I-2), (I-3), (I-4), (I-5), (I-6), (I-7), (I-8) and (I-9) (above), and, optionally, an auxiliary or diluent.

In a further aspect the present invention provides a method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I), or an agrochemically acceptable salt, stereoisomer, enantiomer, tautomer or N-oxide thereof, as defined in any of the embodiments under compounds of formula (I-1), (I-2), (I-3), (I-4), (I-5), (I-6), (I-7), (I-8) and (I-9) (above) or a composition as defined above.

In a yet further aspect, the present invention provides a method for the protection of plant propagation material from the attack by insects, acarines, nematodes or molluscs, which comprises treating the propagation material or the site, where the propagation material is planted, with a composition as defined above.

The process according to the invention for preparing compounds of formula I is carried out in principle by methods known to those skilled in the art. More specifically, the subgroup of compounds of formula I, wherein X is SO (sulfoxide) and/or SOz (sulfone), may be obtained by means of an oxidation reaction of the corresponding sulfide compounds of formula I, wherein X is S, involving reagents such as, for example, m-chloroperoxybenzoic acid (mCPBA), hydrogen peroxide, oxone, sodium periodate, sodium hypochlorite or tert-butyl hypochlorite amongst other oxidants. The oxidation reaction is generally conducted in the presence of a solvent. Examples of the solvent to be used in the reaction include aliphatic halogenated hydrocarbons such as dichloromethane and chloroform; esters, such as ethyl acetate; alcohols such as methanol and ethanol; acetic acid; water; and mixtures thereof. The amount of the oxidant to be used in the reaction is generally 1 to 3 moles, preferably 1 to 1.2 moles, relative to 1 mole of the sulfide compounds I to produce the sulfoxide compounds I, and preferably 2 to 2.2 moles of oxidant, relative to 1 mole of of the sulfide compounds I to produce the sulfone compounds I. Such oxidation reactions are disclosed, for example, in WO 2013/018928.

Compounds of formula I, wherein R₂, R₆ and Q are as defined above, 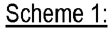 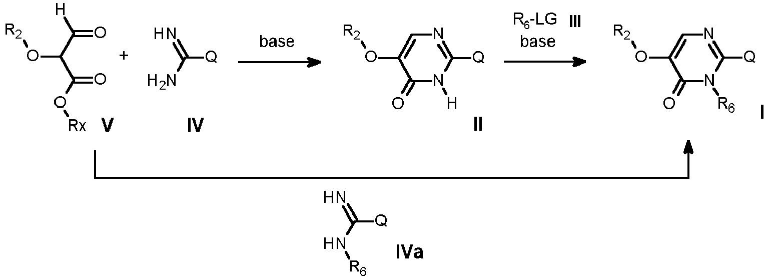 can be prepared (scheme 1) by reacting compounds of formula II, wherein R₂ and Q are as defined in formula I, with compounds of formula III, wherein R₆ is as defined in formula I, and in which LG is a halogen, preferably iodine, bromine or chlorine (or a pseudo-halogen leaving group, such as a (halo)alkyl or aryl sulfonic ester, e.g. triflate or tosylate), in the presence of a base, such as sodium hydride, an alkaline earth metal hydride, or an alkaline earth metal carbonate (e.g. sodium carbonate, potassium carbonate or cesium carbonate) or hydroxide, in an inert solvent such as tetrahydrofuran, dioxane, N,N-dimethylformamide DMF, N,N-dimethylacetamide or acetonitrile and the like, at temperatures between 0 and 120°C, by procedures well known to those skilled in the art.

Compounds of formula II, wherein R₂ and Q are as defined in formula I, can be prepared by reacting compounds of formula IV, or a salt thereof (such as a hydrohalide salt, preferably a hydrochloride or a hydrobromide salt, or a trifluoroacetic acid salt, or any other equivalent salt), wherein Q is as defined in formula I, with compounds of formula V, or a tautomeric form thereof, wherein R₂ is as defined in formula I, and in which Rx is C₁-C₆alkyl, in the presence of a base, such as an alkoxide (preferably sodium or potassium methoxide, or ethoxide, or tert-butoxide), in an alcohol solvent such as methanol, ethanol, isopropanol ort-butanol, at temperatures between 0 and 100°C, preferably between room temperature and and refluxing conditions, in analogy to procedures described in, for example, WO05/073234.

Alternatively, compounds of formula I, wherein R₂, R₆ and Q are as defined above, may be prepared by the reaction of compounds of formula IVa, wherein R₆ and Q are as defined in formula I, with compounds of formula V, or a tautomeric form thereof, wherein R₂ is as defined in formula I, and in which Rx is C₁-C₆alkyl, under similar conditions described above (transformation of compounds IV and compounds V into compounds II in scheme 1).

Compounds of formula III, wherein R₆ is as defined in formula I, and in which LG is a halogen, preferably iodine, bromine or chlorine (or a pseudo-halogen leaving group, such as a (halo)alkyl or aryl sulfonate ester, e.g. triflate or tosylate), are either known, commercially available or may be prepared by methods known to a person skilled in the art.

Compounds of formula IV, or a salt thereof (such as a hydrohalide salt, preferably a hydrochloride or a hydrobromide salt, or a trifluoroacetic acid salt, or any other equivalent salt), wherein Q is as defined in formula I, 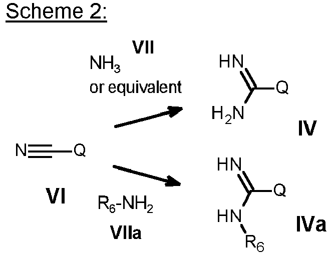 can be prepared (scheme 2) by reacting compounds of formula VI, wherein Q is as defined in formula I, with ammonia NH₃ VII or an ammonia equivalent such as for example ammonium chloride NH₄Cl, ammonium acetate NH₄OAc, ammonium carbonate (NH₄)₂CO₃, and other NH₃ surrogates, preferably in the presence of a Lewis acid, preferably an aluminum-based Lewis acid, such as aluminum trialkyls (e.g. trimethyl aluminum AlMe₃), alkyl aluminum halides (e.g. EtAlCl₂ or Et₂AlCl) or aluminum halides (e.g. aluminum chloride AlCl₃). This transformation is preferably performed in suitable solvents (or diluents) such as dichloromethane, 1,2-dichloroethane, benzene or toluene, at temperatures between 0-150°C, preferably at temperatures ranging from room temperature to the boiling point of the reaction mixture, optionally under microwave irradiation and optionally in a pressurized vessel, in analogy to procedures described in, for example, WO04/078128.

Alternatively, compounds of formula IV (or a salt thereof) may be prepared by reacting a nitrile compound of formula VI, sequentially with i) an alkoxide, preferably sodium methoxide NaOMe or sodium ethoxide NaOEt, in an alcoholic solvent, such as methanol or ethanol, at temperatures between 0 and 100°C, to generate an imidate intermediate (or a salt thereof); followed by ii) treatment of said imidate intermediate with ammonia NH₃ VII (or a salt thereof, or an ammonia surrogate), optionally in the presence of an acid (such as a hydrohalide acid, preferably hydrochloric acid or hydrobromic acid, or any other equivalent acid), at temperatures between 0-180°C. The imidate intermediate (or a salt thereof) may alternatively be prepared under conditions and variants of the Pinner reaction known to a person skilled in the art, typically by treating a compound of the formula VI with a hydrohalide acid, preferably hydrochloric acid, in presence of alcoholic reagents such as methanol or ethanol, preferably in an inert solvent such as diethyl ether, tetrahydrofuran or dioxane, at temperatures between -40 and 50°C, preferably between -20 and 20°C.

The compounds of formula IV-Qa and IV-Qb 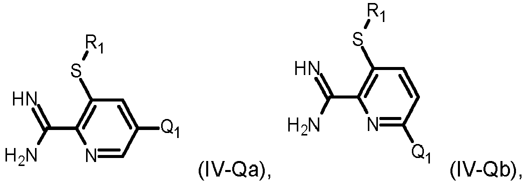 or a salt thereof (such as a hydrohalide salt, preferably a hydrochloride or a hydrobromide salt, or a trifluoroacetic acid salt, or any other equivalent salt), wherein
Q₁ and R₁ as defined under formula I above,
are novel, especially developed for the preparation of the compounds of formula I according to the invention and therefore represent a further object of the invention. The preferences and preferred embodiments of the substituents of the compounds of formula I are also valid for the compounds of formula IV-Qa and IV-Qb.

Similarly, compounds of formula IVa, or a salt thereof (such as a hydrohalide salt, preferably a hydrochloride or a hydrobromide salt, or a trifluoroacetic acid salt, or any other equivalent salt), wherein R₆ and Q are as defined in formula I, may be prepared by reacting nitrile compounds of formula VI under similar conditions described above (transformation of compounds VI and compounds VII into compounds IV in scheme 2), but by replacing reagent VII with compounds of the formula VIIa, (or a salt thereof), wherein R₆ is as defined in formula I.

Reagents of formula VII, and reagents of formula VIIa, or salts thereof, wherein R₆ is as defined in formula I, are either known, commercially available or may be prepared by methods known to a person skilled in the art.

Compounds of formula V, or a tautomeric form thereof, wherein R₂ is as defined in formula I, and in which Rx is C₁-C₆alkyl, 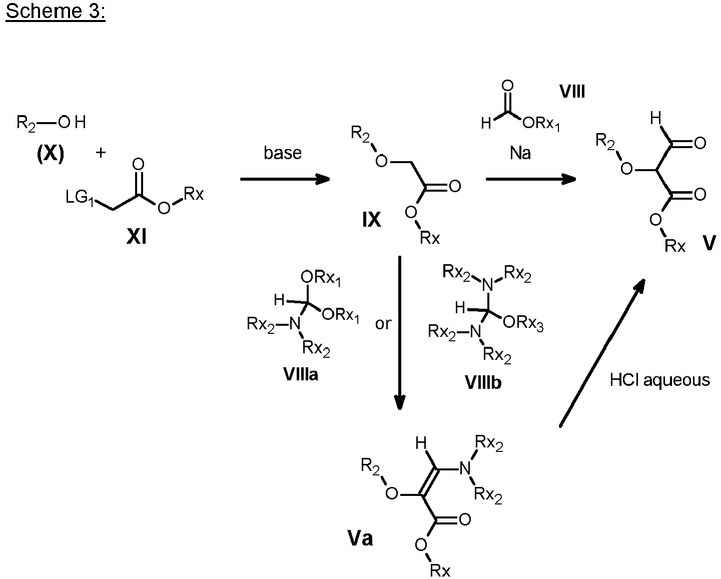 can be prepared (scheme 3) by reacting compounds of formula IX, wherein R₂ is as defined in formula I, and in which Rx is C₁-C₆alkyl, with compounds of formula VIII, wherein Rx₁ is C₁-C₆alkyl, in the presence of sodium Na (or potassium), in solvents such diethyl ether, t-butyl methyl ether or dioxane, at temperatures between 0 and 120°C, preferably between room temperature and refluxing conditions, by procedures described in, for example, WO 05/073234.

The compounds of formula V 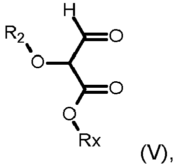 or a tautomeric form thereof, wherein
R₂ is C₁-C₆fluoroalkyl, and Rx is C₁-C₆alkyl,
are novel, especially developed for the preparation of the compounds of formula I according to the invention and therefore represent a further object of the invention. Preferably, R₂ is -CH₂CF₂CHF₂ or - CH₂CF₂CF₃; even more preferably R₂ is -CH₂CF₂CF₃. Preferably, Rx is methyl or ethyl; even more preferably Rx is methyl.

Alternatively, compounds of formula IX, wherein R₂ is as defined in formula I, and in which Rx is C₁-C₆alkyl, can be reacted with a compound of formula VIIIa, wherein Rx₁ is C₁-C₆alkyl (commonly methyl) and Rx₂ is C₁-C₆alkyl (commonly methyl), or with a compound of formula Vlllb, wherein Rx₂ is C₁-C₆alkyl (commonly methyl) and Rx₃ is C₁-C₆alkyl (commonly t-butyl) to form compounds of formula Va, wherein R₂ is as defined in formula I, and in which Rx is C₁-C₆alkyl and Rx₂ is C₁-C₆alkyl. These reactions can be performed in a solvent, with at least one equivalent of Villa or Vlllb, but can also be performed in an excess of Villa or Vlllb, at temperatures ranging from 0°C to the boiling temperature of the reaction mixture. The compounds of formula Va may be hydrolysed to the compounds of formula V under acidic conditions, for example with aqueous hydrochloric acid, and upon heating at temperatures ranging from room temperature to 80°C.

Compounds of formula IX, wherein R₂ is as defined in formula I, and in which Rx is C₁-C₆alkyl, can be prepared by reacting compounds of formula (X), wherein R₂ is as defined in formula I, with compounds of formula XI, wherein Rx is C₁-C₆alkyl, and in which LG₁ is a halogen, preferably iodine, bromine or chlorine (or a pseudo-halogen leaving group, such as a (halo)alkyl or phenyl sulfonate ester, e.g. triflate), in the presence of a base, such as sodium hydride, an alkaline earth metal hydride, or an alkaline earth metal carbonate (e.g. sodium carbonate, potassium carbonate or cesium carbonate) or hydroxide, in an inert solvent such as tetrahydrofuran, dioxane, N,N-dimethylformamide DMF, N,N-dimethylacetamide or acetonitrile and the like, at temperatures between 0 and 120°C, preferably between room temperature and 100°C, by procedures well known to those skilled in the art.

Compounds of formula (X), wherein R₂ is as defined in formula I; and compounds of formula XI, wherein Rx is C₁-C₆alkyl, and in which LG₁ is a halogen, preferably iodine, bromine or chlorine (or a pseudo-halogen leaving group, such as a (halo)alkyl or phenyl sulfonate ester, e.g. triflate); and reagents of formula VIII (e.g ethyl or methyl formate), or compounds of formula Vllla (e.g. dimethylformamide dimethyl acetal) or compounds of formula Vlllb (e.g. Bretherek's reagent) wherein Rx₁ is C₁-C₆alkyl, Rx₂ is C₁-C₆alkyl, Rx₃ is C₁-C₆alkyl are either known, commercially available or may be prepared by methods known to a person skilled in the art.

The subgroup of compounds of formula I, wherein R₂ and R₆ are as defined above and wherein Q is defined as Qb, in which A, Q₁, R₃, X and R₁ are as defined in formula I, may be defined as compounds of formula I-Qb (scheme 4). 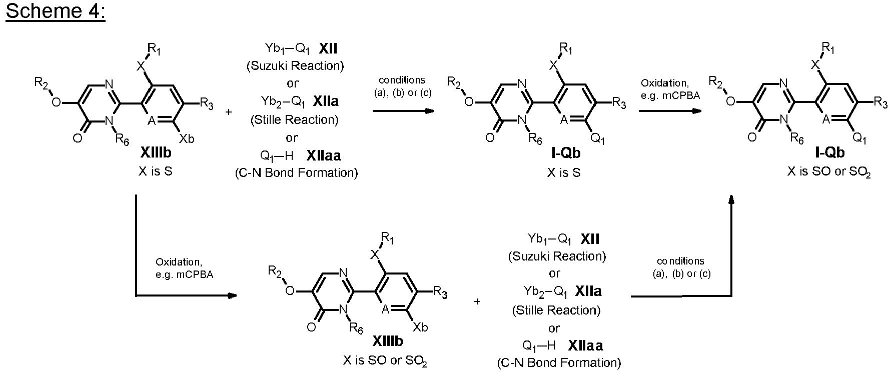
(a) Suzuki reaction: Pd cat. (e.g. Pd(PPh₃)₄ or Pd(dppf)Cl₂), base (e.g. Na₂CO₃), solvent (e.g. 1,2-dimethoxyethane I water), 25-180°C.
(b) Stille reaction: Pd cat. (e.g. Pd(PPh₃)₄ or Pd(PPh₃)Cl₂), solvent (e.g. toluene), 25-180°C.
(c) C-N bond formation: Optional base (e.g. K₂CO₃ or Cs₂CO₃), optional presence of copper or palladium catalyst, optional additive (such as N,N'-dimethylethylenediamine), optional ligand (such as Xantphos), solvent (e.g. dioxane, pyridine or N,N-dimethylformamide DMF), 25-180°C,

In the particular situation within scheme 4 when Q₁ is an optionally substituted triazole linked via a ring nitrogen atom to the ring which contains the group A, then compounds of formula l-Qb, wherein X is SO or SOz, may be prepared from compounds of formula Xlllb, wherein A, R₃, R₁, R₂ and R₆ are as defined in formula I, and in which X is SO or SOz, and wherein Xb is a leaving group like, for example, chlorine, bromine or iodine (preferably chlorine or bromine), or an aryl- or alkylsulfonate such as trifluoromethanesulfonate, by reaction (C-N bond formation) with an optionally substituted triazole Q₁-H (which contains an appropriate NH functionality) (Xllaa), wherein Q₁ is N-linked triazolyl, in solvents such as alcohols (eg. methanol, ethanol, isopropanol, or higher boiling linear or branched alcohols), pyridine or acetic acid, optionally in the presence of an additional base, such as potassium carbonate K₂CO₃ or cesium carbonate Cs₂CO₃, optionally in the presence of a copper catalyst, for example copper(I) iodide, at temperatures between 30-180°C, optionally under microwave irradiation.

In the particular situation within scheme 4 when Q₁ is (oxazolidin-2-one)-3-yl, -N(R₄)COR₅, or - N(R₄)CON(R₄)₂, wherein R₄ and R₅ are as defined in formula I, then compounds of formula I-Qb, wherein X is SO or SOz, may be prepared from compounds of formula XIIIb, wherein A, R₃, R₁, R₂ and R₆ are as defined in formula I, and in which X is SO or SO₂, and wherein Xb is a leaving group like, for example, chlorine, bromine or iodine (preferably chlorine or bromine), or an aryl- or alkylsulfonate such as trifluoromethanesulfonate, by reaction (C-N bond formation) with a reagent Q₁-H (Xllaa) equivalent to oxazolidin-2-one, HN(R₄)COR₅, or HN(R₄)CON(R₄)₂, wherein R₄ and R₅ are as defined in formula I. Such a reaction is performed in the presence of a base, such as potassium carbonate, cesium carbonate, sodium hydroxide, in an inert solvent, such as toluene, dimethylformamide DMF, N-methyl pyrrolidine NMP, dimethyl sulfoxide DMSO, dioxane, tetrahydrofuran THF, and the like, optionally in the presence of a catalyst, for example palladium(II)acetate, bis(dibenzylideneacetone)palladium(0) (Pd(dba)₂) or tris(dibenzylideneacetone)dipalladium(0) (Pd₂(dba)₃, optionally in form of a chloroform adduct), or a palladium pre-catalyst such as for example *tert*-BuBrettPhos Pd G3 [(2-Di-*tert-*butylphosphino-3,6-dimethoxy-2',4',6'-triisopropyl-1,1'-biphenyl)-2-(2'-amino-1,1'-biphenyl)] palladium(II) methanesulfonate or BrettPhos Pd G3 [(2-di-cyclohexylphosphino-3,6-dimethoxy-2',4',6'- triisopropyl-1,1'-biphenyl)-2-(2'-amino-1,1'-biphenyl)]palladium(ll) methanesulfonate, and optionally in the presence of a ligand, for example SPhos, *t*-BuBrettPhos or Xantphos, at temperatures between 60-180°C, optionally under microwave irradiation.

Compounds within scheme 4 wherein Q₁ is -N(R₄)COR₅ can alternatively be prepared by acylating compounds within scheme 4 wherein Q₁ is -NH(R₄) (prepared as described below) with reagents of the formula X₀₁-COR₅ (e.g. acetyl chloride) under conditions known to a person skilled in the art (all substituents as defined above; X₀₁ is a halogen, preferably chlorine).

In the particular situation within scheme 4 when Q₁ is -N(R₄)₂, wherein R₄ is as defined in formula I, then compounds of formula l-Qb, wherein X is SO or SOz, may be prepared from compounds of formula Xlllb, wherein A, R₃, R₁, R₂ and R₆ are as defined in formula I, and in which X is SO or SO₂, and wherein Xb is a leaving group like, for example, chlorine, bromine or iodine (preferably chlorine or bromine), or an aryl- or alkylsulfonate such as trifluoromethanesulfonate, by reaction (C-N bond formation) with a reagent Q₁-H (Xllaa) equivalent to HN(R₄)₂, or a salt thereof (such as a hydrohalide salt, preferably a hydrochloride or a hydrobromide salt, or a trifluoroacetic acid salt, or any other equivalent salt), wherein R₄ is as defined in formula I. Such a reaction is commonly performed in an inert solvent such as alcohols, amides, esters, ethers, nitriles and water, particularly preferred are methanol, ethanol, 2,2,2-trifluoroethanol, propanol, isopropanol, N,N-dimethylformamide, N,N-dimethylacetamide, dioxane, tetrahydrofuran, dimethoxyethane, acetonitrile, ethyl acetate, toluene, water or mixtures thereof, at temperatures between 0-150°C, optionally under microwave irradiation or pressurized conditions using an autoclave, optionally in the presence of a copper catalyst, such as copper powder, copper(I) iodide or copper sulfate (optionally in form of a hydrate), or mixtures thereof, optionaly in presence a ligand, for example diamine ligands (e.g. N,N'-dimethylethylenediamine or *trans-*cyclohexyldiamine) or dibenzylideneacetone (dba), or 1,10-phenanthroline, and optionally in presence of a base such as potassium phosphate.

Reagents HN(R₄)₂, HN(R₄)COR₅, or HN(R₄)CON(R₄)₂, wherein R₄ and R₅ are as defined in formula I, are either known, commercially available or may be prepared by methods known to a person skilled in the art.

Alternatively, compounds of formula l-Qb, wherein X is SO or SO₂, may be prepared by a Suzuki reaction (scheme 4), which involves for example, reacting compounds of formula Xlllb, wherein A, R₃, R₁, R₂ and R₆ are as defined in formula I, and in which X is SO or SO₂, and wherein Xb is a leaving group like, for example, chlorine, bromine or iodine (preferably chlorine or bromine), or an aryl- or alkylsulfonate such as trifluoromethanesulfonate, with compounds of formula (XII), wherein Q₁ is as defined in formula I, and wherein Y_{b1} can be a boron-derived functional group, such as for example B(OH)₂ or B(OR_{b1})₂ wherein R_{b1} can be a C₁-C₄alkyl group or the two groups OR_{b1} can form together with the boron atom a five membered ring, as for example a pinacol boronic ester. The reaction may be catalyzed by a palladium based catalyst, for example tetrakis(triphenyl-phosphine)palladium(0), (1,1'bis(diphenylphosphino)ferrocene)dichloro-palladium-dichloromethane (1:1 complex) or chloro(2-dicyclohexylphosphino-2',4',6'-triisopropyl-1,1'-biphenyl)[2-(2'-amino-1,1'-biphenyl)]palladium(II) (XPhos palladacycle), in presence of a base, like sodium carbonate, tripotassium phosphate or cesium fluoride, in a solvent or a solvent mixture, like, for example dioxane, acetonitrile, N,N-dimethyl-formamide, a mixture of 1 ,2-dimethoxyethane and water or of dioxane/water, or of toluene/water, preferably under inert atmosphere. The reaction temperature can preferentially range from room temperature to the boiling point of the reaction mixture, or the reaction may be performed under microwave irradiation. Such Suzuki reactions are well known to those skilled in the art and have been reviewed, for example, in J.Orgmet. Chem. 576, 1999, 147-168.

Alternatively compounds of formula l-Qb, wherein X is SO or SO₂, may be prepared by a Stille reaction between compounds of formula (Xlla), wherein Q₁ is as defined above, and wherein Y_{b2} is a trialkyl tin derivative, preferably tri-n-butyl tin or tri-methyl tin, and compounds of formula Xlllb, wherein A, R₃, R₁, R₂ and R₆ are as defined in formula I, and in which X is SO or SO₂, and wherein Xb is a leaving group like, for example, chlorine, bromine or iodine (preferably chlorine or bromine), or an aryl- or alkylsulfonate such as trifluoromethanesulfonate. Such Stille reactions are usually carried out in the presence of a palladium catalyst, for example tetrakis(triphenylphosphine)palladium(0), or bis(triphenylphosphine)palladium(II) dichloride, in an inert solvent such as N,N-dimethylformamide, acetonitrile, toluene or dioxane, optionally in the presence of an additive, such as cesium fluoride, or lithium chloride, and optionally in the presence of a further catalyst, for example copper(I)iodide. Such Stille couplings are also well known to those skilled in the art, and have been described in for example J. Org. Chem., 2005, 70, 8601-8604, J. Org. Chem., 2009, 74, 5599-5602, and Angew. Chem. Int. Ed., 2004, 43, 1132-1136.

When Q₁ is a five-membered aromatic ring system linked via a ring nitrogen atom to the ring which contains the substituent A, then compounds of formula l-Qb, wherein X is SO or SO₂, may be prepared from compounds of formula Xlllb, wherein A, R₃, R₁, R₂ and R₆ are as defined in formula I, and in which X is SO or SOz, and wherein Xb is a leaving group like, for example, chlorine, bromine or iodine (preferably chlorine or bromine), or an aryl- or alkylsulfonate such as trifluoromethanesulfonate, by reaction with a heterocycle Q₁-H (which contains an appropriate NH functionality) (Xllaa), wherein Q₁ is as defined above, in the presence of a base, such as potassium carbonate K₂CO₃ or cesium carbonate Cs₂CO₃, optionally in the presence of a copper catalyst, for example copper(I) iodide, with or without an additive such as L-proline, N,N'-dimethylcyclohexane-1 ,2-diamine or N,N'-dimethyl-ethylene-diamine, in an inert solvent such as N-methylpyrrolidone NMP or N,N-dimethylformamide DMF at temperatures between 30-150°C, optionally under microwave irradiation.

Oxidation of compounds of formula Xlllb, wherein A, R₃, R₁, R₂ and R₆ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, with a suitable oxidizing agent, into compounds of formula Xlllb, wherein X is SO or SOz may be achieved under conditions already described above.

A large number of compounds of the formula (XII), (Xlla) and (Xllaa) are commercially available or can be prepared by those skilled in the art.

Alternatively, compounds of formula I-Qb, wherein X is SO or SO₂, may be prepared from compounds of formula Xlllb, wherein X is S (sulfide) by involving the same chemistry as described above (scheme 4), but by changing the order of the steps (i.e. by running the sequence Xlllb (X is S) to I-Qb (X is S) via Suzuki, Stille or C-N bond formation, followed by an oxidation step to form I-Qb (X is SO or SOz).

Compounds of formula Xlllb, wherein A, R₃, R₁, R₂ and R₆ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, 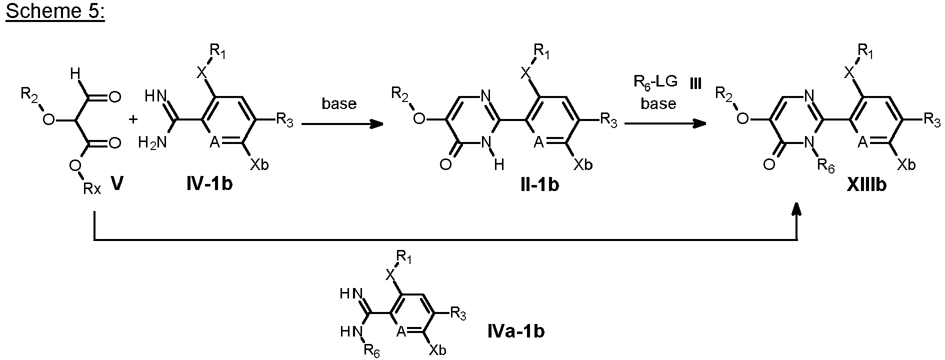 can be prepared (scheme 5) by reacting compounds of formula II-1b, wherein A, R₃, R₁ and R₂ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, with compounds of formula III, wherein R₆ is as defined in formula I, and in which LG is a halogen, preferably iodine, bromine or chlorine (or a pseudo-halogen leaving group, such as a (halo)alkyl or phenyl sulfonate ester, e.g. triflate), under conditions already described above (transformation of compounds II into compounds I in scheme 1).

Compounds of formula II-1b, wherein A, R₃, R₁ and R₂ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, can be prepared by reacting compounds of formula IV-1b, or a salt thereof (such as a hydrohalide salt, preferably a hydrochloride or a hydrobromide salt, or a trifluoroacetic acid salt, or any other equivalent salt), wherein A, R₃ and R₁ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, with compounds of formula V, or a tautomeric form thereof, wherein R₂ is as defined in formula I, and in which Rx is C₁-C₆alkyl, under conditions already described above (condensation of compounds IV and V into compounds II in scheme 1).

Alternatively, compounds of formula Xlllb, may be prepared by the reaction of compounds of formula IVa-1b, or a salt thereof as described above, wherein R₆, A, R₃ and R₁ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, with compounds of formula V, or a tautomeric form thereof, wherein R₂ is as defined in formula I, and in which Rx is C₁-C₆alkyl, under similar conditions described above (condensation of compounds IVa and compounds V into compounds II in scheme 1).

Compounds of formula IV-1b, or a salt thereof (such as a hydrohalide salt, preferably a hydrochloride or a hydrobromide salt, or a trifluoroacetic acid salt, or any other equivalent salt), wherein A, R₃ and R₁ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, 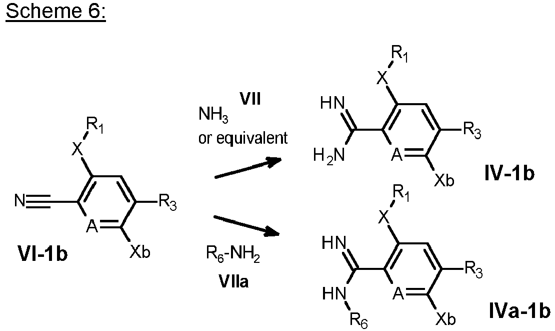 can be prepared (scheme 6) by reacting compounds of formula VI-1b, wherein wherein A, R₃ and R₁ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, with ammonia NH₃ VII or an ammonia surrogate, under similar conditions described above (transformation of compounds VI into compounds IV in scheme 2).

Similarly, compounds of formula IVa-1b, or a salt thereof as described above, wherein R₆, A, R₃ and R₁ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, may be prepared by reacting nitrile compounds of formula VI-1b with compounds of the formula VIIa, (or a salt thereof), wherein R₆ is as defined in formula I, under similar conditions described above (transformation of compounds VI and compounds VIIa into compounds IVa in scheme 2).

The subgroup of compounds of formula I, wherein R₂ and R₆ are as defined above and wherein Q is defined as Qa, in which A, Q₁, R₃, X and R₁ are as defined in formula I, may be defined as compounds of formula I-Qa (scheme 7). 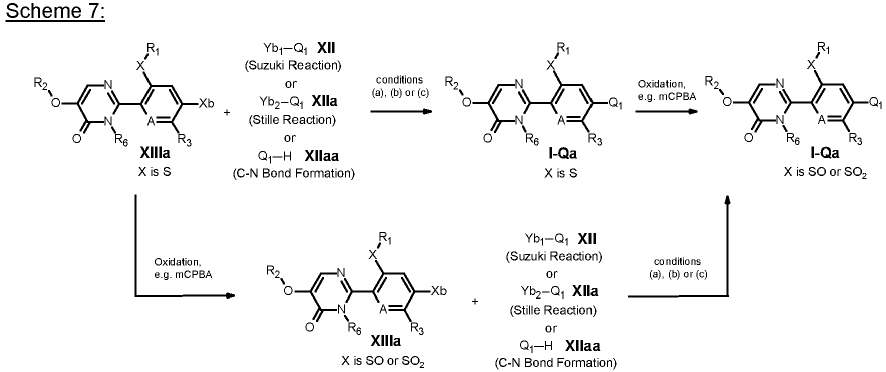
(a) Suzuki reaction: Pd cat. (e.g. Pd(PPh₃)₄ or Pd(dppf)Cl₂), base (e.g. Na₂CO₃), solvent (e.g. 1,2-dimethoxyethane / water), 25-180°C.
(b) Stille reaction: Pd cat. (e.g. Pd(PPh₃)₄ or Pd(PPh₃)Cl₂), solvent (e.g. toluene), 25-180°C.
(c) C-N bond formation: Optional base (e.g. K₂CO₃ or Cs₂CO₃), optional presence of copper or palladium catalyst, optional additive (such as N,N'-dimethylethylenediamine), optional ligand (such as Xantphos), solvent (e.g. dioxane, pyridine or N,N-dimethylformamide DMF), 25-180°C.

The chemistry described previously in scheme 4 to access compounds of formula I-Qb from compounds of formula Xlllb, can be applied analogously (scheme 7) for the preparation of compounds of formula I-Qa from compounds of formula Xllla, wherein all substituent definitions mentioned previously remain valid.

The chemistry described previously in scheme 5 to access compounds of formula Xlllb from compounds of formula IV-1b and compounds of formula V (including the alternative involving IVa-1b), can be applied analogously (scheme 8) for the preparation of compounds of formula Xllla from compounds of formula IV-1a (including the alternative involving IVa-1a), wherein all substituent definitions mentioned previously remain valid.

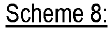

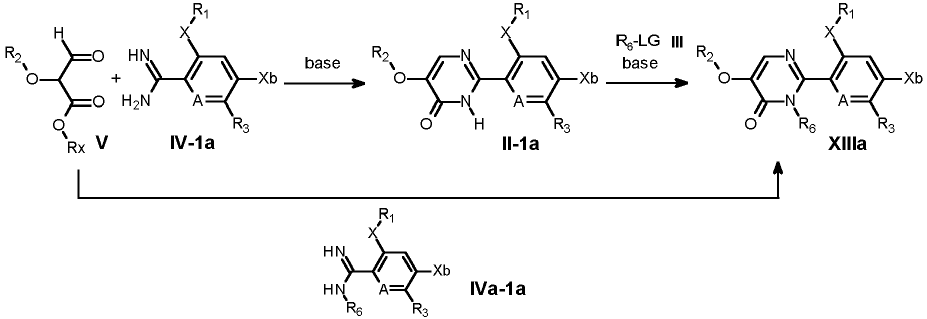

The chemistry described previously in scheme 6 to access compounds of formula IV-1b and compounds of formula IVa-1b from compounds of formula VI-1b can be applied analogously (scheme 9) for the preparation of compounds of formula IV-1a and compounds of formula IVa-1a from compounds of formula VI-1a, wherein all substituent definitions mentioned previously remain valid.

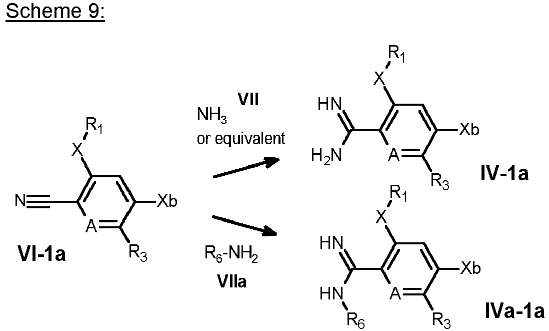

Compounds of formula VI, wherein Q is as defined in formula I; and
compounds of formula VI-1a, wherein A, X, R₁ and R₃ are as defined in formula I, and in which Xb is a leaving group like, for example, chlorine, bromine or iodine (preferably chlorine or bromine), or an aryl- or alkylsulfonate such as trifluoromethanesulfonate; and
compounds of formula VI-1b, wherein A, X, R₁ and R₃ are as defined in formula I, and in which Xb is a leaving group like, for example, chlorine, bromine or iodine (preferably chlorine or bromine), or an aryl- or alkylsulfonate such as trifluoromethanesulfonate;
are either known, commercially available or may be prepared by methods known to a person skilled in the art. See for example: 3-(ethylthio)-2-pyridinecarbonitrile [CAS 342816-54-0], 3-(ethylthio)-5-(trifluoromethyl)-2-pyridinecarbonitrile [CAS 1421952-04-6], 5-cyclopropyl-3-(ethylthio)-2-pyridine-carbonitrile [CAS 2173047-82-8], 6-chloro-3-ethylsulfanyl-pyridine-2-carbonitrile [CAS 102645-27-2] or 5-bromo-3-(ethylthio)-2-pyridinecarbonitrile [1879106-77-0].

Another subgroup of compounds of formula I, wherein R₂ and R₆ are as defined above and wherein Q is defined as Qa, in which A, R₃, X and R₁ are as defined in formula I, and Q₁ represents -C(Rₐₐ)(R_{ab})(CN), wherein Rₐₐ and R_{ab} are independently hydrogen or C₁-C₃ alkyl, or wherein Rₐₐ and R_{ab}, together with the carbon atom to which they are attached, form a 3- to 5-membered carbocyclic ring, may be defined as compounds of formula I-Qaa (scheme 10).

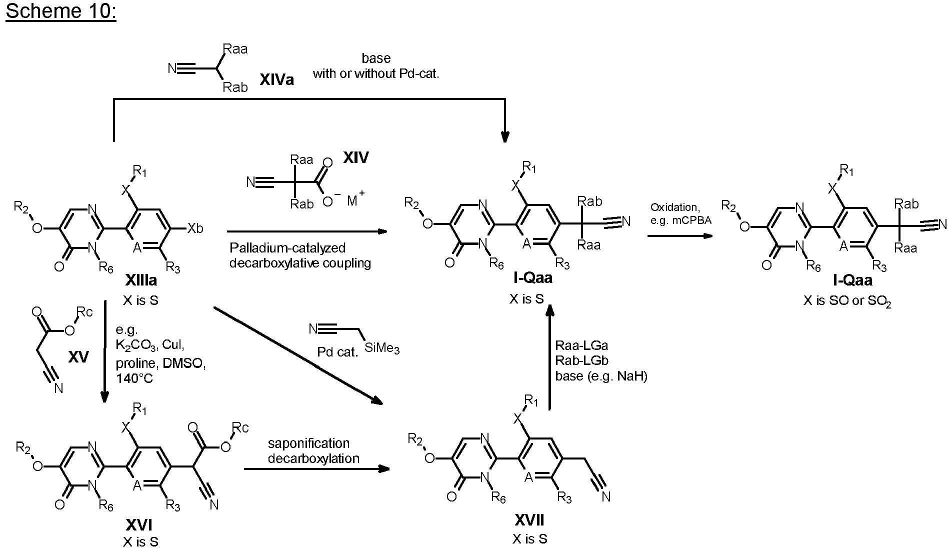

Methods and conditions to access such compounds of formula I-Qaa as defined above are shown in scheme 10. Compounds of formula I-Qaa, wherein X is S (sulfide) and in which the other substituent definitions are as defined above, can be prepared by a palladium-catalyzed decarboxylative coupling between compounds of formula XIV, wherein Rₐₐ and R_{ab} are as defined here above, and M⁺ is a cation, such as a sodium or potassium ion, with compounds of formula Xllla, wherein A, R₃, R₁, R₂ and R₆ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, under conditions described in the literature (see for example Lei Liu et al; Angew. Chem. Int. Ed. (2011), 50, 4470-4474). Compounds of formula XIV may be commercially available or can be made in analogy to literature precedent (for example by hydrolysis of cyanoacetates).

Alternatively, compounds of formula I-Qaa, wherein X is S (sulfide) and in which the other substituent definitions are as defined above, can be prepared from compounds of formula Xllla by treatment with compounds of formula XIVa, wherein Rₐₐ and R_{ab} are as defined above, in the presence or absence of a catalyst such as Pd₂(dba)₃, and a ligand, such as BINAP, using a strong base such as lithium hexamethyldisilazane (LiHMDS), in an inert solvent such as tetrahydrofuran, at temperatures between - 20 and 120 °C. Such methods have been described in the literature, for example, Org. Lett. 2014, 16, 6314-6317; J. Org. Chem. 2005, 70, 10186-10189 or Org. Lett. 2011, 13, 1690-1693. Compounds of formula XIVa may be commercially available or can be made in analogy to literature precedent.

Alternatively, compounds of formula I-Qaa, wherein X is S (sulfide) and in which the other substituent definitions are as defined above, can be obtained by sequential treatment of compound of formula XVII, wherein A, R₃, R₁, R₂ and R₆ are as defined in formula I, and in which X is S (sulfide), with alkylating reagents of general formula Rₐₐ-LGₐ and R_{ab}-LG_{b}, wherein LGₐ and LGb are leaving groups such as halides or sulfonates (preferably Br, I, OSOsMe or OTf), and in which Rₐₐ and R_{ab} are as defined above, in the presence of a base, such as sodium hydride, K₂CO₃, or Cs₂CO₃, in an inert solvent such as tetrahydrofuran, N,N-dimethylformamide, 1,4-dioxane or acetonitrile. Alternatively, compounds of formula XVII can be subjected to an excess of the abovementioned base and reagent Rₐₐ-LGₐ to obtain compounds of formula I-Qaa, wherein Rₐₐ = R_{ab}. Alternatively, in the particular situation wherein Rₐₐ and R_{ab}, together with the carbon atom to which they are attached, form a 3- to 5-membered carbocyclic ring, compounds of formula XVII can be treated with alkylating reagents of the formula LGₐ-Rₐₐ-R_{ab}-LG_{b}, in the presence of the abovementioned base and solvent. 1 ,2-Dibromo-ethane or 1-bromo-3-chloropropane are examples of such reagents.

Compound of formula XVII, wherein A, R₃, R₁, R₂ and R₆ are as defined in formula I, and in which X is S (sulfide), can be prepared by treating compounds of formula Xllla, wherein A, R₃, R₁, R₂ and R₆ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, with the reagent trimethylsilyl-acetonitrile, in the presence of zinc(ll)fluoride, and a palladium(0) catalyst such as tris(dibenzylideneacetone)dipalladium(0)-chloroform adduct (Pd₂(dba)₃), a ligand, for example Xantphos, in an inert solvent, such as DMF at temperatures between 100-160 °C, optionally under microwave heating, under conditions described in the literature, for example, J. Am. Chem. Soc. 2005, 127, 15824-15832. Alternatively, compounds of formula XVII can be obtained from compounds of formula XVI, wherein A, R₃, R₁, R₂ and R₆ are as defined in formula I, and in which X is S (sulfide) and Rc is C₁-C₆alkyl (preferably methyl or ethyl) via saponification/decarboxylation of the ester group C(O)ORc, for example via Krapcho decarboxylation. Such methods (e.g. heating the substrate XVI in the presence of sodium or lithium chloride in aqueous dimethylsulfoxide DMSO) have been described, for example in, Tetradehron Lett. 1974, 15, 1091-1094. Compounds of formula XVI, wherein A, R₃, R₁, R₂ and R₆ are as defined in formula I, and in which X is S (sulfide) and Rc is C₁-C₆alkyl (preferably methyl or ethyl), can be obtained by reaction of compounds of formula Xllla (substituent defined as above) with cyanoacetates of general formula XV, wherein Rc is C₁-C₆alkyl (preferably methyl or ethyl). This reaction may proceed in polar aprotic solvents such as dimethylsulfoxide (DMSO), N,N-dimethylformamide (DMF), or N-methylpyrrolidone (NMP), in the presence of a base, such as K₂CO₃ or Na₂CO₃, optionally in the presence of a copper salt (such as Cul), optionally in the presence of an additive (such as N,N'-dimethylethylenediamine or proline), in the presence of absence of a phase-transfer catalyst ("PTC"), at temperatures between 20-180 °C, preferably 80-150 °C. One example of such reaction conditions is heating the substrate Xllla with ethyl cyanoacetate in the presence of potassium carbonate, and a catalytic amount of copper(I) iodide and proline in dimethylsulfoxide at 140°C. Similarly, transition metal-catalyzed processes are known, see for example, Angew. Chem. Int. Ed. 2011, 50, 4470-4474.

The subgroup of compounds of formula I, wherein Q is defined as Qa in which X is S and A, R₃ and R₁ are as defined in formula I, and wherein Q₁ is 2-pyridyloxy, may be defined as compounds of formula XX (scheme 11).

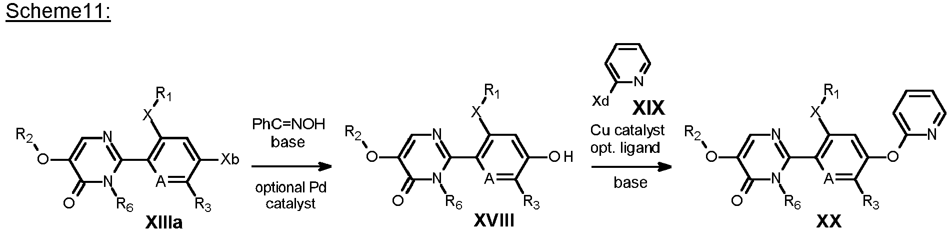

Compound of formula XX, wherein R₂, R₆, A, R₃ and R₁ are as defined in formula I, and in which X is S, can be prepared (scheme 11) by reacting compounds of formula XVIII, wherein R₂, R₆, A, R₃ and R₁ are as defined in formula I, and in which X is S, with compounds of formula XIX, wherein Xd is a leaving group such as, for example, chlorine, bromine or iodine (preferably bromine or iodine), or an aryl-, alkyl- or haloalkylsulfonate such as trifluoromethanesulfonate, in presence of a base such as, for example, sodium hydride, potassium or cesium carbonate, in a suitable solvent such as dioxane, acetonitrile, N,N-dimethylformamide or N,N-dimethylacetamide, in the presence of a copper catalyst, for example copper(I) iodide, optionally in the presence of an additional ligand, at temperatures between 20°C and 200°C, preferably at temperatures ranging from room temperature to the boiling point of the reaction mixture, optionally under microwave irradiation.

Compounds of formula XVIII, wherein R₂, R₆, A, R₃ and R₁ are as defined in formula I, and in which X is S, can be prepared by reacting compounds of formula Xllla, wherein A, R₃, R₁, R₂ and R₆ are as defined in formula I, and in which X is S (sulfide), and wherein Xb is a leaving group like, for example, chlorine, bromine or iodine (preferably chlorine or bromine), or an aryl- or alkylsulfonate such as trifluoromethanesulfonate, with for example benzaldoxime PhC=NOH, preferably (E)-benzaldehyde oxime, in the presence of a base, such as potassium or cesium carbonate, optionally in the presence of a palladium catalyst such as RockPhos-G3-palladacycle ( [(2-Di-*tert*-butylphosphino-3-methoxy-6-methyl-2',4',6'-triisopropyl-1,1'-biphenyl)-2-(2-aminobiphenyl)]palladium(II) methanesulfonate), in an aprotic solvent such as acetonitrile or N,N-dimethylformamide DMF, at temperatures between 0 and 100°C, preferably between room temperature and 80°C, as described, for example, in Angew. Chem. Int. Ed. 56 (16), 4478-4482, 2017.

Compounds of formula XIX (substituents as defined above) are either known compounds, commercially available or can be prepared by known methods.

The reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reactions are advantageously carried out in a temperature range from approximately -80°C to approximately +140°C, preferably from approximately -30°C to approximately +100°C, in many cases in the range between ambient temperature and approximately +80°C.

A compound of formula I can be converted in a manner known per se into another compound of formula I by replacing one or more substituents of the starting compound of formula I in the customary manner by (an)other substituent(s) according to the invention, and by post modification of compounds of with reactions such as oxidation, alkylation, reduction, acylation and other methods known by those skilled in the art.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula I can be prepared in a manner known per se. Thus, for example, acid addition salts of compounds of formula I are obtained by treatment with a suitable acid or a suitable ion exchanger reagent and salts with bases are obtained by treatment with a suitable base or with a suitable ion exchanger reagent.

Salts of compounds of formula I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds of formula I can be converted in a manner known per se into other salts of compounds of formula I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula I, which have salt-forming properties can be obtained in free form or in the form of salts.

The compounds of formula I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds of formula I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

N-oxides can be prepared by reacting a compound of the formula I with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 2000/15615.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds of formula I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds according to the following Tables A-1 to A-81 and Tables B-1 to B-54 below can be prepared according to the methods described above. The examples which follow are intended to illustrate the invention and show preferred compounds of formula I.

The tables A-1 to A-81 below illustrate specific compounds of the invention.

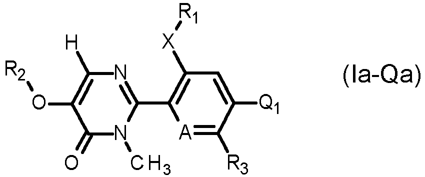

**Table Y: Substituent definitions of Q₁**

| **Index** | **Q₁** | **Index** | **Q₁** |
|---|---|---|---|
| **1** | H | **11** | -N(CH₃)COCH₃ |
| **2** | -N(CH₃)COCH₂CH₃ | **12** | 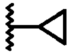 |
| **3** | Cl | **13** | 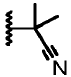 |
| **4** | Br | **14** | 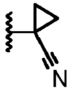 |
| **5** | -N(CH₃)COcycloC3 | **15** | -OCH₂CF₃ |
| **6** | CF₃ | **16** | 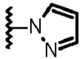 |
| **7** | -NH₂ | **17** | 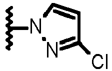 |
| **8** | -NH(CH₃) | **18** | 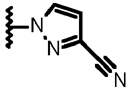 |
| **9** | -N(CH₃)₂ | **19** | 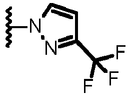 |
| **10** | -NHCOCH₃ | **20** | 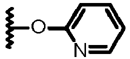 |

In the table Y and in tables A, "cycloC3" represents cyclopropyl.
Table A-1 provides 20 compounds A-1.001 to A-1.020 of formula la-Qa wherein R₁ is CH₂CH₃, R₂ is CH₂CF₂CF₃, R₃ is H, X is S, A is N and Q₁ is as defined in table Y.
Table A-2 provides 20 compounds A-2.001 to A-2.020 of formula la-Qa wherein R₁ is CH₂CH₃, R₂ is CH₂CF₂CF₃, R₃ is H, X is SO, A is N and Q₁ is as defined in table Y.
Table A-3 provides 20 compounds A-3.001 to A-3.020 of formula la-Qa wherein R₁ is CH₂CH₃, R₂ is CH₂CF₂CF₃, R₃ is H, X is SO₂, A is N and Q₁ is as defined in table Y.
Table A-4 provides 20 compounds A-4.001 to A-4.020 of formula la-Qa wherein R₁ is CH₂CH₃, R₂ is CH₂CF₂CHF₂, R₃ is H, X is S, A is N and Q₁ is as defined in table Y.
Table A-5 provides 20 compounds A-5.001 to A-5.020 of formula la-Qa wherein R₁ is CH₂CH₃, R₂ is CH₂CF₂CHF₂, R₃ is H, X is SO, A is N and Q₁ is as defined in table Y.
Table A-6 provides 20 compounds A-6.001 to A-6.020 of formula la-Qa wherein R₁ is CH₂CH₃, R₂ is CH₂CF₂CHF₂, R₃ is H, X is SO₂, A is N and Q₁ is as defined in table Y.
Table A-7 provides 20 compounds A-7.001 to A-7.020 of formula la-Qa wherein R₁ is CH₂CH₃, R₂ is CH₂CHF₂, R₃ is H, X is S, A is N and Q₁ is as defined in table Y.
Table A-8 provides 20 compounds A-8.001 to A-8.020 of formula la-Qa wherein R₁ is CH₂CH₃, R₂ is CH₂CHF₂, R₃ is H, X is SO, A is N and Q₁ is as defined in table Y.
Table A-9 provides 20 compounds A-9.001 to A-9.020 of formula la-Qa wherein R₁ is CH₂CH₃, R₂ is CH₂CHF₂, R₃ is H, X is SO₂, A is N and Q₁ is as defined in table Y.
Table A-10 provides 20 compounds A-10.001 to A-1 0.020 of formula la-Qa wherein R₁ is CH₂CH₃, R₂ is CH₂CF₃, R₃ is H, X is S, A is N and Q₁ is as defined in table Y.
Table A-11 provides 20 compounds A-11.001 to A-11.020 of formula la-Qa wherein R₁ is CH₂CH₃, R₂ is CH₂CF₃, R₃ is H, X is SO, A is N and Q₁ is as defined in table Y.
Table A-12 provides 20 compounds A-12.001 to A-12.020 of formula la-Qa wherein R₁ is CH₂CH₃, R₂ is CH₂CF₃, R₃ is H, X is SO₂, A is N and Q₁ is as defined in table Y.
Table A-13 provides 20 compounds A-13.001 to A-13.020 of formula la-Qa wherein R₁ is CH₂CH₃, R₂ is CH₂CF₂CHFCF₃, R₃ is H, X is S, A is N and Q₁ is as defined in table Y.
Table A-14 provides 20 compounds A-14.001 to A-14.020 of formula la-Qa wherein R₁ is CH₂CH₃, R₂ is CH₂CF₂CHFCF₃, R₃ is H, X is SO, A is N and Q₁ is as defined in table Y.
Table A-15 provides 20 compounds A-15.001 to A-15.020 of formula la-Qa wherein R₁ is CH₂CH₃, R₂ is CH₂CF₂CHFCF₃, R₃ is H, X is SO₂, A is N and Q₁ is as defined in table Y.
Table A-16 provides 20 compounds A-16.001 to A-16.020 of formula la-Qa wherein R₁ is CH₂CH₃, R₂ is SO₂CF₃, R₃ is H, X is S, A is N and Q₁ is as defined in table Y.
Table A-17 provides 20 compounds A-17.001 to A-17.020 of formula la-Qa wherein R₁ is CH₂CH₃, R₂ is SO₂CF₃, R₃ is H, X is SO, A is N and Q₁ is as defined in table Y.
Table A-18 provides 20 compounds A-18.001 to A-18.020 of formula la-Qa wherein R₁ is CH₂CH₃, R₂ is SO₂CF₃, R₃ is H, X is SO₂, A is N and Q₁ is as defined in table Y.
Table A-19 provides 20 compounds A-19.001 to A-19.020 of formula la-Qa wherein R₁ is CH₂CH₃, R₂ is CH₂CF₂CF₃, R₃ is H, X is S, A is CH and Q₁ is as defined in table Y.
Table A-20 provides 20 compounds A-20.001 to A-20.020 of formula la-Qa wherein R₁ is CH₂CH₃, R₂ is CH₂CF₂CF₃, R₃ is H, X is SO, A is CH and Q₁ is as defined in table Y.
Table A-21 provides 20 compounds A-21.001 to A-21.020 of formula la-Qa wherein R₁ is CH₂CH₃, R₂ is CH₂CF₂CF₃, R₃ is H, X is SO₂, A is CH and Q₁ is as defined in table Y.
Table A-22 provides 20 compounds A-22.001 to A-22.020 of formula la-Qa wherein R₁ is CH₂CH₃, R₂ is CH₂CF₂CHF₂, R₃ is H, X is S, A is CH and Q₁ is as defined in table Y.
Table A-23 provides 20 compounds A-23.001 to A-23.020 of formula la-Qa wherein R₁ is CH₂CH₃, R₂ is CH₂CF₂CHF₂, R₃ is H, X is SO, A is CH and Q₁ is as defined in table Y.
Table A-24 provides 20 compounds A-24.001 to A-24.020 of formula la-Qa wherein R₁ is CH₂CH₃, R₂ is CH₂CF₂CHF₂, R₃ is H, X is SO₂, A is CH and Q₁ is as defined in table Y.
Table A-25 provides 20 compounds A-25.001 to A-25.020 of formula la-Qa wherein R₁ is CH₂CH₃, R₂ is CH₂CHF₂, R₃ is H, X is S, A is CH and Q₁ is as defined in table Y.
Table A-26 provides 20 compounds A-26.001 to A-26.020 of formula la-Qa wherein R₁ is CH₂CH₃, R₂ is CH₂CHF₂, R₃ is H, X is SO, A is CH and Q₁ is as defined in table Y.
Table A-27 provides 20 compounds A-27.001 to A-27.020 of formula la-Qa wherein R₁ is CH₂CH₃, R₂ is CH₂CHF₂, R₃ is H, X is SO₂, A is CH and Q₁ is as defined in table Y.
Table A-28 provides 20 compounds A-28.001 to A-28.020 of formula la-Qa wherein R₁ is CH₂CH₃, R₂ is CH₂CF₃, R₃ is H, X is S, A is CH and Q₁ is as defined in table Y.
Table A-29 provides 20 compounds A-29.001 to A-29.020 of formula la-Qa wherein R₁ is CH₂CH₃, R₂ is CH₂CF₃, R₃ is H, X is SO, A is CH and Q₁ is as defined in table Y.
Table A-30 provides 20 compounds A-30.001 to A-30.020 of formula la-Qa wherein R₁ is CH₂CH₃, R₂ is CH₂CF₃, R₃ is H, X is SO₂, A is CH and Q₁ is as defined in table Y.
Table A-31 provides 20 compounds A-31.001 to A-31.020 of formula la-Qa wherein R₁ is CH₂CH₃, R₂ is CH₂CF₂CHFCF₃, R₃ is H, X is S, A is CH and Q₁ is as defined in table Y.
Table A-32 provides 20 compounds A-32.001 to A-32.020 of formula la-Qa wherein R₁ is CH₂CH₃, R₂ is CH₂CF₂CHFCF₃, R₃ is H, X is SO, A is CH and Q₁ is as defined in table Y.
Table A-33 provides 20 compounds A-33.001 to A-33.020 of formula la-Qa wherein R₁ is CH₂CH₃, R₂ is CH₂CF₂CHFCF₃, R₃ is H, X is SO₂, A is CH and Q₁ is as defined in table Y.
Table A-34 provides 20 compounds A-34.001 to A-34.020 of formula la-Qa wherein R₁ is CH₂CH₃, R₂ is SO₂CF₃, R₃ is H, X is S, A is CH and Q₁ is as defined in table Y.
Table A-35 provides 20 compounds A-35.001 to A-35.020 of formula la-Qa wherein R₁ is CH₂CH₃, R₂ is SO₂CF₃, R₃ is H, X is SO, A is CH and Q₁ is as defined in table Y.
Table A-36 provides 20 compounds A-36.001 to A-36.020 of formula la-Qa wherein R₁ is CH₂CH₃, R₂ is SO₂CF₃, R₃ is H, X is SO₂, A is CH and Q₁ is as defined in table Y.
Table A-37 provides 20 compounds A-37.001 to A-37.020 of formula la-Qa wherein R₁ is CH₂CH₃, R₂ is CH₂CF₂CF₃, R₃ is CH₃, X is S, A is N and Q₁ is as defined in table Y.
Table A-38 provides 20 compounds A-38.001 to A-38.020 of formula la-Qa wherein R₁ is CH₂CH₃, R₂ is CH₂CF₂CF₃, R₃ is CH₃, X is SO, A is N and Q₁ is as defined in table Y.
Table A-39 provides 20 compounds A-39.001 to A-39.020 of formula la-Qa wherein R₁ is CH₂CH₃, R₂ is CH₂CF₂CF₃, R₃ is CH₃, X is SO₂, A is N and Q₁ is as defined in table Y.
Table A-40 provides 20 compounds A-40.001 to A-40.020 of formula la-Qa wherein R₁ is CH₂CH₃, R₂ is CH₂CF₂CHF₂, R₃ is CH₃, X is S, A is N and Q₁ is as defined in table Y.
Table A-41 provides 20 compounds A-41.001 to A-41.020 of formula la-Qa wherein R₁ is CH₂CH₃, R₂ is CH₂CF₂CHF₂, R₃ is CH₃, X is SO, A is N and Q₁ is as defined in table Y.
Table A-42 provides 20 compounds A-42.001 to A-42.020 of formula la-Qa wherein R₁ is CH₂CH₃, R₂ is CH₂CF₂CHF₂, R₃ is CH₃, X is SO₂, A is N and Q₁ is as defined in table Y.
Table A-43 provides 20 compounds A-43.001 to A-43.020 of formula la-Qa wherein R₁ is CH₂CH₃, R₂ is CH₂CHF₂, R₃ is CH₃, X is S, A is N and Q₁ is as defined in table Y.
Table A-44 provides 20 compounds A-44.001 to A-44.020 of formula la-Qa wherein R₁ is CH₂CH₃, R₂ is CH₂CHF₂, R₃ is CH₃, X is SO, A is N and Q₁ is as defined in table Y.
Table A-45 provides 20 compounds A-45.001 to A-45.020 of formula la-Qa wherein R₁ is CH₂CH₃, R₂ is CH₂CHF₂, R₃ is CH₃, X is SO₂, A is N and Q₁ is as defined in table Y.
Table A-46 provides 20 compounds A-46.001 to A-46.020 of formula la-Qa wherein R₁ is CH₂CH₃, R₂ is CH₂CF₃, R₃ is CH₃, X is S, A is N and Q₁ is as defined in table Y.
Table A-47 provides 20 compounds A-47.001 to A-47.020 of formula la-Qa wherein R₁ is CH₂CH₃, R₂ is CH₂CF₃, R₃ is CH₃, X is SO, A is N and Q₁ is as defined in table Y.
Table A-48 provides 20 compounds A-48.001 to A-48.020 of formula la-Qa wherein R₁ is CH₂CH₃, R₂ is CH₂CF₃, R₃ is CH₃, X is SO₂, A is N and Q₁ is as defined in table Y.
Table A-49 provides 20 compounds A-49.001 to A-49.020 of formula la-Qa wherein R₁ is CH₂CH₃, R₂ is CH₂CF₂CHFCF₃, R₃ is CH₃, X is S, A is N and Q₁ is as defined in table Y.
Table A-50 provides 20 compounds A-50.001 to A-50.020 of formula la-Qa wherein R₁ is CH₂CH₃, R₂ is CH₂CF₂CHFCF₃, R₃ is CH₃, X is SO, A is N and Q₁ is as defined in table Y.
Table A-51 provides 20 compounds A-51.001 to A-51.020 of formula la-Qa wherein R₁ is CH₂CH₃, R₂ is CH₂CF₂CHFCF₃, R₃ is CH₃, X is SO₂, A is N and Q₁ is as defined in table Y.
Table A-52 provides 20 compounds A-52.001 to A-52.020 of formula la-Qa wherein R₁ is CH₂CH₃, R₂ is SO₂CF₃, R₃ is CH₃, X is S, A is N and Q₁ is as defined in table Y.
Table A-53 provides 20 compounds A-53.001 to A-53.020 of formula la-Qa wherein R₁ is CH₂CH₃, R₂ is SO₂CF₃, R₃ is CH₃, X is SO, A is N and Q₁ is as defined in table Y.
Table A-54 provides 20 compounds A-54.001 to A-54.020 of formula la-Qa wherein R₁ is CH₂CH₃, R₂ is SO₂CF₃, R₃ is CH₃, X is SO₂, A is N and Q₁ is as defined in table Y.
Table A-55 provides 20 compounds A-55.001 to A-55.020 of formula la-Qa wherein R₁ is - CH₂cyclopropyl, R₂ is CH₂CF₂CF₃, R₃ is H, X is S, A is N and Q₁ is as defined in table Y.
Table A-56 provides 20 compounds A-56.001 to A-56.020 of formula la-Qa wherein R₁ is - CH₂cyclopropyl, R₂ is CH₂CF₂CF₃, R₃ is H, X is SO, A is N and Q₁ is as defined in table Y.
Table A-57 provides 20 compounds A-57.001 to A-57.020 of formula la-Qa wherein R₁ is - CH₂cyclopropyl, R₂ is CH₂CF₂CF₃, R₃ is H, X is SO₂, A is N and Q₁ is as defined in table Y.
Table A-58 provides 20 compounds A-58.001 to A-58.020 of formula la-Qa wherein R₁ is - CH₂cyclopropyl, R₂ is CH₂CF₂CHF₂, R₃ is H, X is S, A is N and Q₁ is as defined in table Y.
Table A-59 provides 20 compounds A-59.001 to A-59.020 of formula la-Qa wherein R₁ is - CH₂cyclopropyl, R₂ is CH₂CF₂CHF₂, R₃ is H, X is SO, A is N and Q₁ is as defined in table Y.
Table A-60 provides 20 compounds A-60.001 to A-60.020 of formula la-Qa wherein R₁ is - CH₂cyclopropyl, R₂ is CH₂CF₂CHF₂, R₃ is H, X is SO₂, A is N and Q₁ is as defined in table Y.
Table A-61 provides 20 compounds A-61.001 to A-61.020 of formula la-Qa wherein R₁ is - CH₂cyclopropyl, R₂ is CH₂CF₃, R₃ is H, X is S, A is N and Q₁ is as defined in table Y.
Table A-62 provides 20 compounds A-62.001 to A-62.020 of formula la-Qa wherein R₁ is - CH₂cyclopropyl, R₂ is CH₂CF₃, R₃ is H, X is SO, A is N and Q₁ is as defined in table Y.
Table A-63 provides 20 compounds A-63.001 to A-63.020 of formula la-Qa wherein R₁ is - CH₂cyclopropyl, R₂ is CH₂CF₃, R₃ is H, X is SO₂, A is N and Q₁ is as defined in table Y.
Table A-64 provides 20 compounds A-64.001 to A-64.020 of formula la-Qa wherein R₁ is - CH₂cyclopropyl, R₂ is CH₂CF₂CF₃, R₃ is H, X is S, A is CH and Q₁ is as defined in table Y.
Table A-65 provides 20 compounds A-65.001 to A-65.020 of formula la-Qa wherein R₁ is - CH₂cyclopropyl, R₂ is CH₂CF₂CF₃, R₃ is H, X is SO, A is CH and Q₁ is as defined in table Y.
Table A-66 provides 20 compounds A-66.001 to A-66.020 of formula la-Qa wherein R₁ is - CH₂cyclopropyl, R₂ is CH₂CF₂CF₃, R₃ is H, X is SO₂, A is CH and Q₁ is as defined in table Y.
Table A-67 provides 20 compounds A-67.001 to A-67.020 of formula la-Qa wherein R₁ is - CH₂cyclopropyl, R₂ is CH₂CF₂CHF₂, R₃ is H, X is S, A is CH and Q₁ is as defined in table Y.
Table A-68 provides 20 compounds A-68.001 to A-68.020 of formula la-Qa wherein R₁ is - CH₂cyclopropyl, R₂ is CH₂CF₂CHF₂, R₃ is H, X is SO, A is CH and Q₁ is as defined in table Y.
Table A-69 provides 20 compounds A-69.001 to A-69.020 of formula la-Qa wherein R₁ is - CH₂cyclopropyl, R₂ is CH₂CF₂CHF₂, R₃ is H, X is SO₂, A is CH and Q₁ is as defined in table Y.
Table A-70 provides 20 compounds A-70.001 to A-70.020 of formula la-Qa wherein R₁ is - CH₂cyclopropyl, R₂ is CH₂CF₃, R₃ is H, X is S, A is CH and Q₁ is as defined in table Y.
Table A-71 provides 20 compounds A-71.001 to A-71.020 of formula la-Qa wherein R₁ is - CH₂cyclopropyl, R₂ is CH₂CF₃, R₃ is H, X is SO, A is CH and Q₁ is as defined in table Y.
Table A-72 provides 20 compounds A-72.001 to A-72.020 of formula la-Qa wherein R₁ is - CH₂cyclopropyl, R₂ is CH₂CF₃, R₃ is H, X is SO₂, A is CH and Q₁ is as defined in table Y.
Table A-73 provides 20 compounds A-73.001 to A-73.020 of formula la-Qa wherein R₁ is - CH₂cyclopropyl, R₂ is CH₂CF₂CF₃, R₃ is CH₃, X is S, A is N and Q₁ is as defined in table Y.
Table A-74 provides 20 compounds A-74.001 to A-74.020 of formula la-Qa wherein R₁ is - CH₂cyclopropyl, R₂ is CH₂CF₂CF₃, R₃ is CH₃, X is SO, A is N and Q₁ is as defined in table Y.
Table A-75 provides 20 compounds A-75.001 to A-75.020 of formula la-Qa wherein R₁ is - CH₂cyclopropyl, R₂ is CH₂CF₂CF₃, R₃ is CH₃, X is SO₂, A is N and Q₁ is as defined in table Y.
Table A-76 provides 20 compounds A-76.001 to A-76.020 of formula la-Qa wherein R₁ is - CH₂cyclopropyl, R₂ is CH₂CF₂CHF₂, R₃ is CH₃, X is S, A is N and Q₁ is as defined in table Y.
Table A-77 provides 20 compounds A-77.001 to A-77.020 of formula la-Qa wherein R₁ is - CH₂cyclopropyl, R₂ is CH₂CF₂CHF₂, R₃ is CH₃, X is SO, A is N and Q₁ is as defined in table Y.
Table A-78 provides 20 compounds A-78.001 to A-78.020 of formula la-Qa wherein R₁ is - CH₂cyclopropyl, R₂ is CH₂CF₂CHF₂, R₃ is CH₃, X is SOz, A is N and Q₁ is as defined in table Y.
Table A-79 provides 20 compounds A-79.001 to A-79.020 of formula la-Qa wherein R₁ is - CH₂cyclopropyl, R₂ is CH₂CF₃, R₃ is CH₃, X is S, A is N and Q₁ is as defined in table Y.
Table A-80 provides 20 compounds A-80.001 to A-80.020 of formula la-Qa wherein R₁ is - CH₂cyclopropyl, R₂ is CH₂CF₃, R₃ is CH₃, X is SO, A is N and Q₁ is as defined in table Y.
Table A-81 provides 20 compounds A-81.001 to A-81.020 of formula la-Qa wherein R₁ is - CH₂cyclopropyl, R₂ is CH₂CF₃, R₃ is CH₃, X is SO₂, A is N and Q₁ is as defined in table Y.

The tables B-1 to B-54 below illustrate further specific compounds of the invention.

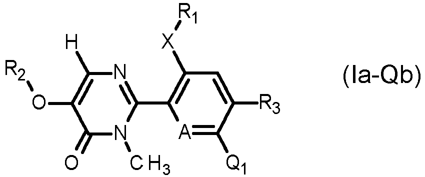

**Table Z: Substituent definitions of Q₁**

| **Index** | **Q₁** | **Index** | **Q₁** |
|---|---|---|---|
| **1** | H | **12** | 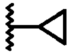 |
| **2** | Cl | **13** | -N(H)CONH₂ |
| **3** | Br | **14** | -N(H)CONH(CH₃) |
| **4** | -N(CH₃)COCH₂CH₃ | **15** | -N(H)CON(CH₃)₂ |
| **5** | -NH₂ | **16** | -N(CH₃)CONH₂ |
| **6** | -NH(CH₃) | **17** | -N(CH₃)CONH(CH₃) |
| **7** | -N(CH₃)₂ | **18** | -N(CH₃)CON(CH₃)₂ |
| **8** | -NHCOCH₃ | **19** | 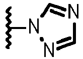 |
| **9** | -N(CH₃)COCH₃ | **20** | 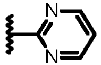 |
| **10** | -NHCOcycloC3 | **21** | 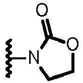 |
| **11** | -N(CH₃)COcycloC3 | | |

In the table Z and in tables B, "cycloC3" represents cyclopropyl.
Table B-1 provides 21 compounds B-1.001 to B-1.021 of formula la-Qb wherein R₁ is CH₂CH₃, R₂ is CH₂CF₂CF₃, R₃ is H, X is S, A is N and Q₁ is as defined in table Z.
Table B-2 provides 21 compounds B-2.001 to B-2.021 of formula la-Qb wherein R₁ is CH₂CH₃, R₂ is CH₂CF₂CF₃, R₃ is H, X is SO, A is N and Q₁ is as defined in table Z.
Table B-3 provides 21 compounds B-3.001 to B-3.021 of formula la-Qb wherein R₁ is CH₂CH₃, R₂ is CH₂CF₂CF₃, R₃ is H, X is SO₂, A is N and Q₁ is as defined in table Z.
Table B-4 provides 21 compounds B-4.001 to B-4.021 of formula la-Qb wherein R₁ is CH₂CH₃, R₂ is CH₂CF₂CHF₂, R₃ is H, X is S, A is N and Q₁ is as defined in table Z.
Table B-5 provides 21 compounds B-5.001 to B-5.021 of formula la-Qb wherein R₁ is CH₂CH₃, R₂ is CH₂CF₂CHF₂, R₃ is H, X is SO, A is N and Q₁ is as defined in table Z.
Table B-6 provides 21 compounds B-6.001 to B-6.021 of formula la-Qb wherein R₁ is CH₂CH₃, R₂ is CH₂CF₂CHF₂, R₃ is H, X is SO₂, A is N and Q₁ is as defined in table Z.
Table B-7 provides 21 compounds B-7.001 to B-7.021 of formula la-Qb wherein R₁ is CH₂CH₃, R₂ is CH₂CHF₂, R₃ is H, X is S, A is N and Q₁ is as defined in table Z.
Table B-8 provides 21 compounds B-8.001 to B-8.021 of formula la-Qb wherein R₁ is CH₂CH₃, R₂ is CH₂CHF₂, R₃ is H, X is SO, A is N and Q₁ is as defined in table Z.
Table B-9 provides 21 compounds B-9.001 to B-9.021 of formula la-Qb wherein R₁ is CH₂CH₃, R₂ is CH₂CHF₂, R₃ is H, X is SO₂, A is N and Q₁ is as defined in table Z.
Table B-10 provides 21 compounds B-10.001 to B-10.021 of formula la-Qb wherein R₁ is CH₂CH₃, R₂ is CH₂CF₃, R₃ is H, X is S, A is N and Q₁ is as defined in table Z.
Table B-11 provides 21 compounds B-11.001 to B-11.021 of formula la-Qb wherein R₁ is CH₂CH₃, R₂ is CH₂CF₃, R₃ is H, X is SO, A is N and Q₁ is as defined in table Z.
Table B-12 provides 21 compounds B-12.001 to B-12.021 of formula la-Qb wherein R₁ is CH₂CH₃, R₂ is CH₂CF₃, R₃ is H, X is SO₂, A is N and Q₁ is as defined in table Z.
Table B-13 provides 21 compounds B-13.001 to B-13.021 of formula la-Qb wherein R₁ is CH₂CH₃, R₂ is CH₂CF₂CHFCF₃, R₃ is H, X is S, A is N and Q₁ is as defined in table Z.
Table B-14 provides 21 compounds B-14.001 to B-14.021 of formula la-Qb wherein R₁ is CH₂CH₃, R₂ is CH₂CF₂CHFCF₃, R₃ is H, X is SO, A is N and Q₁ is as defined in table Z.
Table B-15 provides 21 compounds B-15.001 to B-15.021 of formula la-Qb wherein R₁ is CH₂CH₃, R₂ is CH₂CF₂CHFCF₃, R₃ is H, X is SO₂, A is N and Q₁ is as defined in table Z.
Table B-16 provides 21 compounds B-16.001 to B-16.021 of formula la-Qb wherein R₁ is CH₂CH₃, R₂ is SO₂CF₃, R₃ is H, X is S, A is N and Q₁ is as defined in table Z.
Table B-17 provides 21 compounds B-17.001 to B-17.021 of formula la-Qb wherein R₁ is CH₂CH₃, R₂ is SO₂CF₃, R₃ is H, X is SO, A is N and Q₁ is as defined in table Z.
Table B-18 provides 21 compounds B-18.001 to B-18.021 of formula la-Qb wherein R₁ is CH₂CH₃, R₂ is SO₂CF₃, R₃ is H, X is SO₂, A is N and Q₁ is as defined in table Z.
Table B-19 provides 21 compounds B-19.001 to B-19.021 of formula la-Qb wherein R₁ is CH₂CH₃, R₂ is CH₂CF₂CF₃, R₃ is H, X is S, A is CH and Q₁ is as defined in table Z.
Table B-20 provides 21 compounds B-20.001 to B-20.021 of formula la-Qb wherein R₁ is CH₂CH₃, R₂ is CH₂CF₂CF₃, R₃ is H, X is SO, A is CH and Q₁ is as defined in table Z.
Table B-21 provides 21 compounds B-21.001 to B-21.021 of formula la-Qb wherein R₁ is CH₂CH₃, R₂ is CH₂CF₂CF₃, R₃ is H, X is SOz, A is CH and Q₁ is as defined in table Z.
Table B-22 provides 21 compounds B-22.001 to B-22.021 of formula la-Qb wherein R₁ is CH₂CH₃, R₂ is CH₂CF₂CHF₂, R₃ is H, X is S, A is CH and Q₁ is as defined in table Z.
Table B-23 provides 21 compounds B-23.001 to B-23.021 of formula la-Qb wherein R₁ is CH₂CH₃, R₂ is CH₂CF₂CHF₂, R₃ is H, X is SO, A is CH and Q₁ is as defined in table Z.
Table B-24 provides 21 compounds B-24.001 to B-24.021 of formula la-Qb wherein R₁ is CH₂CH₃, R₂ is CH₂CF₂CHF₂, R₃ is H, X is SOz, A is CH and Q₁ is as defined in table Z.
Table B-25 provides 21 compounds B-25.001 to B-25.021 of formula la-Qb wherein R₁ is CH₂CH₃, R₂ is CH₂CHF₂, R₃ is H, X is S, A is CH and Q₁ is as defined in table Z.
Table B-26 provides 21 compounds B-26.001 to B-26.021 of formula la-Qb wherein R₁ is CH₂CH₃, R₂ is CH₂CHF₂, R₃ is H, X is SO, A is CH and Q₁ is as defined in table Z.
Table B-27 provides 21 compounds B-27.001 to B-27.021 of formula la-Qb wherein R₁ is CH₂CH₃, R₂ is CH₂CHF₂, R₃ is H, X is SOz, A is CH and Q₁ is as defined in table Z.
Table B-28 provides 21 compounds B-28.001 to B-28.021 of formula la-Qb wherein R₁ is CH₂CH₃, R₂ is CH₂CF₃, R₃ is H, X is S, A is CH and Q₁ is as defined in table Z.
Table B-29 provides 21 compounds B-29.001 to B-29.021 of formula la-Qb wherein R₁ is CH₂CH₃, R₂ is CH₂CF₃, R₃ is H, X is SO, A is CH and Q₁ is as defined in table Z.
Table B-30 provides 21 compounds B-30.001 to B-30.021 of formula la-Qb wherein R₁ is CH₂CH₃, R₂ is CH₂CF₃, R₃ is H, X is SOz, A is CH and Q₁ is as defined in table Z.
Table B-31 provides 21 compounds B-31.001 to B-31.021 of formula la-Qb wherein R₁ is CH₂CH₃, R₂ is CH₂CF₂CHFCF₃, R₃ is H, X is S, A is CH and Q₁ is as defined in table Z.
Table B-32 provides 21 compounds B-32.001 to B-32.021 of formula la-Qb wherein R₁ is CH₂CH₃, R₂ is CH₂CF₂CHFCF₃, R₃ is H, X is SO, A is CH and Q₁ is as defined in table Z.
Table B-33 provides 21 compounds B-33.001 to B-33.021 of formula la-Qb wherein R₁ is CH₂CH₃, R₂ is CH₂CF₂CHFCF₃, R₃ is H, X is SOz, A is CH and Q₁ is as defined in table Z.
Table B-34 provides 21 compounds B-34.001 to B-34.021 of formula la-Qb wherein R₁ is CH₂CH₃, R₂ is SO₂CF₃, R₃ is H, X is S, A is CH and Q₁ is as defined in table Z.
Table B-35 provides 21 compounds B-35.001 to B-35.021 of formula la-Qb wherein R₁ is CH₂CH₃, R₂ is SO₂CF₃, R₃ is H, X is SO, A is CH and Q₁ is as defined in table Z.
Table B-36 provides 21 compounds B-36.001 to B-36.021 of formula la-Qb wherein R₁ is CH₂CH₃, R₂ is SO₂CF₃, R₃ is H, X is SOz, A is CH and Q₁ is as defined in table Z.
Table B-37 provides 21 compounds B-37.001 to B-37.021 of formula la-Qb wherein R₁ is - CHzcyclopropyl, R₂ is CH₂CF₂CF₃, R₃ is H, X is S, A is N and Q₁ is as defined in table Z.
Table B-38 provides 21 compounds B-38.001 to B-38.021 of formula la-Qb wherein R₁ is - CHzcyclopropyl, R₂ is CH₂CF₂CF₃, R₃ is H, X is SO, A is N and Q₁ is as defined in table Z.
Table B-39 provides 21 compounds B-39.001 to B-39.021 of formula la-Qb wherein R₁ is - CHzcyclopropyl, R₂ is CH₂CF₂CF₃, R₃ is H, X is SOz, A is N and Q₁ is as defined in table Z.
Table B-40 provides 21 compounds B-40.001 to B-40.021 of formula la-Qb wherein R₁ is - CHzcyclopropyl, R₂ is CH₂CF₂CHF₂, R₃ is H, X is S, A is N and Q₁ is as defined in table Z.
Table B-41 provides 21 compounds B-41.001 to B-41.021 of formula la-Qb wherein R₁ is - CHzcyclopropyl, R₂ is CH₂CF₂CHF₂, R₃ is H, X is SO, A is N and Q₁ is as defined in table Z.
Table B-42 provides 21 compounds B-42.001 to B-42.021 of formula la-Qb wherein R₁ is - CHzcyclopropyl, R₂ is CH₂CF₂CHF₂, R₃ is H, X is SOz, A is N and Q₁ is as defined in table Z.
Table B-43 provides 21 compounds B-43.001 to B-43.021 of formula la-Qb wherein R₁ is - CHzcyclopropyl, R₂ is CH₂CF₃, R₃ is H, X is S, A is N and Q₁ is as defined in table Z.
Table B-44 provides 21 compounds B-44.001 to B-44.021 of formula la-Qb wherein R₁ is - CHzcyclopropyl, R₂ is CH₂CF₃, R₃ is H, X is SO, A is N and Q₁ is as defined in table Z.
Table B-45 provides 21 compounds B-45.001 to B-45.021 of formula la-Qb wherein R₁ is - CHzcyclopropyl, R₂ is CH₂CF₃, R₃ is H, X is SOz, A is N and Q₁ is as defined in table Z.
Table B-46 provides 21 compounds B-46.001 to B-46.021 of formula la-Qb wherein R₁ is - CHzcyclopropyl, R₂ is CH₂CF₂CF₃, R₃ is H, X is S, A is CH and Q₁ is as defined in table Z.
Table B-47 provides 21 compounds B-47.001 to B-47.021 of formula la-Qb wherein R₁ is - CHzcyclopropyl, R₂ is CH₂CF₂CF₃, R₃ is H, X is SO, A is CH and Q₁ is as defined in table Z.
Table B-48 provides 21 compounds B-48.001 to B-48.021 of formula la-Qb wherein R₁ is - CHzcyclopropyl, R₂ is CH₂CF₂CF₃, R₃ is H, X is SOz, A is CH and Q₁ is as defined in table Z.
Table B-49 provides 21 compounds B-49.001 to B-49.021 of formula la-Qb wherein R₁ is - CH₂cyclopropyl, R₂ is CH₂CF₂CHF₂, R₃ is H, X is S, A is CH and Q, is as defined in table Z.
Table B-50 provides 21 compounds B-50.001 to B-50.021 of formula la-Qb wherein R₁ is - CH₂cyclopropyl, R₂ is CH₂CF₂CHF₂, R₃ is H, X is SO, A is CH and Q, is as defined in table Z.
Table B-51 provides 21 compounds B-51.001 to B-51.021 of formula la-Qb wherein R₁ is - CH₂cyclopropyl, R₂ is CH₂CF₂CHF₂, R₃ is H, X is SO₂, A is CH and Q, is as defined in table Z.
Table B-52 provides 21 compounds B-52.001 to B-52.021 of formula la-Qb wherein R₁ is - CH₂cyclopropyl, R₂ is CH₂CF₃, R₃ is H, X is S, A is CH and Q, is as defined in table Z.
Table B-53 provides 21 compounds B-53.001 to B-53.021 of formula la-Qb wherein R₁ is - CH₂cyclopropyl, R₂ is CH₂CF₃, R₃ is H, X is SO, A is CH and Q, is as defined in table Z.
Table B-54 provides 21 compounds B-54.001 to B-54.021 of formula la-Qb wherein R₁ is - CH₂cyclopropyl, R₂ is CH₂CF₃, R₃ is H, X is SO₂, A is CH and Q, is as defined in table Z.

The methods referred to in the subsequent paragraphs of this description are to be interpreted as with the exception in the use in a method for treatment of the human or animal body by therapy.

The compounds of formula I according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate, a good activity corresponding to a destruction rate (mortality) of at least 50 to 60%.

Examples of the above mentioned animal pests are:
from the order *Acarina,* for example,
Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura,* for example,
Haematopinus spp., Linognathus spp., Pediculus spp., Pemphigus spp. and Phylloxera spp.;
from the order *Coleoptera,* for example,
Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.; from the order *Diptera,* for example,
Aedes spp., Anopheles spp, Antherigona soccata,Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example,
Acanthocoris scabrator, Acrosternum spp, Adelphocoris lineolatus, Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euschistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris, ;
from the order *Hymenoptera,* for example,
Acromyrmex, Arge spp, Atta spp., Cephus spp., Diprion spp., Diprionidae, Gilpinia polytoma, Hoplo-campa spp., Lasius spp., Monomorium pharaonis, Neodiprion spp., Pogonomyrmex spp, Slenopsis invicta, Solenopsis spp. and Vespa spp.;
from the order *Isoptera,* for example,
Coptotermes spp, Corniternes cumulans, Incisitermes spp, Macrotermes spp, Mastotermes spp, Microtermes spp, Reticulitermes spp.; Solenopsis geminate from the order *Lepidoptera,* for example,
Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Gra-pholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
Damalinea spp. and Trichodectes spp.;
from the order *Orthoptera,* for example,
Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera,* for example,
Liposcelis spp.;
from the order *Siphonaptera,* for example,
Ceratophyllus spp., Ctenocephalides spp. and Xenopsylla cheopis;
from the order *Thysanoptera,* for example,
Calliothrips phaseoli, Frankliniella spp., Heliothrips spp, Hercinothrips spp., Parthenothrips spp, Scirtothrips aurantii, Sericothrips variabilis, Taeniothrips spp., Thrips spp;
from the order *Thysanura,* for example, Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family and latex plants.

The compositions and/or methods of the present invention may be also used on any ornamental and/or vegetable crops, including flowers, shrubs, broad-leaved trees and evergreens.

For example the invention may be used on any of the following ornamental species: *Ageratum* spp., *Alonsoa* spp., *Anemone* spp., *Anisodontea capsenisis, Anthemis* spp., *Antirrhinum* spp., *Aster* spp., *Begonia* spp. (e.g. *B. elatior, B. semperflorens, B. tubéreux*)*, Bougainvillea* spp., *Brachycome* spp., *Brassica* spp. (ornamental), *Calceolaria* spp., *Capsicum annuum, Catharanthus roseus, Canna* spp., *Centaurea* spp., *Chrysanthemum* spp., *Cineraria* spp. (C. *maritime), Coreopsis* spp., *Crassula coccinea, Cuphea ignea, Dahlia* spp., *Delphinium* spp., *Dicentra spectabilis, Dorotheantus* spp., *Eustoma grandiflorum, Forsythia* spp., *Fuchsia* spp., *Geranium gnaphalium, Gerbera* spp., *Gomphrena globosa, Heliotropium* spp., *Helianthus* spp., *Hibiscus* spp., *Hortensia* spp., *Hydrangea* spp., *Hypoestes phyllostachya, Impatiens* spp. (*I*. *Walleriana*)*, Iresines* spp., *Kalanchoe* spp., *Lantana camara, Lavatera trimestris, Leonotis leonurus, Lilium* spp., *Mesembryanthemum* spp., *Mimulus* spp., *Monarda* spp., *Nemesia* spp., *Tagetes* spp., *Dianthus* spp. (carnation), *Canna* spp., *Oxalis* spp., *Bellis* spp., *Pelargonium* spp. *(P. peltatum, P. Zonale*)*, Viola* spp. (pansy), *Petunia* spp., *Phlox* spp., *Plecthranthus* spp., *Poinsettia* spp., *Parthenocissus* spp. *(P. quinquefolia, P. tricuspidata), Primula* spp., *Ranunculus* spp., *Rhododendron* spp., *Rosa* spp. (rose), *Rudbeckia* spp., *Saintpaulia* spp., *Salvia* spp., *Scaevola aemola, Schizanthus wisetonensis, Sedum* spp., *Solanum* spp., *Surfinia* spp., *Tagetes* spp., *Nicotinia* spp., *Verbena* spp., *Zinnia* spp. and other bedding plants.

For example the invention may be used on any of the following vegetable species: *Allium* spp. (A. *sativum, A.. cepa, A. oschaninii, A. Porrum, A. ascalonicum, A. fistulosum), Anthriscus cerefolium, Apium graveolus, Asparagus officinalis, Beta vulgarus, Brassica* spp. (B. *Oleracea, B. Pekinensis, B. rapa*)*, Capsicum annuum, Cicer arietinum, Cichorium endivia, Cichorum* spp. (C. *intybus, C. endivia), Citrillus lanatus, Cucumis* spp. (C. *sativus, C. melo*)*, Cucurbita* spp. (C. *pepo, C. maxima*)*, Cyanara* spp. (C. *scolymus, C. cardunculus*)*, Daucus carota, Foeniculum vulgare, Hypericum* spp., *Lactuca sativa, Lycopersicon* spp. (*L. esculentum, L. lycopersicum*)*, Mentha* spp., *Ocimum basilicum, Petroselinum crispum, Phaseolus* spp. (P. *vulgaris, P. coccineus*)*, Pisum sativum, Raphanus sativus, Rheum rhaponticum, Rosemarinus* spp., *Salvia* spp., *Scorzonera hispanica, Solanum melongena, Spinacea oleracea, Valerianella* spp. (*V. locusta, V. eriocarpa*) and *Vicia faba.* Preferred ornamental species include African violet, *Begonia, Dahlia, Gerbera, Hydrangea, Verbena, Rosa, Kalanchoe, Poinsettia, Aster, Centaurea, Coreopsis, Delphinium, Monarda, Phlox, Rudbeckia, Sedum, Petunia, Viola, Impatiens, Geranium, Chrysanthemum, Ranunculus, Fuchsia, Salvia, Hortensia,* rosemary, sage, St. Johnswort, mint, sweet pepper, tomato and cucumber.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds of the invention may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1Ab, Cry1Ac, Cry1F, Cry1 Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
**6. 1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603 × MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch). The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818 and EP-A-0 353 191. The methods of producing such transgenic plants are generally known to the person skilled in the art and are described, for example, in the publications mentioned above.

Crops may also be modified for enhanced resistance to fungal (for example Fusarium, Anthracnose, or Phytophthora), bacterial (for example Pseudomonas) or viral (for example potato leafroll virus, tomato spotted wilt virus, cucumber mosaic virus) pathogens.

Crops also include those that have enhanced resistance to nematodes, such as the soybean cyst nematode.

Crops that are tolerance to abiotic stress include those that have enhanced tolerance to drought, high salt, high temperature, chill, frost, or light radiation, for example through expression of NF-YB or other proteins known in the art.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Further areas of use of the compositions according to the invention are the protection of stored goods and store rooms and the protection of raw materials, such as wood, textiles, floor coverings or buildings, and also in the hygiene sector, especially the protection of humans, domestic animals and productive livestock against pests of the mentioned type.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO 2005113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera,* especially against woodborers listed in the following tables A and B:

**Table A. Examples of exotic woodborers of economic importance.**

| Family | Species | Host or Crop Infested |
|---|---|---|
| Buprestidae | *Agrilus planipennis* | Ash |
| Cerambycidae | *Anoplura glabripennis* | Hardwoods |
| Scolytidae | *Xylosandrus crassiusculus* | Hardwoods |
| | *X. mutilatus* | Hardwoods |
| | *Tomicus piniperda* | Conifers |

**Table B. Examples of native woodborers of economic importance.**

| Family | Species | Host or Crop Infested |
|---|---|---|
| Buprestidae | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | *Texania campestris* | Basswood, Beech, Maple, Oak, Sycamore, Willow, Yellow-poplar |
| Cerambycidae | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| Sesiidae | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black Cherry |
| | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

The present invention may be also used to control any insect pests that may be present in turfgrass, including for example beetles, caterpillars, fire ants, ground pearls, millipedes, sow bugs, mites, mole crickets, scales, mealybugs ticks, spittlebugs, southern chinch bugs and white grubs. The present invention may be used to control insect pests at various stages of their life cycle, including eggs, larvae, nymphs and adults.

In particular, the present invention may be used to control insect pests that feed on the roots of turfgrass including white grubs (such as *Cyclocephala spp.* (e.g. masked chafer, C. *lurida*)*, Rhizotrogus spp.* (e.g. European chafer, *R. majalis*)*, Cotinus spp.* (e.g. Green June beetle, C. *nitida*), *Popillia spp.* (e.g. Japanese beetle, *P. japonica*)*, Phyllophaga spp.* (e.g. May/June beetle), *Ataenius spp.* (e.g. Black turfgrass ataenius, A. *spretulus*)*, Maladera spp.* (e.g. Asiatic garden beetle, M. *castanea*) and *Tomarus spp.*)*,* ground pearls (*Margarodes* spp.), mole crickets (tawny, southern, and short-winged; *Scapteriscus* spp., *Gryllotalpa africana*) and leatherjackets (European crane fly, *Tipula spp.*)*.*

The present invention may also be used to control insect pests of turfgrass that are thatch dwelling, including armyworms (such as fall armyworm *Spodoptera frugiperda,* and common armyworm *Pseudaletia unipuncta*), cutworms, billbugs (*Sphenophorus spp.,* such as S. *venatus verstitus* and S. *parvulus*)*,* and sod webworms (such as *Crambus spp.* and the tropical sod webworm, *Herpetogramma phaeopteralis*)*.*

The present invention may also be used to control insect pests of turfgrass that live above the ground and feed on the turfgrass leaves, including chinch bugs (such as southern chinch bugs, *Blissus insularis*)*,* Bermudagrass mite (*Eriophyes cynodoniensis),* rhodesgrass mealybug (*Antonina graminis*)*,* two-lined spittlebug (*Propsapia bicincta*)*,* leafhoppers, cutworms (*Noctuidae* family), and greenbugs. The present invention may also be used to control other pests of turfgrass such as red imported fire ants (*Solenopsis invicta*) that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..

Of the order Mallophagida: Trimenopon spp., Menopon spp., Trinoton spp., Bovicola spp., Werneckiella spp., Lepikentron spp., Damalina spp., Trichodectes spp. and Felicola spp..

Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..

Of the order Siphonapterida, for example Pulex spp., Ctenocephalides spp., Xenopsylla spp., Ceratophyllus spp..

Of the order Heteropterida, for example Cimex spp., Triatoma spp., Rhodnius spp., Panstrongylus spp..

Of the order Blattarida, for example Blatta orientalis, Periplaneta americana, Blattelagermanica and Supella spp..

Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..

Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirexjuvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The compounds according to the invention can be used as pesticidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, *N*-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 l/ha, especially from 10 to 1000 l/ha.

Preferred formulations can have the following compositions (weight %):
Emulsifiable concentrates:

| | |
|---|---|
| active ingredient: | 1 to 95 %, preferably 60 to 90 % |

| | |
|---|---|
| surface-active agent: | 1 to 30 %, preferably 5 to 20 % |
| liquid carrier: | 1 to 80 %, preferably 1 to 35 % |

Dusts:

| | |
|---|---|
| active ingredient: | 0.1 to 10 %, preferably 0.1 to 5 % |
| solid carrier: | 99.9 to 90 %, preferably 99.9 to 99 % |

Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

Granules:

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

| Wettable powders | a) | b) | c) |
|---|---|---|---|
| active ingredients | 25 % | 50 % | 75 % |
| sodium lignosulfonate | 5% | 5 % | - |
| sodium lauryl sulfate | 3% | - | 5% |
| sodium diisobutylnaphthalenesulfonate | - | 6% | 10% |
| phenol polyethylene glycol ether (7-8 mol of ethylene oxide) | - | 2% | - |
| highly dispersed silicic acid | 5% | 10% | 10% |
| Kaolin | 62 % | 27 % | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25 % | 50 % | 75 % |
| light mineral oil | 5% | 5% | 5% |
| highly dispersed silicic acid | 5% | 5 % | - |
| Kaolin | 65 % | 40 % | - |
| Talcum | - | | 20% |

The combination is thoroughly mixed with the adjuvants and the mixture is thoroughly ground in a suitable mill, affording powders that can be used directly for seed treatment.

| Emulsifiable concentrate | |
|---|---|
| active ingredients | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5 % | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

| Extruder granules | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

| Coated granules | |
|---|---|
| Active ingredients | 8 % |
| polyethylene glycol (mol. wt. 200) | 3 % |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5 % |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

### Preparatory Examples:

"Mp" means melting point in °C. Free radicals represent methyl groups. ¹ H NMR measurements were recorded on a Brucker 400MHz spectrometer, chemical shifts are given in ppm relevant to a TMS standard. Spectra measured in deuterated solvents as indicated. Either one of the LCMS and/or GCMS methods below was used to characterize the compounds. The characteristic LCMS and GCMS values obtained for each compound were the retention time ("Rt", recorded in minutes) and the measured molecular ion (M+H)⁺ or (M-H)⁻.

### LCMS and GCMS Methods:

### Method 1:

Spectra were recorded on a Mass Spectrometer from Waters (ZQ Single quadrupole mass spectrometer) equipped with an electrospray source (Polarity: positive or negative ions, Capillary: 3.00 kV, Cone range: 30-60 V, Extractor: 2.00 V, Source Temperature: 150°C, Desolvation Temperature: 350°C, Cone Gas Flow: 0 L/Hr, Desolvation Gas Flow: 650 L/Hr, Mass range: 100 to 900 Da) and an Acquity UPLC from Waters: Binary pump, heated column compartment and diode-array detector. Solvent degasser, binary pump, heated column compartment and diode-array detector. Column: Waters UPLC HSS T3, 1.8 µm, 30 × 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH: gradient: 0 min 0% B, 100%A; 1.2-1.5min 100% B; Flow (ml/min) 0.85.

### Method 2:

Spectra were recorded on a Mass Spectrometer from Waters (SQD or ZQ Single quadrupole mass spectrometer) equipped with an electrospray source (Polarity: positive or negative ions, Capillary: 3.00 kV, Cone range: 30-60 V, Extractor: 2.00 V, Source Temperature: 150°C, Desolvation Temperature: 350°C, Cone Gas Flow: 0 L/Hr, Desolvation Gas Flow: 650 L/Hr, Mass range: 100 to 900 Da) and an Acquity UPLC from Waters: Binary pump, heated column compartment and diode-array detector. Solvent degasser, binary pump, heated column compartment and diode-array detector. Column: Waters UPLC HSS T3, 1.8 µm, 30 × 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH; gradient: 0 min 0% B, 100% A; 2.7-3.0 min 100% B; Flow (ml/min) 0.85.

### Method 3:

GCMS analyses were performed on a Thermo Electron instrument where a TRACE GC ULTRA gas chromatograph (equipped with a Zebron Phenomenex ZB-5ms 15 m, diam: 0.25 mm, 0.25 µm column; H₂ flow 1.2 mL/min, temp injector: 250°C, temp detector: 220°C; method: start at 40°C, then 40°C/min until 320°C, hold 2 min at 320°C) was linked to a DSQ mass spectrometer characterizing the compounds by chemical ionization in the positive ion mode (CI+) using methane as a reagent gas.

### Example H1: Preparation of 2-(3-ethylsulfanyl-2-pyridyl)-3-methyl-5-(2,2,3,3,3-pentafluoropropoxy)pyrimidin-4-one (compound P1)

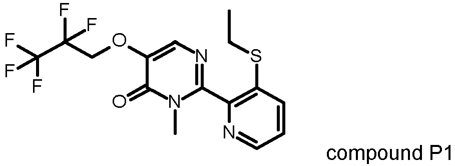

### Step 1: Preparation of methyl 2-(2,2,3,3,3-pentafluoropropoxy)acetate (intermediate 11)

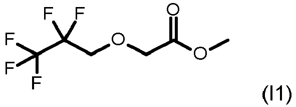

Potassium carbonate (5.42 g, 39.2 mmol, 1.2 eq.) was added to a mixture of 2,2,3,3,3-pentafluoro-1-propanol (3.95 mL, 39.2 mmol, 1.2 eq.) and methyl 2-bromoacetate (3.01 mL, 32.7 mmol) in acetonitrile (40.0 mL) at room temperature. The reaction mixture was stirred for 2 hours, then filtered. The filtrate was concentrated, diluted with water and the aqueous phase extracted twice with ethyl acetate. The combined organic layers were washed with an aqueous saturated sodium hydrogeno-carbonate solution, dried over magnesium sulfate, filtered and concentrated under vacuum. The crude yellow oil was used without further purification. ¹H NMR (400 MHz, CDCl₃) δ ppm: 3.79 (s, 3H), 4.11 (m, 2H), 4.27 (s, 2H).

### Step 2: Preparation of a mixture of ethyl and methyl 3-oxo-2-(2,2,3,3,3-pentafluoropropoxy)propanoate (intermediate I2)

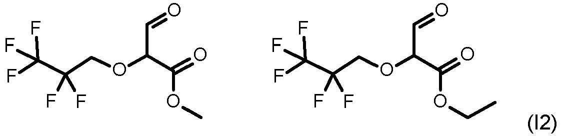

Methyl 2-(2,2,3,3,3-pentafluoropropoxy)acetate (intermediate I1 prepared above) (1.98 g, 8.91 mmol) and ethyl formate (1.25 mL, 15.2 mmol, 1.7 eq.) were added to a mixture of sodium (freshly cut, 311 mg, 13.4 mmol, 1.5 eq.) in diethyl ether (8.9 mL) under argon atmosphere. The reaction mixture was heated at reflux, generating a gas evolution when reaching 30°C. After 2 hours at reflux, the mixture was cooled to room temperature and carefully quenched with iced water. The aqueous phase was extracted twice with diethyl ether, then acidified to pH ca. 5 by addition of an aqueous 1M hydrochloric acid solution, and extracted again twice with diethyl ether. The combined organic phases were washed with a saturated sodium hydrogenocarbonate aqueous solution, dried over magnesium sulfate, filtered and concentrated under vacuum to give a crude mixture of ethyl and methyl 3-oxo-2-(2,2,3,3,3-pentafluoropropoxy)propanoate as a yellow oil, which was used without further purification. GCMS (method 3): 251 (M+H)⁺, retention time 4.11 min for the methyl ester; respectively 265 (M+H)⁺, retention time 4.40 min for the ethyl ester.

### Step 3: Preparation of 3-ethylsulfanylpyridine-2-carboxamidine (intermediate I3)

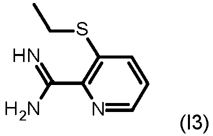

A 2.0 M trimethylaluminum solution in toluene (37.0 mL, 74.0 mmol, 1.22 eq.) was added dropwise to a suspension of ammonium hydrochloride (3.91 g, 74.0 mmol, 1.22 eq.) in toluene (100 mL) at 0°C. After stirring for 30 minutes, a solution of 3-ethylsulfanylpyridine-2-carbonitrile (prepared as described in WO14/104407) (60.9 mmol, 10.0 g) in toluene (15.0 mL) was added dropwise, and the reaction mixture was heated at 90°C and stirred overnight. After cooling to room temperature, ethyl acetate was added, and the reaction mixture was carefully quenched by dropwise addition of a 4 N sodium hydroxide aqueous solution at 0°C. The aqueous phase was extracted twice with ethyl acetate, the combined organic layers were dried over sodium sulfate, filtered and concentrated to give a first crop of the desired crude amidine I3. The aqueous phase was saturated with sodium chloride and thoroughly extracted three times with ethyl acetate and three times with dichloromethane. The combined organic layers were dried over sodium sulfate, filtered and concentrated to give another portion of crude 3-ethylsulfanylpyridine-2-carboxamidine. Both crude materials were used directly in the next step without further purification. LCMS (method 1): 182 (M+H)⁺, Rt 0.18 min.

### Step 4: Preparation of 2-(3-ethylsulfanyl-2-pyridyl)-5-(2,2,3,3,3-pentafluoropropoxy)pyrimidin-4-ol (intermediate I4)

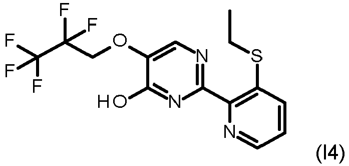

A 5.40 M sodium methanolate solution in methanol (0.562 mL, 3.03 mmol, 1.1 eq.) was added to a solution of crude mixture of ethyl and methyl 3-oxo-2-(2,2,3,3,3-pentafluoropropoxy)propanoate (intermediate I2 prepared above in step 2) (0.793 g, estimated 3.17 mmol, 1.15 eq.) and crude 3-ethylsulfanylpyridine-2-carboxamidine (intermediate I3 prepared above in step 3) (0.50 g, 2.76 mmol) in methanol (9.8 mL). The reaction mixture was heated at reflux for 2 hours. After cooling to room temperature, the mixture was quenched with acetic acid (0.19 mL, 3.31 mmol, 1.20 eq.), diluted with water, and the aqueous phase was extracted twice with ethyl acetate. The combined organic phases were washed with a saturated ammonium chloride aqueous solution, dried over magnesium sulfate, filtered and concentrated. The residue was purified by flash chromatography over silica gel (ethyl acetate in cyclohexane) to afford the desired product as a beige solid. LCMS (method 1): 382 (M+H)⁺, Rt 1.00 min.

### Step 5: Preparation of 2-(3-ethylsulfanyl-2-pyridyl)-3-methyl-5-(2,2,3,3,3-pentafluoro-propoxy)pyrimidin-4-one (compound P1)

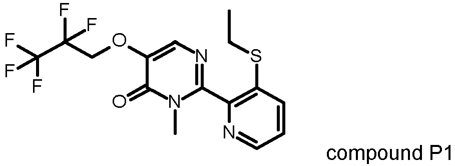

lodomethane (0.373 mL, 6.0 mmol, 4.0 eq.) was added to a suspension of 2-(3-ethylsulfanyl-2-pyridyl)-5-(2,2,3,3,3-pentafluoropropoxy)pyrimidin-4-ol (intermediate I4 prepared above) (0.572 g, 1.50 mmol) and potassium carbonate (0.303 g, 3.0 mmol, 2.0 eq.) in dimethylformamide (12.0 mL) in a microwave vial. The reaction mixture was degassed and filled with argon, the vial was caped and the reaction mixture heated at 100°C for 1 hour. After cooling to room temperature, the reaction mixture was poured into water, the aqueous phase was extracted three times with ethyl acetate, the combined organic phases were dried over magnesium sulfate, filtered and concentrated. The residue was purified by flash chromatography over silica gel (ethyl acetate in cyclohexane) to afford the desired compound P1 as an amber gum. LCMS (method 1): 396 (M+H)⁺, Rt 0.97 min.

### Example H2: Preparation of 2-(3-ethylsulfonyl-2-pyridyl)-3-methyl-5-(2,2,3,3,3-pentafluoropropoxy)pyrimidin-4-one (compound P2)

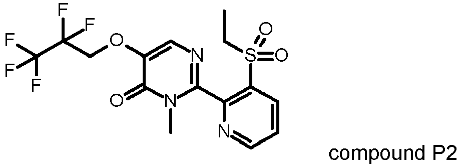

3-Chloroperbenzoic acid (0.319 g, 1.39 mmol, 2.30 eq.) was added portionwise to a 0°C cooled solution of 2-(3-ethylsulfanyl-2-pyridyl)-3-methyl-5-(2,2,3,3,3-pentafluoropropoxy)pyrimidin-4-one (compound P1 prepared as described above) (0.238 g, 0.602 mmol) in ethyl acetate (2.4 mL). After stirring for 16 hours at room temperature, the reaction mixture was quenched with a 10% w/w aqueous sodium thiosulfate solution. A saturated sodium hydrogenocarbonate aqueous solution was added after stirring for 10 minutes, and the aqueous phase was extracted twice with ethyl acetate. The combined organic phases were dried over magnesium sulfate, filtered and concentrated. The crude material was purified by flash chromatography over silica gel (ethyl acetate in cyclohexane) to give the desired product as a white solid (181 mg). LCMS (method 1): 428 (M+H)⁺, Rt 0.89 min.

### Example H3: Preparation of 2-(5-bromo-3-ethylsulfanyl-2-pyridyl)-3-methyl-5-(2,2,3,3,3-pentafluoropropoxy)pyrimidin-4-one (compound P3)

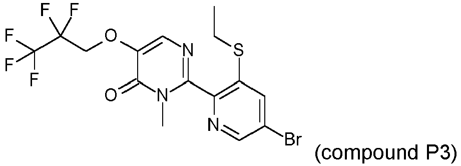

### Step 1: Preparation of 5-bromo-3-ethylsulfanyl-pyridine-2-carboxamidine (intermediate I5)

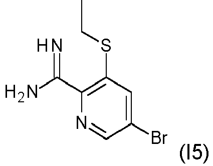

To a suspension of ammonium hydrochloride (9.35 g, 175 mmol, 2.50 equiv.) in toluene (170 mL) was added dropwise at 0 °C trimethylaluminium (2 M in toluene, 77.0 mL, 154 mmol, 2.20 equiv.). The reaction mixture was allowed to reach room temperature and kept stirring after no more gas evolution occurred. Then a solution of 5-bromo-3-ethylsulfanyl-pyridine-2-carbonitrile (CAS 1879106-77-0) (17.0 g, 69.9 mmol) in toluene (26 mL) was added to the reaction mixture. It was heated up to 90 °C and stirred overnight. After cooling down to 0 °C, methanol (150 mL) was added portionwise, followed by a mixture of methanol:water (120 mL, 4:1). It was then stirred at room temperature for 1 hour. The resulting suspension was filtered and the filtrate was concentrated to afford the desired crude amidine I5, which was used directly in the next step without further purification. LCMS (method 1): 260/262 (M+H)⁺ (bromo pattern), Rt 0.25 min.

### Step 2: Preparation of methyl 2-(2,2,3,3,3-pentafluoropropoxy)acetate (intermediate 11)

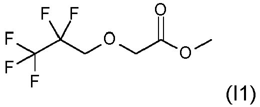

To a mixture of 2,2,3,3,3-pentafluoro-1-propanol (CAS 422-05-9) (39.5 mL, 392 mmol, 1.20 eq.) and methyl 2-bromoacetate (30.9 mL, 327 mmol) in acetonitrile (150 mL) was added at room temperature potassium carbonate (54.2 g, 392 mmol, 1.20 eq.). The reaction mixture was heated up to 50 °C and stirred overnight. After cooling down to room temperature, it was extracted three times with pentane. The combined organic layers were dried over magnesium sulfate, filtered and concentrated to afford a first portion of the desired crude I1. The acetonitrile layer was distilled off to afford a second portion of desired crude I1. Both crude materials were used directly in the next step without further purification.

¹H NMR (400 MHz, CDCl₃) δ ppm: 3.79 (s, 3H), 4.11 (m, 2H), 4.27 (s, 2H).

### Step 3: Preparation of methyl (E)-3-(dimethylamino)-2-(2,2,3,3,3-pentafluoropropoxy)prop-2-enoate (intermediate III2)

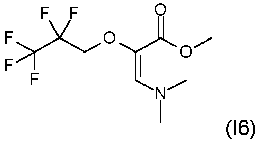

A mixture of methyl 2-(2,2,3,3,3-pentafluoropropoxy)acetate (intermediate I1 prepared as described above) (10.0 g, 45.0 mmol) and 1-tert-butoxy-N,N,N',N'-tetramethyl-methanediamine (10.2 mL, 49.5 mmol, 1.10 equiv.) was heated up to 60 °C and stirred for 2 hours. After cooling down to room temperature, the reaction mixture was diluted with water and ethyl acetate. The organic layer was washed with saturated sodium hydrogenocarbonate aqueous solution and the aqueous layer was extracted two times with ethyl acetate. The combined organic layers were dried over magnesium sulfate, filtered and concentrated to afford the desired crude I6, which was used directly in the next step without further purification. ¹H NMR (400 MHz, CDCl₃) δ ppm: 3.02-3.08 (m, 6 H), 3.70 (s, 3 H), 4.16-4.24 (m, 2 H), 6.85 (s, 1 H).

### Step 4: Preparation of methyl 3-oxo-2-(2,2,3,3,3-pentafluoropropoxy)propanoate (intermediate I7)

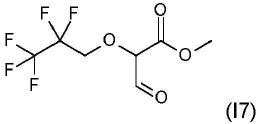

Methyl (E)-3-(dimethylamino)-2-(2,2,3,3,3-pentafluoropropoxy)prop-2-enoate (intermediate I6 prepared as described above) (5.70 g, 21.0 mmol) was dissolved in tetrahydrofuran (40 mL) and 1 M hydrochloric acid (40 mL). The reaction mixture was heated up to 45 °C and stirred for 1.5 hours. After cooling down to room temperature, the reaction mixture was diluted with water and extracted with ethyl acetate. The combined organic layers were washed with water and brine, dried over magnesium sulfate, filtered and concentrated to afford the desired crude I7, which was used directly in the next step without further purification.

### Step 5: Preparation of 2-(5-bromo-3-ethylsulfanyl-2-pyridyl)-5-(2,2,3,3,3-pentafluoropropoxy)-1H-pyrimi-din-6-one (intermediate Int-5)

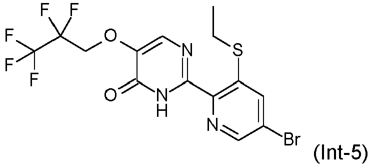

To a solution of 5-bromo-3-ethylsulfanyl-pyridine-2-carboxamidine (intermediate I5 prepared as described above) (2.60 g, 10.0 mmol) and methyl 3-oxo-2-(2,2,3,3,3-pentafluoropropoxy)propanoate (intermediate I7 prepared as described above) (4.20 g, 17.0 mmol, 1.70 equiv.) in ethanol (80 mL) was added potassium tert-butoxide (2.40 g, 20.0 mmol, 2.00 equiv.). The reaction mixture was heated up to 80 °C and stirred for 30 minutes. After cooling down to room temperature, the reaction mixture was diluted with water and extracted with ethyl acetate. The combined organic layers were washed with water and brine, dried over magnesium sulfate, filtered and concentrated under reduced pressure. The residue was purified by flash chromatography over silica gel (ethyl acetate in cyclohexane) to afford the desired product as a white/yellow solid. LCMS (method 1): 460/462 (M+H)⁺ (bromo pattern), Rt 1.13 min. ¹H NMR (400 MHz, CDCl₃) δ ppm: 1.43-1.49 (m, 3 H), 2.92-2.99 (m, 2 H), 4.76 (td, *J* = 12.84, 1.10 Hz, 2 H), 7.82 (s, 1 H), 7.95 (s, 1 H), 8.41 (d, *J* = 2.20 Hz, 1 H), 11.15 (br s, 1 H).

### Step 6: Preparation of 2-(5-bromo-3-ethylsulfanyl-2-pyridyl)-3-methyl-5-(2,2,3,3,3-pentafluoropropoxy)-pyrimidin-4-one (compound P3)

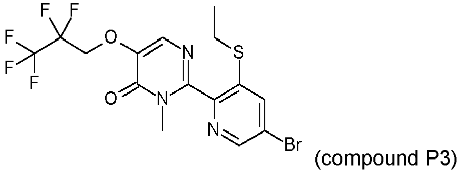

A suspension of methyl 4-methylbenzenesulfonate (1.14 mL, 7.57 mmol, 1.10 equiv.), potassium carbonate (1.39 g, 13.8 mmol, 2.00 equiv.) and 2-(5-bromo-3-ethylsulfanyl-2-pyridyl)-5-(2,2,3,3,3-pentafluoropropoxy)-1H-pyrimidin-6-one (intermediate Int-5 prepared as described above) (3.17 g, 6.88 mmol) in acetonitrile (55 mL) was stirred at room temperature overnight. The reaction mixture was directly absorbed on Isolute and purified by flash chromatography over silica gel (ethyl acetate in cyclohexane). The selected fractions were evaporated under reduced pressure and the residue was dissolved in boiling cyclohexane (20 mL). The obtained solution was cooled down to room temperature and the resulting precipitate was filtered to afford the desired product as a light yellow solid. LCMS (method 1): 474/476 (M+H)⁺ (bromo pattern), Rt 1.09 min. ¹H NMR (400 MHz, CDCl₃) δ ppm: 1.32-1.38 (m, 3 H), 2.98 (q, *J* = 7.34 Hz, 2 H), 3.38 (s, 3 H), 4.72 (td, *J* = 12.75, 0.92 Hz, 2 H), 7.82-7.84 (m, 1 H), 7.86-7.89 (m, 1 H), 8.53 (d, *J=* 1.83 Hz, 1 H).

### Example H4: Preparation of N-[5-ethylsulfanyl-6-[1-methyl-6-oxo-5-(2,2,3,3,3-pentafluoropropoxy)-pyrimidin-2-yl]-3-pyridyl]-N-methyl-acetamide (compound P4)

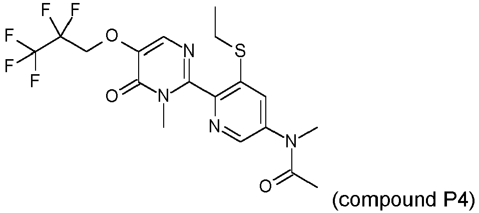

In a microwave vial, to a solution of 2-(5-bromo-3-ethylsulfanyl-2-pyridyl)-3-methyl-5-(2,2,3,3,3-pentafluoropropoxy)pyrimidin-4-one (compound P3 prepared as described above) (0.200 g, 0.422 mmol) in 1,4-dioxane (1.2 mL) were added N-methylacetamide (97.6 µL, 1.27 mmol, 3.00 equiv.), cesium carbonate (0.192 g, 0.591 mmol, 1.40 equiv.) and 4,5-bis(diphenylphosphino)-9,9-dimethylxanthene (XantPhos) (34.2 mg, 59.1 µmol, 0.140 equiv.). The vial was purged with argon for 5 minutes and then tris(dibenzylideneacetone)dipalladium(0) (3.86 mg, 4.22 µmol, 0.010 equiv.) was added. The vial was stirred under microwave irradiation at 160 °C for 1 hour. After cooling down to room temperature, the reaction mixture was diluted with dichloromethane and water. The aqueous layer was extracted twice with dichloromethane. The combined organic layers were washed with water and brine, then directly absorbed on Isolute and purified by flash chromatography over silica gel (methanol in dichloromethane) to afford the desired product as a yellow resin. LCMS (method 1): 467 (M+H)⁺, Rt 0.93 min.

### Example H5: Preparation of N-[5-ethylsulfonyl-6-[1-methyl-6-oxo-5-(2,2,3,3,3-pentafluoropropoxy)-pyrimidin-2-yll-3-pyridyll-N-methyl-acetamide (compound P5)

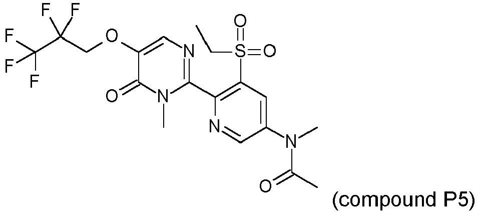

To a solution of N-[5-ethylsulfanyl-6-[1-methyl-6-oxo-5-(2,2,3,3,3-pentafluoropropoxy)pyrimidin-2-yl]-3-pyridyl]-N-methyl-acetamide (compound P4 prepared as described above) (0.204 g, 0.437 mmol) in dichloromethane (8.2 mL) was added at 0 °C 3-chloroperbenzoic acid (0.189 g, 0.875 mmol, 2.00 equiv.). The reaction mixture was stirred at 0 °C for 30 minutes, then at room temperature for 1 hour. The reaction mixture was then poured on saturated sodium hydrogenocarbonate aqueous solution and dichloromethane was added. The layers were separated, and the organic layer was washed with sodium hydroxide aqueous solution. The aqueous layers were extracted with dichloromethane. The combined organic layers were washed with 40% sodium hydrogen sulfite aqueous solution, dried over sodium sulfate, filtered and concentrated under reduced pressure. The residue was purified by flash chromatography over silica gel (methanol in dichloromethane) to afford the desired product as a yellow resin. LCMS (method 1): 499 (M+H)⁺, Rt 0.88 min. ¹H NMR (400 MHz, CDCl₃) δ ppm: 1.31-1.38 (m, 3 H), 2.15-2.33 (m, 3 H), 3.37 (s, 3 H), 3.46-3.54 (m, 5 H), 4.75 (t, *J* = 12.84 Hz, 2 H), 7.76 (s, 1 H), 8.31 (s, 1 H), 8.89 (br s, 1 H).

### Example H6: Preparation of 2-(6-chloro-3-ethylsulfanyl-2-pyridyl)-3-methyl-5-(2,2,3,3,3-pentafluoropro-poxv)pvrimidin-4-one (compound P6)

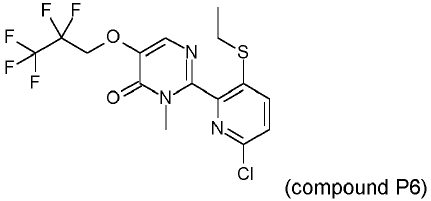

### Step 1: Preparation of 6-chloro-3-ethylsulfanyl-pyridine-2-carboxamidine (intermediate I8)

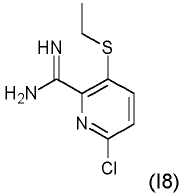

To a suspension of ammonium hydrochloride (4.81 g, 90.0 mmol, 2.50 equiv.) in toluene (72 mL) was added dropwise at 0 °C trimethylaluminium (2 M in toluene, 40.0 mL, 79.2 mmol, 2.20 equiv.). The reaction mixture was allowed to reach room temperature and stirring was continued until no more gas was evolved. Then a solution of 6-chloro-3-ethylsulfanyl-pyridine-2-carbonitrile (CAS 102645-27-2) (7.15 g, 36.0 mmol) in toluene (11 mL) was added to the reaction mixture. It was heated up to 90 °C and stirred overnight. After cooling down to 0 °C, methanol (70 mL) was added portionwise, followed by a mixture of methanol:water (56 mL, 4:1). It was then stirred at room temperature for 1 hour. The resulting suspension was filtered and the filtrate was concentrated to afford the desired crude amidine I8, which was used directly in the next step without further purification. LCMS (method 1): 216/218 (M+H)⁺, Rt 0.22 min.

### Step 2: Preparation of 2-(6-chloro-3-ethylsulfanyl-2-pyridyl)-5-(2,2,3,3,3-pentafluoropropoxy)-1H-pyri-midin-6-one (intermediate Int-2)

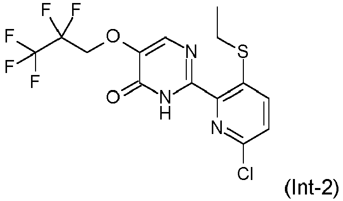

To a solution of 6-chloro-3-ethylsulfanyl-pyridine-2-carboxamidine (intermediate I8 prepared as described above) (1.25 g, 5.80 mmol) and methyl 3-oxo-2-(2,2,3,3,3-pentafluoropropoxy)propanoate (intermediate I7 prepared as described above) (2.90 g, 11.6 mmol, 2.00 equiv.) in ethanol (30 mL) was added potassium tert-butoxide (1.37 g, 11.6 mmol, 2.00 equiv.). The reaction mixture was heated up to 80 °C and stirred for 30 minutes. After cooling down to room temperature, the reaction mixture was diluted with water and extracted with ethyl acetate. The combined organic layers were washed with water and brine, dried over magnesium sulfate, filtered and concentrated under reduced pressure. The residue was purified by flash chromatography over silica gel (ethyl acetate in cyclohexane) to afford the desired product as a white/yellow solid. LCMS (method 1): 416/418 (M+H)⁺, Rt 1.08 min. ¹H NMR (400 MHz, CDCl₃) δ ppm: 1.41-1.46 (m, 3 H), 2.96 (q, *J* = 7.34 Hz, 2 H), 4.76 (td, *J* = 12.93, 0.92 Hz, 2 H), 7.38 (d, *J* = 8.44 Hz, 1 H), 7.69 (d, *J* = 8.44 Hz, 1 H), 7.95 (s, 1 H), 11.11 (br s, 1 H).

### Step 3: Preparation of 2-(6-chloro-3-ethylsulfanyl-2-pyridyl)-3-methyl-5-(2,2,3,3,3-pentafluoropropoxy)-pyrimidin-4-one (compound P6)

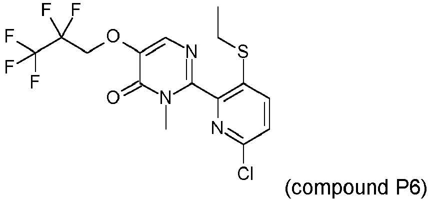

A suspension of methyl 4-methylbenzenesulfonate (0.566 mL, 3.75 mmol, 1.10 equiv.), potassium carbonate (0.689 g, 6.82 mmol, 2.00 equiv.) and 2-(6-chloro-3-ethylsulfanyl-2-pyridyl)-5-(2,2,3,3,3-pentafluoropropoxy)-1H-pyrimidin-6-one (intermediate Int-2 prepared as described above) (1.42 g, 3.41 mmol) in acetonitrile (27 mL) was heated up to 50 °C and stirred for 1.5 hours. The reaction mixture was directly adsorbed on Isolute and purified by flash chromatography over silica gel (ethyl acetate in cyclohexane) to afford the desired product. LCMS (method 1): 430/432 (M+H)⁺, Rt 1.06 min.

### Example H7: Preparation of 2-(6-chloro-3-ethylsulfonyl-2-pyridyl)-3-methyl-5-(2,2,3,3,3-pentafluoropro-poxv)pvrimidin-4-one (compound P7)

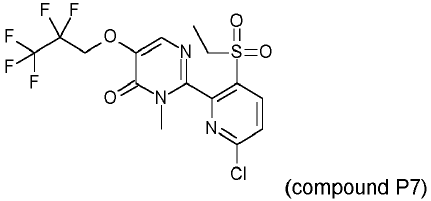

To a solution of 2-(6-chloro-3-ethylsulfanyl-2-pyridyl)-3-methyl-5-(2,2,3,3,3-pentafluoropropoxy)pyrimi-din-4-one (compound P6 prepared as described above) (1.35 g, 3.14 mmol) in dichloromethane (54 mL) was added at 0 °C 3-chloroperbenzoic acid (1.41 g, 6.27 mmol, 2.00 equiv.). The reaction mixture was stirred at 0 °C for 30 minutes, then at room temperature overnight. The reaction mixture was then poured on saturated sodium hydrogen carbonate aqueous solution and dichloromethane was added. The layers were separated and the organic layer was washed with sodium hydroxide aqueous solution. The aqueous layers were extracted with dichloromethane. The combined organic layers were washed with 40% sodium hydrogen sulfite aqueous solution, dried over sodium sulfate, filtered and concentrated under reduced pressure. The residue was purified by flash chromatography over silica gel (methanol in dichloromethane) to afford the desired product as a colorless oil. LCMS (method 1): 462/464 (M+H)⁺, Rt 1.00 min. ¹H NMR (400 MHz, CDCl₃) δ ppm: 1.33 (t, *J=* 7.52 Hz, 3 H), 3.37 (s, 3 H), 3.41-3.51 (m, 2 H), 4.74 (td, *J* = 12.84, 1.10 Hz, 2 H), 7.70 (d, *J=* 8.44 Hz, 1 H), 7.75 (s, 1 H), 8.34-8.40 (m, 1 H).

### Example H8: Preparation of 2-[3-ethylsulfonyl-6-(methylamino)-2-pyridyl]-3-methyl-5-(2,2,3,3,3-pentafluoropropoxy)pyrimidin-4-one (compound P8)

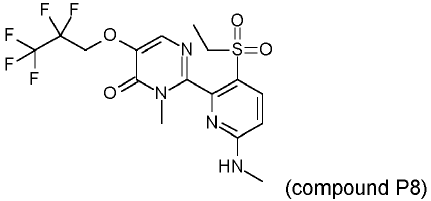

To a solution of 2-(6-chloro-3-ethylsulfonyl-2-pyridyl)-3-methyl-5-(2,2,3,3,3-pentafluoropropoxy)-pyrimidin-4-one (compound P7 prepared as described above) (0.564 g, 1.22 mmol) in tetrahydrofuran (2 mL) was added at room temperature methylamine (2 M in tetrahydrofuran, 3.05 mL, 6.11 mmol, 5.00 equiv.). The reaction mixture was stirred under microwave irradiation at 120 °C for 30 minutes. After cooling down to room temperature, the reaction mixture was directly adsorbed on Isolute and purified by flash chromatography over silica gel (ethyl acetate in cyclohexane) to afford the desired product.

LCMS (method 1): 457 (M+H)⁺, Rt 0.92 min.

### Example H9: Preparation of N-[5-ethylsulfonyl-6-[1-methyl-6-oxo-5-(2,2,3,3,3-pentafluoropropoxy)-pyrimidin-2-yl]-2-pyridyl]-N-methyl-acetamide (compound P9)

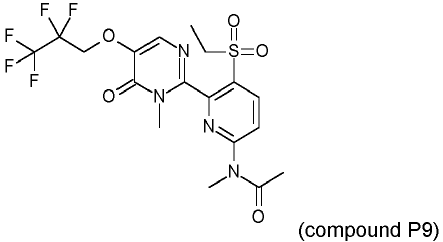

To a solution of 2-[3-ethylsulfonyl-6-(methylamino)-2-pyridyl]-3-methyl-5-(2,2,3,3,3-pentafluoropropoxy)pyrimidin-4-one (compound P8 prepared as described above) (35 mg, 0.077 mmol) in dichloromethane (2 mL) were added acetyl chloride (5.5 µL, 0.077 mmol, 1.0 equiv.) and N,N-diethylethanamine (12 µL, 0.084 mmol, 1.1 equiv.). The reaction mixture was stirred at room temperature for 40 minutes. Additional acetyl chloride (5.5 µL) and N,N-diethylethanamine (12 µL) were added again and it was stirred for 2 more hours. Catalytic amount of 4-dimethylaminopyridine was added and it was stirred at 45 °C overnight. Additional acetyl chloride (5.5 µL) and N,N-diethylethanamine (12 µL) were added again, before the mixture was concentrated. Pyridine (3 mL) was added to the residue, as well as additional acetyl chloride (2 × 5.5 µL) and it was stirred at 60 °C until full conversion was reached. After cooling down to room temperature, the reaction mixture was diluted with water and extracted with dichloromethane. The combined organic layers were washed with water and brine, dried over magnesium sulfate, filtered and concentrated under reduced pressure. The residue was purified first by flash chromatography over silica gel (ethyl acetate in cyclohexane), then by reverse phase chromatography to afford the desired product as a red resin. LCMS (method 1): 499 (M+H)⁺, Rt 0.93 min. ¹H NMR (400 MHz, CDCl₃) δ ppm: 1.26-1.36 (m, 3 H), 2.42 (s, 3 H), 3.34-3.36 (m, 3 H), 3.38-3.45 (m, 2 H), 3.53 (s, 3 H), 4.75 (br t, *J=* 12.84 Hz, 2 H), 7.76 (s, 1 H), 8.24-8.35 (m, 2 H).

### Example H10: Preparation of 1-[5-ethylsulfanyl-6-[1-methyl-6-oxo-5 (2,2,3,3,3pentafluoropropoxy) pvrimidin-2-vll-3-pvridvllcvclopropanecarbonitrile (compound P10)

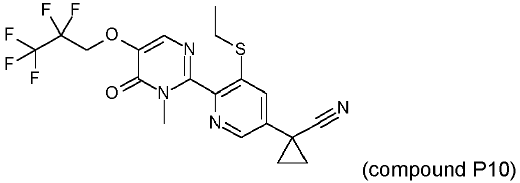

### Step 1: Preparation of ethyl 2-cyano-2-[5-ethylsulfanyl-6-[1-methyl-6-oxo-5-(2,2,3,3,3-pentafluoro - propoxy)pyrimidin-2-yl]-3-pyridyl]acetate (intermediate Int-4)

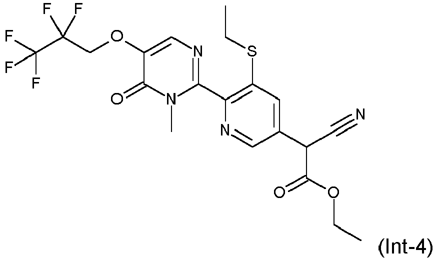

To a solution of 2-(5-bromo-3-ethylsulfanyl-2-pyridyl)-3-methyl-5-(2,2,3,3,3-pentafluoropropoxy)pyri-midin-4-one (compound P3 prepared as described above) (0.460 g, 0.970 mmol) in dimethyl sulfoxide (2.91 mL) were added ethyl 2-cyanoacetate (0.518 mL, 4.85 mmol, 5.00 eq.), L-proline (0.0226 g, 0.194 mmol, 0.20 eq.), potassium carbonate (1.34 g, 9.70 mmol, 10.0 eq.) and copper(I) iodide (0.0185 g, 0.0970 mmol, 0.10 eq.) at room temperature under Argon. The reaction mixture was stirred at 140°C for 1 h. After cooling down to 0°C, HCl (1N, 12.0 mL) was added dropwise very slowly due to strong foaming and the mixture was extracted with ethyl acetate. The combined organic layers were washed with water and brine, dried over magnesium sulfate, filtered and concentrated under reduced pressure. The residue was purified by flash chromatography over silica gel (ethyl acetate in cyclohexane) to afford the desired product as brown oil. LCMS (method 1): 507 (M+H)⁺, Rt 1.05 min.

### Step 2: Preparation of 2-[5-ethylsulfanyl-6-[1-methyl-6-oxo-5-(2,2,3,3,3-pentafluoropropoxy)pyrimidin-2-yll-3-pyridyllacetonitrile (intermediate Int-3)

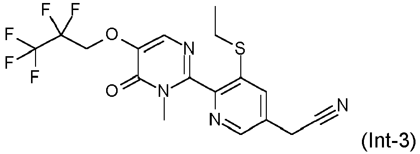

To a solution of ethyl 2-cyano-2-[5-ethylsulfanyl-6-[1-methyl-6-oxo-5-(2,2,3,3,3-pentafluoropropoxy)-pyrimidin-2-yl]-3-pyridyl]acetate (intermediate Int-4 prepared as described above) (0.238 g, 0.470 mmol) in dimethyl sulfoxide (5.00 mL) and water (3.00 mL) was added sodium chloride (0.277 g, 4.70 mmol, 10.0 eq.) at room temperature. The reaction mixture was stirred at 120°C for 4.5 h, adding more sodium chloride from time to time. Then it was cooled down to room temperature and stirred over night. Additional sodium chloride was added, it was heated up again to 140°C and stirred for 6 h. After cooling down to room temperature, the reaction mixture was diluted with water and extracted with ethyl acetate. The combined organic layers were washed with water and brine, dried over magnesium sulfate, filtered and concentrated under reduced pressure. The residue was purified by flash chromatography over silica gel (ethyl acetate in cyclohexane) to afford the desired product as light brown resin. LCMS (method 1): 435 (M+H)⁺, Rt 0.96 min. ¹H NMR (400 MHz, CDCl₃) δ ppm: 1.35 (t, 3 H), 3.01 (q, 2 H), 3.39 (s, 3 H), 3.89 (s, 2 H), 4.69-4.77 (t, 2 H), 7.79 (d, 1 H), 7.88 (s, 1 H), 8.43 (d, 1 H).

### Step 3: Preparation of 1-[5-ethylsulfanyl-6-[1-methyl-6-oxo-5 (2,2,3,3,3pentafluoropropoxy) pyrimidin-2-yl]-3-pyridyl]cyclopropanecarbonitrile (compound P10)

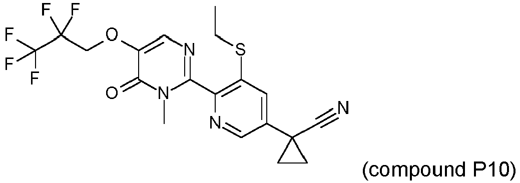

To a solution of 2-[5-ethylsulfanyl-6-[1-methyl-6-oxo-5-(2,2,3,3,3-pentafluoropropoxy)pyrimidin-2-yl]-3-pyridyl]acetonitrile (intermediate Int-3 prepared as described above) (0.158 g, 0.364 mmol) in acetonitrile (1.09 mL) was added cesium carbonate (0.356 g, 1.09 mmol, 3.00 eq.) followed by dropwise addition of 1,2-dibromoethane (0.0642 mL, 0.727 mmol, 2.00 eq.) at room temperature. The reaction mixture was stirred at 80°C for 24 h. After cooling down to room temperature, the reaction mixture was diluted with water and extracted with ethyl acetate. The combined organic layers were dried over magnesium sulfate, filtered and concentrated under reduced pressure. The residue was purified by flash chromatography over silica gel (ethyl acetate in cyclohexane) to afford the desired product as a colorless oil. LCMS (method 1): 461 (M+H)⁺, Rt 1.02 min. ¹H NMR (400 MHz, CDCl₃) δ ppm: 1.35 (t, 3 H), 1.58 (m, 2 H), 1.92 (m, 2 H), 2.95-3.03 (m, 2 H), 3.38 (s, 3 H), 4.69-4.77 (t, 2 H), 7.76 (d, 1 H), 7.82 (s, 1 H), 8.31 (d, 1 H).

### Example H11: Preparation of 1-[5-ethylsulfonyl-6-[1-methyl-6-oxo-5-(2,2,3,3,3-pentafluoropropoxy)-pvrimidin-2-vll-3-pvridvllcvclopropanecarbonitrile (compound P11)

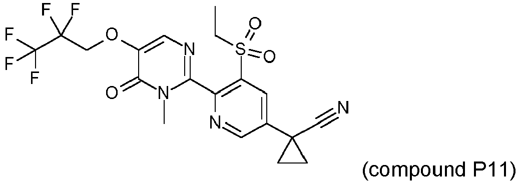

To a solution of 1-[5-ethylsulfanyl-6-[1-methyl-6-oxo-5 (2,2,3,3,3pentafluoropropoxy) pyrimidin-2-yl]-3-pyridyl]cyclopropanecarbonitrile (compound P10 prepared as described above) (0.0310 g, 0.0673 mmol) in dichloromethane (1.24 mL) was added mCPBA (0.0302 g, 0.135 mmol, 2.00 eq.) at 0°C. The reaction mixture was stirred at room temperature for 15 min and was poured into sat. aq. sodium bicarbonate solution. The mixture was extracted with dichloromethane, the organic phase was separated and washed with a solution of sodium hydroxide. The aqueous layer was extracted once again with dichloromethane and the combined organic layers were washed with a 40% sodium bisulfite solution. Afterwards the organic layer was dried over sodium sulfate, filtered and concentrated under reduced pressure after checking the presence of peroxides with a starch paper test. The reside was purified by flash chromatography over silica gel (ethyl acetate in cyclohexane) to afford the desired product as light yellow solid. LCMS (method 1): 493 (M+H)⁺, Rt 0.96 min. ¹H NMR (400 MHz, CDCl₃) δ ppm: 1.35 (t, 3 H), 1.65 (m, 2 H), 2.02 (m, 2 H), 3.32 (s, 3 H), 3.45-3.53 (q, 2 H), 4.70-4.79 (t, 2 H), 7.76 (d, 1 H), 8.20 (s, 1 H), 8.95 (d, 1 H).

### Example H12: Preparation of 2-[3-ethylsulfanyl-5-(2-pyridyloxy)-2-pyridyl]-3-methyl-5-(2,2,3,3,3-pentafluoropropoxy)pyrimidin-4-one (compound P19)

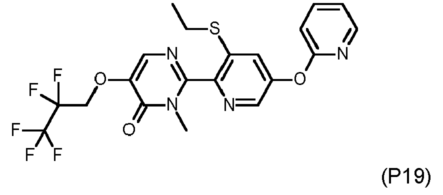

### Step 1: Preparation of 2-(3-ethylsulfanyl-5-hydroxy-2-pyridyl)-3-methyl-5-(2,2,3,3,3-pentafluoropropoxy)pyrimidin-4-one (intermediate Int-1)

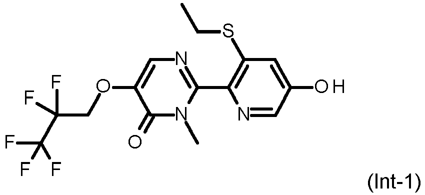

A mixture of 2-(5-bromo-3-ethylsulfanyl-2-pyridyl)-3-methyl-5-(2,2,3,3,3-pentafluoropropoxy)pyrimidin-4-one (compound P3 prepared as described above) (100 mg, 0.211 mmol), cesium carbonate (2.0 eq., 0.422 mmol) and (E)-benzaldehyde oxime (1.0 eq., 0.211 mmol) in N,N-dimethylformamide (2 mL/mmol) was heated to 50°C overnight, then at 85°C for three days. A further amount of (E)-benzaldehyde oxime (0.5 eq.) was added during this extended heating time. The reaction mixture was diluted with dichloromethane, water and aqueous sodium hydrogen carbonate. The separated aqueous layer was acidified to pH 5 and extracted with dichloromethane (3x). The combined organic phases were dried over sodium sulfate, filtered and concentrated. The residue was purified by flash chromatography on silica gel (ethyl acetate gradient in cyclohexane) to afford 2-(3-ethylsulfanyl-5-hydroxy-2-pyridyl)-3-methyl-5-(2,2,3,3,3-pentafluoro-propoxy)pyrimidin-4-one. LCMS (method 1): 412 (M+H)⁺, Rt 0.92 min.

### Step 2: Preparation of 2-f3-ethylsulfanyl-5-(2-pyridyloxy)-2-pyridyll-3-methyl-5-(2,2,3,3,3-pentafluoropropoxy)pyrimidin-4-one (compound P19)

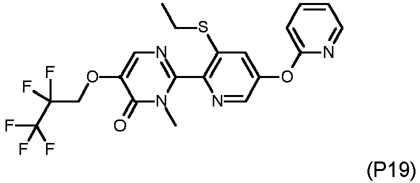

A mixture of 2-(3-ethylsulfanyl-5-hydroxy-2-pyridyl)-3-methyl-5-(2,2,3,3,3-pentafluororopoxy)pyrimidin-4-one (intermediate Int-1 prepared as described above) (38 mg, 0.092 mmol), potassium carbonate (2 eq., 0.185 mmol), 2-iodopyridine (1.3 eq., 0.120 mmol), sodium iodide (0.1 eq., 0.0092 mmol) and copper iodide (0.1 eq., 0.0092 mmol) in N,N-dimethylformamide (4 mL) was stirred at 100°C overnight, then at 120°C for 3 hours and at 143°C for one hour. The reaction mixture was diluted with ethyl acetate and water, the aqueous layer extracted with ethyl acetate (3x), the combined organic layers washed with water and brine, dried over sodium sulfate, filtered and concentrated *in vacuo.* The residue was purified by flash chromatography on silica gel (ethyl acetate gradient in cyclohexane) to afford 2-[3-ethylsulfanyl-5-(2-pyridyloxy)-2-pyridyl]-3-methyl-5-(2,2,3,3,3-pentafluoropropoxy)pyrimidin-4-one (compound P19). LCMS (method 1): 489 (M+H)⁺, Rt 1.06 min.

**Table P: Examples of compounds of formula (I)**

| No. | IUPAC name | Structures | LCMS | | | Mp (°C) |
|---|---|---|---|---|---|---|
| | | | Rₜ (min) | [M+H]⁺ (measured) | Method | |
| P1 | 2-(3-ethylsulfanyl-2-pyridyl)-3-methyl-5-(2,2,3,3,3-pentafluoropropoxy)pyrimi din-4-one | 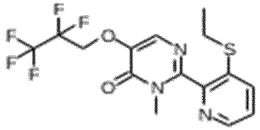 | 0.97 | 396 | 1 | - |
| P2 | 2-(3-ethylsulfonyl-2-pyridyl)-3-methyl-5-(2,2,3,3,3-pentafluoropropoxy)pyrimi din-4-one | 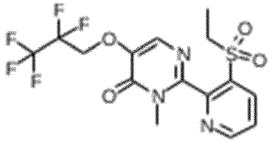 | 0.89 | 428 | 1 | 107 - 118 |
| P3 | 2-(5-bromo-3-ethylsulfanyl-2-pyridyl)-3-methyl-5-(2,2,3,3,3-pentafluoropropoxy)pyrimi din-4-one | 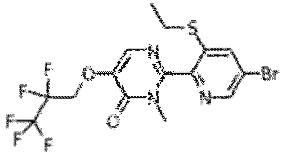 | 1.09 | 474/476 | 1 | - |
| P4 | N-[5-ethylsulfanyl-6-[1-methyl-6-oxo-5-(2,2,3,3,3-pentafluoropropoxy)pyrimi din-2-yl]-3-pyridyl]-N-methyl-acetamide | 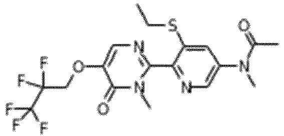 | 0.93 | 467 | 1 | - |
| P5 | N-[5-ethylsulfonyl-6-[1-methyl-6-oxo-5-(2,2,3,3,3-pentafluoropropoxy)pyrimi din-2-yl]-3-pyridyl]-N-methyl-acetamide | 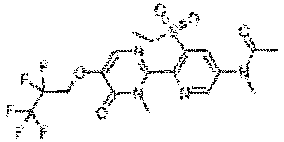 | 0.88 | 499 | 1 | - |
| P6 | 2-(6-chloro-3-ethylsulfanyl-2-pyridyl)-3-methyl-5-(2,2,3,3,3-pentafluoropropoxy)pyrimi din-4-one | 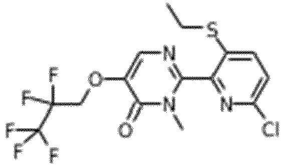 | 1.06 | 430/432 | 1 | - |
| P7 | 2-(6-chloro-3-ethylsulfonyl-2-pyridyl)-3-methyl-5-(2,2,3,3,3-pentafluoropropoxy)pyrimi din-4-one | 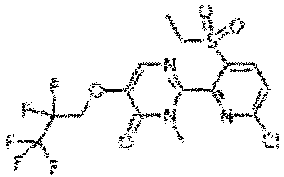 | 1.00 | 462/464 | 1 | - |
| P8 | 2-[3-ethylsulfonyl-6-(methylamino)-2-pyridyl]-3-methyl-5-(2,2,3,3,3-pentafluoropropoxy)pyrimi din-4-one | 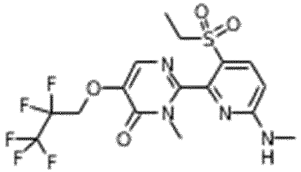 | 0.92 | 457 | 1 | - |
| P9 | N-[5-ethylsulfonyl-6-[1-methyl-6-oxo-5-(2, 2, 3, 3, 3-pentafluoropropoxy)pyrimi din-2-yl]-2-pyridyl]-N-methyl-acetamide | 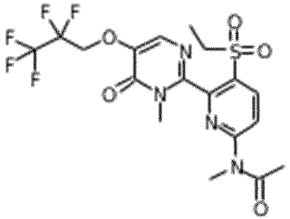 | 0.93 | 499 | 1 | - |
| P10 | 1-[5-ethylsulfanyl-6-[1-methyl-6-oxo-5-(2,2,3,3,3-pentafluoropropoxy)pyrimi din-2-yl]-3-pyridyl]cyclopropanecarbo nitrile | 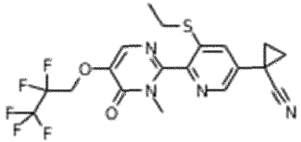 | 1.02 | 461 | 1 | - |
| P11 | 1-[5-ethylsulfonyl-6-[1-methyl-6-oxo-5-(2,2,3,3,3-pentafluoropropoxy)pyrimi din-2-yl]-3-pyridyl]cyclopropanecarbo nitrile | 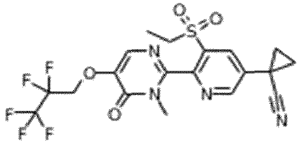 | 0.96 | 493 | 2 | - |
| P12 | 2-[5-ethylsulfanyl-6-[1-methyl-6-oxo-5-(2,2,3,3,3-pentafluoropropoxy)pyrimi din-2-yl]-3-pyridyl]-2-methyl-propanenitrile | 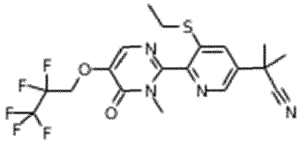 | 1.04 | 463 | 1 | - |
| P13 | 2-[5-ethylsulfonyl-6-[1-methyl-6-oxo-5-(2,2,3,3,3-pentafluoropropoxy)pyrimi din-2-yl]-3-pyridyl]-2-methyl-propanenitrile | 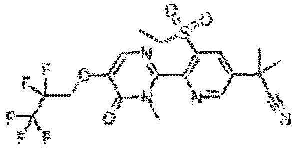 | 0.99 | 495 | 1 | - |
| P14 | 2-[3-ethylsulfonyl-6-(1,2,4-triazol-1-yl)-2-pyridyl]-3-methyl-5-(2,2,3,3,3-pentafluoropropoxy)pyrimi din-4-one | 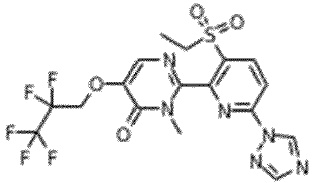 | 0.92 | 495 | 1 | - |
| P15 | 1-[5-ethylsulfonyl-6-[1-methyl-6-oxo-5-(2,2,3,3,3-pentafluoropropoxy)pyrimi din-2-yl]-2-pyridyl]-1,3-dimethyl-urea | 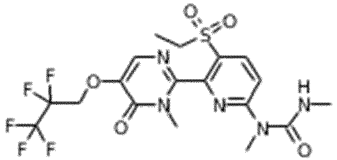 | 0.90 | 514 | 1 | - |
| P16 | N-[5-ethylsulfonyl-6-[1-methyl-6-oxo-5-(2,2,3,3,3-pentafluoropropoxy)pyrimi din-2-yl]-2-pyridyl]-N-methyl-cyclopropanecarboxamide | 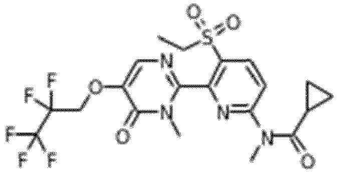 | 1.00 | 525 | 1 | - |
| P17 | 2-(5-cyclopropyl-3-ethylsulfanyl-2-pyridyl)-3-methyl-5-(2,2,3,3,3-pentafluoropropoxy)pyrimi din-4-one | 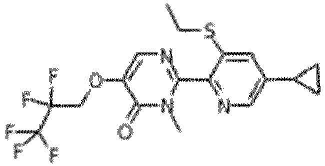 | 1.09 | 436 | 1 | - |
| P18 | 2-(5-cyclopropyl-3-ethylsulfonyl-2-pyridyl)-3-methyl-5-(2,2,3,3,3-pentafluoropropoxy)pyrimi din-4-one | 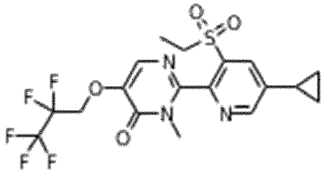 | 1.01 | 468 | 1 | 134 - 141 |
| P19 | 2-[3-ethylsulfanyl-5-(2-pyridyloxy)-2-pyridyl]-3-methyl-5-(2,2,3,3,3-pentafluoropropoxy)pyrimi din-4-one | 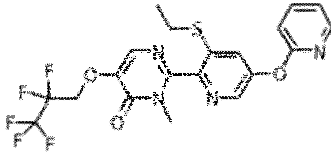 | 1.06 | 489 | 1 | - |
| P20 | 2-[3-ethylsulfonyl-5-(2-pyridyloxy)-2-pyridyl]-3-methyl-5-(2,2,3,3,3-pentafluoropropoxy)pyrimi din-4-one | 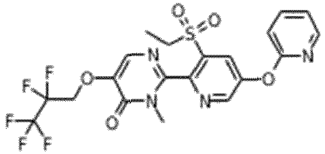 | 1.05 | 521 | 1 | - |

**Table Int: Examples of intermediates**

| No. | IUPAC name | Structures | LCMS | | | Mp (°C) |
|---|---|---|---|---|---|---|
| | | | Rt (min) | [M+H]⁺ (measured) | Method | |
| Int-1 | 2-(3-ethylsulfanyl-5-hydroxy-2-pyridyl)-3-methyl-5-(2,2,3,3,3-pentafluoropropoxy)pyrimi din-4-one | 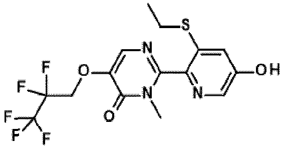 | 0.92 | 412 | 1 | - |
| lnt-2 | 2-(6-chloro-3-ethylsulfanyl-2-pyridyl)-5-(2,2,3,3,3-pentafluoropropoxy)-1H-pyrimidin-6-one | 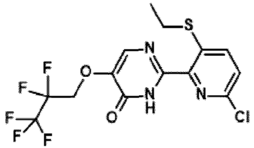 | 1.08 | 416/418 | 1 | - |
| Int-3 | 2-(5-ethylsulfanyl-6-(1-methyl-6-oxo-5-(2,2,3,3,3-pentafluoropropoxy)pyrimi din-2-yl]-3-pyridyl]acetonitrile | 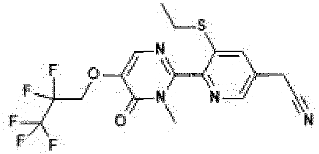 | 0.96 | 435 | 1 | - |
| Int-4 | ethyl 2-cyano-2-[5-ethylsulfanyl-6-[1-methyl-6-oxo-5-(2,2,3,3,3-pentafluoropropoxy)pyrimi din-2-yl]-3-pyridyl]acetate | 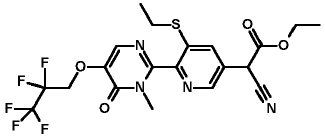 | 1.05 | 507 | 1 | - |
| Int-5 | 2-(5-bromo-3-ethylsulfanyl-2-pyridyl)-5-(2,2,3,3,3-pentafluoropropoxy)-1H-pyrimidin-6-one. | 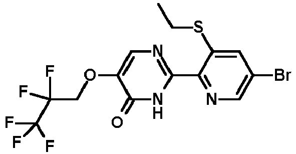 | 1.13 | 460/462 | 1 | - |

The activity of the compositions according to the invention can be broadened considerably, and adapted to prevailing circumstances, by adding other insecticidally, acaricidally and/or fungicidally active ingredients. The mixtures of the compounds of formula I with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use.

Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and Bacillus thuringiensis preparations.

The following mixtures of the compounds of formula I with active ingredients are preferred (the abbreviation "TX" means "one compound selected from the group consisting of the compounds described in Tables A-1 to A-81 and Tables B-1 to B-54 and Table P of the present invention"):
an adjuvant selected from the group of substances consisting of petroleum oils (alternative name) (628) + TX,
an adjuvant selected from the group of substances consisting of petroleum oils (alternative name) (628) + TX,
an acaricide selected from the group of substances consisting of 1 ,1-bis(4-chlorophenyl)-2-ethoxyethanol (IUPAC name) (910) + TX, 2,4-dichlorophenyl benzenesulfonate (lUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos (alternative name) [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate (alternative name) + TX, bromocyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben (alternative name) + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulfide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel (alternative name) [CCN] + TX, coumaphos (174) + TX, crotamiton (alternative name) [CCN] + TX, crotoxyphos (1010) + TX, cufraneb (1013) + TX, cyanthoate (1020) + TX, cyflumetofen (CAS Reg. No.: 400882-07-7) + TX, cyhalothrin (196) + TX, cyhexatin (199) + TX, cypermetrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulfon (1039) + TX, diafenthiuron (226) + TX, dialifos (1042) + TX, diazinon (227) + TX, dichlofluanid (230) + TX, dichlorvos (236) + TX, dicliphos (alternative name) + TX, dicofol (242) + TX, dicrotophos (243) + TX, dienochlor (1071) + TX, dimefox (1081) + TX, dimethoate (262) + TX, dinactin (alternative name) (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram (alternative name) [CCN] + TX, disulfoton (278) + TX, DNOC (282) + TX, dofenapyn (1113) + TX, doramectin (alternative name) [CCN] + TX, endosulfan (294) + TX, endothion (1121) + TX, EPN (297) + TX, eprinomectin (alternative name) [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad (alternative name) + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (lUPAC/Chemical Abstracts name) (1216) + TX, hexythiazox (441) + TX, iodomethane (IUPAC name) (542) + TX, isocarbophos (alternative name) (473) + TX, isopropyl O-(methoxyaminothiophosphoryl)salicylate (IUPAC name) (473) + TX, ivermectin (alternative name) [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen (alternative name) [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime (alternative name) [CCN] + TX, mipafox (1293) + TX, monocrotophos (561) + TX, morphothion (1300) + TX, moxidectin (alternative name) [CCN] + TX, naled (567) + TX, NC-184 (compound code) + TX, NC-512 (compound code) + TX, nifluridide (1309) + TX, nikkomycins (alternative name) [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (alternative name) (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (alternative name) (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos (alternative name) + TX, selamectin (alternative name) [CCN] + TX, SI-0009 (compound code) + TX, sophamide (1402) + TX, spirodiclofen (738) + TX, spiromesifen (739) + TX, SSI-121 (development code) (1404) + TX, sulfiram (alternative name) [CCN] + TX, sulfluramid (750) + TX, sulfotep (753) + TX, sulfur (754) + TX, SZI-121 (development code) (757) + TX, tau-fluvalinate (398) + TX, tebufenpyrad (763) + TX, TEPP (1417) + TX, terbam (alternative name) + TX, tetrachlorvinphos (777) + TX, tetradifon (786) + TX, tetranactin (alternative name) (653) + TX, tetrasul (1425) + TX, thiafenox (alternative name) + TX, thiocarboxime (1431) + TX, thiofanox (800) + TX, thiometon (801) + TX, thioquinox (1436) + TX, thuringiensin (alternative name) [CCN] + TX, triamiphos (1441) + TX, triarathene (1443) + TX, triazophos (820) + TX, triazuron (alternative name) + TX, trichlorfon (824) + TX, trifenofos (1455) + TX, trinactin (alternative name) (653) + TX, vamidothion (847) + TX, vaniliprole [CCN] and YI-5302 (compound code) + TX,
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,
an anthelmintic selected from the group of substances consisting of abamectin (1) + TX, crufomate (1011) + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX,
an avicide selected from the group of substances consisting of chloralose (127) + TX, endrin (1122) + TX, fenthion (346) + TX, pyridin-4-amine (IUPAC name) (23) and strychnine (745) + TX,
a bactericide selected from the group of substances consisting of 1-hydroxy-1*H*-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX,
a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H. megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinemema carpocapsae* (alternative name) (742) + TX, *Steinernema feltiae* (alternative name) (742) + TX, *Steinemema glaseri* (alternative name) (742) + TX, *Steinernema riobrave* (alternative name) (742) + TX, *Steinemema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX,
a soil sterilant selected from the group of substances consisting of iodomethane (IUPAC name) (542) and methyl bromide (537) + TX,
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX,
an insect pheromone selected from the group of substances consisting of (E)-dec-5-en-1-yl acetate with (E)-dec-5-en-1-ol (IUPAC name) (222) + TX, (E)-tridec-4-en-1-yl acetate (IUPAC name) (829) + TX, (E)-6-methylhept-2-en-4-ol (IUPAC name) (541) + TX, (*E,Z*)-tetradeca-4,10-dien-1-yl acetate (IUPAC name) (779) + TX, (Z)-dodec-7-en-1-yl acetate (IUPAC name) (285) + TX, (Z)-hexadec-11-enal (IUPAC name) (436) + TX, (Z)-hexadec-11-en-1-yl acetate (IUPAC name) (437) + TX, (Z)-hexadec-13-en-11-yn-1-yl acetate (IUPAC name) (438) + TX, (Z)-icos-13-en-10-one (IUPAC name) (448) + TX, (Z)-tetradec-7-en-1-al (IUPAC name) (782) + TX, (Z)-tetradec-9-en-1-ol (IUPAC name) (783) + TX, (Z)-tetradec-9-en-1-yl acetate (IUPAC name) (784) + TX, (7E,9Z)-dodeca-7,9-dien-1-yl acetate (IUPAC name) (283) + TX, (9*Z*,11*E*)-tetradeca-9,11-dien-1-yl acetate (IUPAC name) (780) + TX, (9Z,12E)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B, (alternative name) (839) + TX, trimedlure B₂ (alternative name) (839) + TX, trimedlure C (alternative name) (839) and trunc-call (alternative name) [CCN] + TX,
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,
an insecticide selected from the group of substances consisting of 1-dichloro-1-nitroethane (IUPAC/ChemicalAbstracts name) (1058) + TX, 1,1-dichloro-2,2-bis(4-ethylphenyl)ethane (IUPAC name) (1056), + TX, 1,2-dichloropropane (IUPAC/Chemical Abstracts name) (1062) + TX, 1,2-dichloropropane with 1,3-dichloropropene (IUPAC name) (1063) + TX, 1-bromo-2-chloroethane (IUPAC/ChemicalAbstracts name) (916) + TX, 2,2,2-trichloro-1-(3,4-dichlorophenyl)ethyl acetate (IUPAC name) (1451) + TX, 2,2-dichlorovinyl 2-ethylsulfinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/Chemical Abstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion (alternative name) [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin (alternative name) [CCN] + TX, allyxycarb (866) + TX, alpha-cypermethrin (202) + TX, alpha-ecdysone (alternative name) [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (alternative name) (41) + TX, azamethiphos (42) + TX, azinphos-ethyl (44) + TX, azinphos-methyl (45) + TX, azothoate (889) + TX, *Bacillus thuringiensis* delta endotoxins (alternative name) (52) + TX, barium hexafluorosilicate (alternative name) [CCN] + TX, barium polysulfide (IUPAC/Chemical Abstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin S-cyclopentenyl isomer (alternative name) (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate (alternative name) + TX, bromfenvinfos (914) + TX, bromocyclen (918) + TX, bromo-DDT (alternative name) [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben (alternative name) + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/ChemicalAbstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (alternative name) (725) + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin (alternative name) + TX, cismethrin (80) + TX, clocythrin (alternative name) + TX, cloethocarb (999) + TX, closantel (alternative name) [CCN] + TX, clothianidin (165) + TX, copper acetoarsenite [CCN] + TX, copper arsenate [CCN] + TX, copper oleate [CCN] + TX, coumaphos (174) + TX, coumithoate (1006) + TX, crotamiton (alternative name) [CCN] + TX, crotoxyphos (1010) + TX, crufomate (1011) + TX, cryolite (alternative name) (177) + TX, CS 708 (development code) (1012) + TX, cyanofenphos (1019) + TX, cyanophos (184) + TX, cyanthoate (1020) + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate (alternative name) [CCN] + TX, d-limonene (alternative name) [CCN] + TX, d-tetramethrin (alternative name) (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dimpropyridaz + TX, dialifos (1042) + TX, diamidafos (1044) + TX, diazinon (227) + TX, dicapthon (1050) + TX, dichlofenthion (1051) + TX, dichlorvos (236) + TX, dicliphos (alternative name) + TX, dicresyl (alternative name) [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor (alternative name) [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin (alternative name) [CCN] + TX, DSP (1115) + TX, ecdysterone (alternative name) [CCN] + TX, El 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin (alternative name) [CCN] + TX, esfenvalerate (302) + TX, etaphos (alternative name) [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (alternative name) (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad (alternative name) + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (alternative name) (473) + TX, isodrin (1235) + TX, isofenphos (1236) + TX, isolane (1237) + TX, isoprocarb (472) + TX, isopropyl *O*-(methoxyaminothiophosphoryl)salicylate (IUPAC name) (473) + TX, isoprothiolane (474) + TX, isothioate (1244) + TX, isoxathion (480) + TX, ivermectin (alternative name) [CCN] + TX, jasmolin I (696) + TX, jasmolin II (696) + TX, jodfenphos (1248) + TX, juvenile hormone I (alternative name) [CCN] + TX, juvenile hormone II (alternative name) [CCN] + TX, juvenile hormone III (alternative name) [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, m-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (alternative name) (519) + TX, metam-sodium (519) + TX, methacrifos (1266) + TX, methamidophos (527) + TX, methanesulfonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (alternative name) (533) + TX, methoxychlor (534) + TX, methoxyfenozide (535) + TX, methyl bromide (537) + TX, methyl isothiocyanate (543) + TX, methylchloroform (alternative name) [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime (alternative name) [CCN] + TX, mipafox (1293) + TX, mirex (1294) + TX, monocrotophos (561) + TX, morphothion (1300) + TX, moxidectin (alternative name) [CCN] + TX, naftalofos (alternative name) [CCN] + TX, naled (567) + TX, naphthalene (IUPAC/Chemical Abstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O*-5-dichloro-4-iodophenyl O-ethyl ethylphosphonothioate (IUPAC name) (1057) + TX, O,O-diethyl *O*-4-methyl-2-oxo-2*H*-chromen-7-yl phosphorothioate (IUPAC name) (1074) + TX, O,O-diethyl *O*-6-methyl-2-propylpyrimidin-4-yl phosphorothioate (IUPAC name) (1075) + TX, O,O,O',O'-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron (alternative name) [CCN] + TX, pentachlorophenol (623) + TX, pentachlorophenyl laurate (IUPAC name) (623) + TX, permethrin (626) + TX, petroleum oils (alternative name) (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I (alternative name) [CCN] + TX, precocene II (alternative name) [CCN] + TX, precocene III (alternative name) [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrazophos (693) + TX, pyresmethrin (1367) + TX, pyrethrin I (696) + TX, pyrethrin II (696) + TX, pyrethrins (696) + TX, pyridaben (699) + TX,
pyridalyl (700) + TX, pyridaphenthion (701) + TX, pyrimidifen (706) + TX, pyrimitate (1370) + TX, pyriproxyfen (708) + TX, quassia (alternative name) [CCN] + TX, quinalphos (711) + TX, quinalphos-methyl (1376) + TX, quinothion (1380) + TX, quintiofos (1381) + TX, R-1492 (development code) (1382) + TX, rafoxanide (alternative name) [CCN] + TX, resmethrin (719) + TX, rotenone (722) + TX, RU 15525 (development code) (723) + TX, RU 25475 (development code) (1386) + TX, ryania (alternative name) (1387) + TX, ryanodine (traditional name) (1387) + TX, sabadilla (alternative name) (725) + TX, schradan (1389) + TX, sebufos (alternative name) + TX, selamectin (alternative name) [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/Chemical Abstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinosad (737) + TX, spiromesifen (739) + TX, spirotetrmat (CCN) + TX, sulcofuron (746) + TX, sulcofuron-sodium (746) + TX, sulfluramid (750) + TX, sulfotep (753) + TX, sulfuryl fluoride (756) + TX, sulprofos (1408) + TX, tar oils (alternative name) (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam (alternative name) + TX, terbufos (773) + TX, tetrachloroethane [CCN] + TX, tetrachlorvinphos (777) + TX, tetramethrin (787) + TX, theta-cypermethrin (204) + TX, thiacloprid (791) + TX, thiafenox (alternative name) + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin (alternative name) [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron (alternative name) + TX, trichlorfon (824) + TX, trichlormetaphos-3 (alternative name) [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (alternative name) (725) + TX, veratrine (alternative name) (725) + TX, XMC (853) + TX, xylylcarb (854) + TX, YI-5302 (compound code) + TX, zeta-cypermethrin (205) + TX, zetamethrin (alternative name) + TX, zinc phosphide (640) + TX, zolaprofos (1469) and ZXI 8901 (development code) (858) + TX, cyantraniliprole [736994-63-19 + TX, chlorantraniliprole [500008-45-7] + TX, cyenopyrafen [560121-52-0] + TX, cyflumetofen [400882-07-7] + TX, pyrifluquinazon [337458-27-2] + TX, spinetoram [187166-40-1 + 187166-15-0] + TX, spirotetramat [203313-25-1] + TX, sulfoxaflor [946578-00-3] + TX, flufiprole [704886-18-0] + TX, meperfluthrin [915288-13-0] + TX, tetramethylfluthrin [84937-88-2] + TX, triflumezopyrim (disclosed in WO 2012/092115) + TX, fluxametamide (WO 2007/026965) + TX, epsilon-metofluthrin [240494-71-7] + TX, epsilon-momfluorothrin [1065124-65-3] + TX,
fluazaindolizine [1254304-22-7] + TX, chloroprallethrin [399572-87-3] + TX, fluxametamide [928783-29-3] + TX, cyhalodiamide [1262605-53-7] + TX, tioxazafen [330459-31-9] + TX, broflanilide [1207727-04-5] + TX, flufiprole [704886-18-0] + TX, cyclaniliprole [1031756-98-5] + TX, tetraniliprole [1229654-66-3] + TX, guadipyr (described in WO2010/060231) + TX, cycloxaprid (described in WO 2005/077934) + TX, spiropidion + TX, Afidopyropen + TX, flupyrimin + TX, Momfluorothrin + TX, kappa-bifenthrin + TX, kappa-tefluthrin + TX, Dichloromezotiaz + TX, Tetrachloraniliprole + TX, benzpyrimoxan + TX
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX,
a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX, fluopyram + TX,
a nitrification inhibitor selected from the group of substances consisting of potassium ethylxanthate [CCN] and nitrapyrin (580) + TX,
a plant activator selected from the group of substances consisting of acibenzolar (6) + TX, acibenzolar-S-methyl (6) + TX, probenazole (658) and *Reynoutria sachalinensis* extract (alternative name) (720) + TX,
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX,
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX,
a virucide selected from the group of substances consisting of imanin (alternative name) [CCN] and ribavirin (alternative name) [CCN] + TX,
a wound protectant selected from the group of substances consisting of mercuric oxide (512) + TX, octhilinone (590) and thiophanate-methyl (802) + TX,
and biologically active compounds selected from the group consisting of azaconazole (60207-31-0] + TX, bitertanol [70585-36-3] + TX, bromuconazole [116255-48-2] + TX, cyproconazole [94361-06-5] + TX, difenoconazole [119446-68-3] + TX, diniconazole [83657-24-3] + TX, epoxiconazole [106325-08-0] + TX, fenbuconazole [114369-43-6] + TX, fluquinconazole [136426-54-5] + TX, flusilazole [85509-19-9] + TX, flutriafol [76674-21-0] + TX, hexaconazole [79983-71-4] + TX, imazalil [35554-44-0] + TX, imibenconazole [86598-92-7] + TX, ipconazole [125225-28-7] + TX, metconazole [125116-23-6] + TX, myclobutanil [88671-89-0] + TX, pefurazoate [101903-30-4] + TX, penconazole [66246-88-6] + TX, prothioconazole [178928-70-6] + TX, pyrifenox [88283-41-4] + TX, prochloraz [67747-09-5] + TX, propiconazole [60207-90-1] + TX, simeconazole [149508-90-7] + TX, tebuconazole [107534-96-3] + TX, tetraconazole [112281-77-3] + TX, triadimefon [43121-43-3] + TX, triadimenol [55219-65-3] + TX, triflumizole [99387-89-0] + TX, triticonazole [131983-72-7] + TX, ancymidol [12771-68-5] + TX, fenarimo! [60168-88-9] + TX, nuarimol [63284-71-9] + TX, bupirimate [41483-43-6] + TX, dimethirimol [5221-53-4] + TX, ethirimol [23947-60-6] + TX, dodemorph [1593-77-7] + TX, fenpropidine [67306-00-7] + TX, fenpropimorph [67564-91-4] + TX, spiroxamine [118134-30-8] + TX, tridemorph [81412-43-3] + TX, cyprodinil [121552-61-2] + TX, mepanipyrim [110235-47-7] + TX, pyrimethanil [53112-28-0] + TX, fenpiclonil [74738-17-3] + TX, fludioxonil [131341-86-1] + TX, benalaxyl [71626-11-4] + TX, furalaxyl [57646-30-7] + TX, metalaxyl [57837-19-1] + TX, R-metalaxyl [70630-17-0] + TX, ofurace [58810-48-3] + TX, oxadixyl [77732-09-3] + TX, benomyl [17804-35-2] + TX, carbendazim [10605-21-7] + TX, debacarb [62732-91-6] + TX, fuberidazole [3878-19-1] + TX, thiabendazole [148-79-8] + TX, chlozolinate [84332-86-5] + TX, dichlozoline [24201-58-9] + TX, iprodione [36734-19-7] + TX, myclozoline [54864-61-8] + TX, procymidone [32809-16-8] + TX, vinclozoline [50471-44-8] + TX, boscalid [188425-85-6] + TX, carboxin [5234-68-4] + TX, fenfuram [24691-80-3] + TX, flutolanil [66332-96-5] + TX, mepronil [55814-41-0] + TX, oxycarboxin [5259-88-1] + TX, penthiopyrad [183675-82-3] + TX, thifluzamide [130000-40-7] + TX, guazatine [108173-90-6] + TX, dodine [2439-10-3] [112-65-2] (free base) + TX, iminoctadine [13516-27-3] + TX, azoxystrobin [131860-33-8] + TX, dimoxystrobin [149961-52-4] + TX, enestroburin {Proc. BCPC, Int. Congr., Glasgow, 2003, 1, 93} + TX, fluoxastrobin [361377-29-9] + TX, kresoxim-methyl [143390-89-0] + TX, metominostrobin [133408-50-1] + TX, trifloxystrobin [141517-21-7] + TX, orysastrobin [248593-16-0] + TX, picoxystrobin [117428-22-5] + TX,
pyraclostrobin [175013-18-0] + TX, ferbam [14484-64-1] + TX, mancozeb [8018-01-7] + TX, maneb [12427-38-2] + TX, metiram [9006-42-2] + TX, propineb [12071-83-9] + TX, thiram [137-26-8] + TX, zineb [12122-67-7] + TX, ziram [137-30-4] + TX, captafol [2425-06-1] + TX, captan [133-06-2] + TX, dichlofluanid [1085-98-9] + TX, fluoroimide [41205-21-4] + TX, folpet [133-07-3] + TX, tolylfluanid [731-27-1] + TX, bordeaux mixture [8011-63-0] + TX, copperhydroxid [20427-59-2] + TX, copperoxychlorid [1332-40-7] + TX, coppersulfat [7758-98-7] + TX, copperoxid [1317-39-1] + TX, mancopper [53988-93-5] + TX, oxine-copper [10380-28-6] + TX, dinocap [131-72-6] + TX, nitrothal-isopropyl [10552-74-6] + TX, edifenphos [17109-49-8] + TX, iprobenphos [26087-47-8] + TX, isoprothiolane [50512-35-1] + TX, phosdiphen [36519-00-3] + TX, pyrazophos [13457-18-6] + TX, tolclofos-methyl [57018-04-9] + TX, acibenzolar-S-methyl [135158-54-2] + TX, anilazine [101-05-3] + TX, benthiavalicarb [413615-35-7] + TX, blasticidin-S [2079-00-7] + TX, chinomethionat [2439-01-2] + TX, chloroneb [2675-77-6] + TX, chlorothalonil *[1897-45-6]* + TX, cyflufenamid *[180409-60-3]* + TX, cymoxanil *[57966-95-7]* + TX, dichlone *[117-80-6]* + TX, diclocymet [139920-32-4] + TX, diclomezine *[62865-36-5]* + TX, dicloran *[99-30-9]* + TX, diethofencarb *[87130-20-9]* + TX, dimethomorph *[110488-70-5]* + TX, SYP-LI90 (Flumorph) *[211867-47-9]* + TX, dithianon *[3347-22-6]* + TX, ethaboxam *[162650-77-3]* + TX, etridiazole *[2593-15-9]* + TX, famoxadone *[131807-57-3]* + *TX,* fenamidone *[161326-34-7]* + TX, fenoxanil *[115852-48-7]* + TX, fentin *[668-34-8]* + TX, ferimzone *[89269-64-7]* + TX, fluazinam *[79622-59-6]* + TX, fluopicolide [239110-15-7] + TX, flusulfamide [106917-52-6] + TX, fenhexamid [126833-17-8] + TX, fosetyl-aluminium [39148-24-8] + TX, hymexazol [10004-44-1] + TX, iprovalicarb [140923-17-7] + TX, IKF-916 (Cyazofamid) *[120116-88-3]* + TX, kasugamycin [6980-18-3] + TX, methasulfocarb *[66952-49-6]* + TX, metrafenone *[220899-03-*6] + TX, pencycuron *[66063-05-6]* + TX, phthalide [27355-22-2] + TX, polyoxins [11113-80-7] + TX, probenazole [27605-76-1] + TX, propamocarb [25606-41-1] + TX, proquinazid [189278-12-4] + TX, pyroquilon [57369-32-1] + TX, quinoxyfen [124495-18-7] + TX, quintozene [82-68-8] + TX, sulfur [7704-34-9] + TX, tiadinil [223580-51-6] + TX, triazoxide [72459-58-6] + TX, tricyclazole [41814-78-2] + TX, triforine [26644-46-2] + TX, validamycin [37248-47-8] + TX, zoxamide (RH7281) [156052-68-5] + TX, mandipropamid [374726-62-2] + TX, isopyrazam [881685-58-1] + TX, sedaxane [874967-67-6] + TX, 3-difluoromethyl-1-methyl-1H-pyrazole-4-carboxylic acid (9-dichloromethylene-1 ,2,3,4-tetrahydro-1,4-methano-naphthalen-5-yl)-amide (disclosed in WO 2007/048556) + TX, 3-difluoromethyl-1-methyl-1H-pyrazole-4-carboxylic acid (3',4',5'-trifluoro-biphenyl-2-yl)-amide (disclosed in WO 2006/087343) + TX, [(3S,4R,4aR,6S,6aS, 12R, 12aS, 12bS)-3-[(cyclopropylcarbonyl)oxy]-1,3,4,4a,5,6,6a,12,12a,12b-decahydro-6,12-dihydroxy-4,6a,12b-trimethyl-11-oxo-9-(3-pyridinyl)-2*H*,11*H*naphtho[2,1-*b*]pyrano[3,4-*e*]pyran-4-yl]methyl-cyclopropanecarboxylate [915972-17-7] + TX and 1,3,5-trimethyl-N-(2-methyl-1-oxopropyl)-N-[3-(2-methylpropyl)-4-[2,2,2-trifluoro-1-methoxy-1-(trifluoromethyl)ethyl]phenyl]-1H-pyrazole-4-carboxamide [926914-55-8] + TX; lancotrione [1486617-21-3] + TX, florpyrauxifen [943832-81-3] ] + TX, ipfentrifluconazole[1417782-08-1] + TX,
mefentrifluconazole [1417782-03-6] + TX, quinofumelin [861647-84-9] + TX, chloroprallethrin [399572-87-3] + TX, cyhalodiamide [1262605-53-7] ] + TX, fluazaindolizine [1254304-22-7] + TX, fluxametamide [928783-29-3] + TX, epsilon-metofluthrin [240494-71-7] + TX, epsilon-momfluorothrin [1065124-65-3] + TX, pydiflumetofen [1228284-64-7] + TX, kappa-bifenthrin [439680-76-9] + TX, broflanilide [1207727-04-5] + TX, dicloromezotiaz [1263629-39-5] + TX, dipymetitrone [16114-35-5] + TX, pyraziflumid [942515-63-1] + TX, kappa-tefluthrin [391634-71-2] + TX, fenpicoxamid [517875-34-2] + TX; fluindapyr [1383809-87-7] + TX; alpha-bromadiolone [28772-56-7] + TX; flupyrimin [1689566-03-7] + TX; benzpyrimoxan [1449021-97-9] + TX; acynonapyr [1332838-17-1] + TX; inpyrfluxam [1352994-67-2] + TX, isoflucypram [1255734-28-1] + TX; rescalure [64309-03-1] + TX; aminopyrifen [1531626-08-0] + TX; tyclopyrazoflor [1477919-27-9] + TX; and spiropidion [1229023-00-0] + TX; and microbials including: *Acinetobacter Iwoffii* + TX, *Acremonium alternatum* + TX + TX, *Acremonium cephalosporium* + TX + TX, *Acremonium diospyri* + TX, *Acremonium obclavatum* + TX, *Adoxophyes orana granulovirus* (AdoxGV) (Capex^{®}) + TX, *Agrobacterium radiobacter* strain K84 (Galltrol-A^{®}) + TX, *Alternaria alternate* + TX, *Alternaria cassia* + TX, *Alternaria destruens* (Smolder^{®}) + TX, *Ampelomyces quisqualis* (AQ10^{®}) + TX, *Aspergillus flavus* AF36 (AF36^{®}) + TX, *Aspergillus flavus* NRRL 21882 (Aflaguard^{®}) + TX, *Aspergillus* spp. + TX, *Aureobasidium pullulans* + TX, *Azospirillum* + TX, (MicroAZ^{®} + TX, TAZO B^{®}) + TX, *Azotobacter* + TX, *Azotobacter chroocuccum* (Azotomeal^{®}) + TX, *Azotobacter* cysts (Bionatural Blooming Blossoms^{®}) + TX, *Bacillus amyloliquefaciens* + TX, *Bacillus cereus* + TX, *Bacillus chitinosporus* strain CM-1 + TX, *Bacillus chitinosporus* strain AQ746 + TX, *Bacillus licheniformis* strain HB-2 (Biostart^{™} Rhizoboost^{®}) + TX, *Bacillus licheniformis* strain 3086 (EcoGuard^{®} + TX, Green Releaf^{®}) + TX, *Bacillus circulans* + TX, *Bacillus firmus* (BioSafe^{®} + TX, BioNem-WP^{®} + TX, VOTiVO^{®}) + TX, *Bacillus firmus* strain I-1582 + TX, *Bacillus macerans* + TX, *Bacillus marismortui* + TX, *Bacillus megaterium* + TX, *Bacillus mycoides* strain AQ726 + TX, *Bacillus papillae* (Milky Spore Powder^{®}) + TX, *Bacillus pumilus* spp. + TX, *Bacillus pumilus* strain GB34 (Yield Shield^{®}) + TX, *Bacillus pumilus* strain AQ717 + TX, *Bacillus pumilus* strain QST 2808 (Sonata^{®} + TX, Ballad Plus^{®}) + TX, *Bacillus spahericus* (VectoLex^{®}) + TX, *Bacillus* spp. + TX, *Bacillus* spp. strain AQ175 + TX, *Bacillus* spp. strain AQ177 + TX, *Bacillus* spp. strain AQ178 + TX, *Bacillus subtilis* strain QST 713 (CEASE^{®} + TX, Serenade^{®} + TX, Rhapsody^{®}) + TX, *Bacillus subtilis* strain QST 714 (JAZZ^{®}) + TX, *Bacillus subtilis* strain AQ153 + TX, *Bacillus subtilis* strain AQ743 + TX, *Bacillus subtilis* strain QST3002 + TX, *Bacillus subtilis* strain QST3004 + TX, *Bacillus subtilis var. amyloliquefaciens* strain FZB24 (Taegro^{®} + TX, Rhizopro^{®}) + TX, *Bacillus thuringiensis* Cry 2Ae + TX, *Bacillus thuringiensis* CrylAb + TX, *Bacillus thuringiensis aizawai* GC 91 (Agree^{®}) + TX, *Bacillus thuringiensis israelensis* (BMP123^{®} + TX, Aquabac^{®} + TX, VectoBac^{®}) + TX, *Bacillus thuringiensis kurstaki* (Javelin^{®} + TX, Deliver^{®} + TX, CryMax^{®} + TX, Bonide^{®} + TX, Scutella WP^{®} + TX, Turilav WP ^{®} + TX, Astuto^{®} + TX, Dipel WP^{®} + TX, Biobit^{®} + TX, Foray^{®}) + TX, *Bacillus thuringiensis kurstaki* BMP 123 (Baritone^{®}) + TX, *Bacillus thuringiensis kurstaki* HD-1 (Bioprotec-CAF / 3P^{®}) + TX, *Bacillus thuringiensis* strain BD#32 + TX, *Bacillus thuringiensis* strain AQ52 + TX, *Bacillus thuringiensis var. aizawai* (XenTari^{®} + TX, DiPei^{®}) + TX, bacteria spp.
(GROWMEND^{®} + TX, GROWSWEET^{®} + TX, Shootup^{®}) + TX, bacteriophage of *Clavipacter michiganensis* (AgriPhage^{®}) + TX, Bakflor^{®} + TX, *Beauveria bassiana* (Beaugenic^{®} + TX, Brocaril WP^{®}) + TX, *Beauveria bassiana* GHA (Mycotrol ES^{®} + TX, Mycotrol O^{®} + TX, BotaniGuard^{®}) + TX, *Beauveria brongniartii* (Engerlingspilz^{®} + TX, Schweizer Beauveria^{®} + TX, Melocont^{®}) + TX, *Beauveria* spp. + TX, *Botrytis cineria* + TX, *Bradyrhizobium japonicum* (TerraMax^{®}) + TX, *Brevibacillus brevis* + TX, *Bacillus thuringiensis tenebrionis* (Novodor^{®}) + TX, BtBooster + TX, *Burkholderia cepacia* (Deny^{®} + TX, Intercept^{®} + TX, Blue Circle^{®}) + TX, *Burkholderia gladii* + TX, *Burkholderia gladioli* + TX, *Burkholderia* spp. + TX, Canadian thistle fungus (CBH Canadian Bioherbicide^{®}) + TX, *Candida butyri* + TX, *Candida famata* + TX, *Candida fructus* + TX, *Candida glabrata* + TX, *Candida guilliermondii* + TX, *Candida melibiosica* + TX, *Candida oleophila* strain O + TX, *Candida parapsilosis* + TX, *Candida pelliculosa* + TX, *Candida pulcherrima* + TX, *Candida reukaufii* + TX, *Candida saitoana* (Bio-Coat^{®} + TX, Biocure^{®}) + TX, *Candida sake* + TX, *Candida* spp. + TX, *Candida tenius* + TX, *Cedecea dravisae* + TX, *Cellulomonas flavigena* + TX, *Chaetomium cochliodes* (Nova-Cide^{®}) + TX, *Chaetomium globosum* (Nova-Cide^{®}) + TX, *Chromobacterium subtsugae* strain PRAA4-1T (Grandevo^{®}) + TX, *Cladosporium cladosporioides* + TX, *Cladosporium oxysporum* + TX, *Cladosporium chlorocephalum* + TX, *Cladosporium* spp. + TX, *Cladosporium tenuissimum* + TX, *Clonostachys rosea* (EndoFine^{®}) + TX, *Colletotrichum acutatum* + TX, *Coniothyrium minitans* (Cotans WG^{®}) + TX, *Coniothyrium* spp. + TX, *Cryptococcus albidus* (YIELDPLUS^{®}) + TX, *Cryptococcus humicola* + TX, *Cryptococcus infirmominiatus* + TX, *Cryptococcus laurentii* + TX, *Cryptophlebia leucotreta granulovirus* (Cryptex^{®}) + TX, *Cupriavidus campinensis* + TX, *Cydia pomonella granulovirus* (CYD-X^{®}) + TX, *Cydia pomonella granulovirus* (Madex^{®} + TX, Madex Plus^{®} + TX, Madex Maxi Carpovirusine^{®}) + TX, *Cylindrobasidium laeve* (Stumpout^{®}) + TX, *Cylindrocladium* + TX, *Debaryomyces hansenii* + TX, *Drechslera hawaiinensis* + TX, *Enterobacter cloacae* + TX, *Enterobacteriaceae* + TX, *Entomophtora virulenta* (Vektor^{®}) + TX, *Epicoccum nigrum* + TX, *Epicoccum purpurascens* + TX, *Epicoccum* spp. + TX, *Filobasidium floriforme* + TX, *Fusarium acuminatum* + TX, *Fusarium chlamydosporum* + TX, *Fusarium oxysporum* (Fusaclean^{®} / Biofox C^{®}) + TX, *Fusarium proliferatum* + TX, *Fusarium* spp. + TX, *Galactomyces geotrichum* + TX, *Gliocladium catenulatum* (Primastop^{®} + TX, Prestop^{®}) + TX, *Gliocladium roseum* + TX, *Gliocladium* spp. (SoilGard^{®}) + TX, *Gliocladium virens* (Soilgard^{®}) + TX, *Granulovirus* (Granupom^{®}) + TX, *Halobacillus halophilus* + TX, *Halobacillus litoralis* + TX, *Halobacillus trueperi* + TX, *Halomonas* spp. + TX, *Halomonas subglaciescola* + TX, *Halovibrio variabilis* + TX, *Hanseniaspora uvarum* + TX, *Helicoverpa armigera nucleopolyhedrovirus* (Helicovex^{®}) + TX, *Helicoverpa zea nuclear polyhedrosis virus* (Gemstar^{®}) + TX, Isoflavone - formononetin (Myconate^{®}) + TX, *Kloeckera apiculata* + TX, *Kloeckera* spp. + TX, *Lagenidium giganteum* (Laginex^{®}) + TX, *Lecanicillium longisporum* (Vertiblast^{®}) + TX, *Lecanicillium muscarium* (Vertikil^{®}) + TX, *Lymantria Dispar nucleopolyhedrosis virus* (Disparvirus^{®}) + TX, *Marinococcus halophilus* + TX, *Meira geulakonigii* + TX, *Metarhizium anisopliae* (Met52^{®}) + TX, *Metarhizium anisopliae* (Destruxin WP^{®}) + TX, *Metschnikowia fruticola* (Shemer^{®}) + TX, *Metschnikowia pulcherrima* + TX, *Microdochium dimerum* (Antibot^{®}) + TX, *Micromonospora coerulea* + TX, *Microsphaeropsis ochracea* + TX, *Muscodor albus* 620 (Muscudor^{®}) + TX, *Muscodor roseus* strain A3-5 + TX, *Mycorrhizae* spp. (AMykor^{®} + TX, Root Maximizer^{®}) + TX, *Myrothecium verrucaria* strain AARC-0255 (DiTera^{®}) + TX, BROS PLUS^{®} + TX, *Ophiostoma piliferum* strain D97 (Sylvanex^{®}) + TX, *Paecilomyces farinosus* + TX, *Paecilomyces fumosoroseus* (PFR-97^{®} + TX, PreFeRal^{®}) + TX, *Paecilomyces linacinus* (Biostat WP^{®}) + TX, *Paecilomyces lilacinus* strain 251 (MeloCon WG^{®}) + TX, *Paenibacillus polymyxa* + TX, *Pantoea agglomerans* (BlightBan C9-1^{®}) + TX, *Pantoea* spp. + TX, *Pasteuria* spp. (Econem^{®}) + TX, *Pasteuria nishizawae* + TX, *Penicillium aurantiogriseum* + TX,
*Penicillium billai* (Jumpstart^{®} + TX, TagTeam^{®}) + TX, *Penicillium brevicompactum* + TX, *Penicillium frequentans* + TX, *Penicillium griseofulvum* + TX, *Penicillium purpurogenum* + TX, *Penicillium* spp. + TX, *Penicillium viridicatum* + TX, *Phlebiopsis gigantean* (Rotstop^{®}) + TX, phosphate solubilizing bacteria (Phosphomeal^{®}) + TX, *Phytophthora cryptogea* + TX, *Phytophthora palmivora* (Devine^{®}) + TX, *Pichia anomala* + TX, *Pichia guilermondii* + TX, *Pichia membranaefaciens* + TX, *Pichia onychis* + TX, *Pichia stipites* + TX, *Pseudomonas aeruginosa* + TX, *Pseudomonas aureofasciens* (Spot-Less Biofungicide^{®}) + TX, *Pseudomonas cepacia* + TX, *Pseudomonas chlororaphis* (AtEze^{®}) + TX, *Pseudomonas corrugate* + TX, *Pseudomonas fluorescens* strain A506 (BlightBan A506^{®}) + TX, *Pseudomonas putida* + TX, *Pseudomonas reactans* + TX, *Pseudomonas* spp. + TX, *Pseudomonas syringae* (Bio-Save^{®}) + TX, *Pseudomonas viridiflava* + TX, *Pseudomons fluorescens* (Zequanox^{®}) + TX, *Pseudozyma flocculosa* strain PF-A22 UL (Sporodex L^{®}) + TX, *Puccinia canaliculata* + TX, *Puccinia thlaspeos* (Wood Warrior^{®}) + TX, *Pythium paroecandrum* + TX, *Pythium oligandrum* (Polygandron^{®} + TX, Polyversum^{®}) + TX, *Pythium periplocum* + TX, *Rhanella aquatilis* + TX, *Rhanella* spp. + TX, *Rhizobia* (Dormal^{®} + TX, Vault^{®}) + TX, *Rhizoctonia* + TX, *Rhodococcus globerulus* strain AQ719 + TX, *Rhodosporidium diobovatum* + TX, *Rhodosporidium toruloides* + TX, *Rhodotorula* spp. + TX, *Rhodotorula glutinis* + TX, *Rhodotorula graminis* + TX, *Rhodotorula mucilagnosa* + TX, *Rhodotorula rubra* + TX, *Saccharomyces cerevisiae* + TX, *Salinococcus roseus* + TX, *Sclerotinia minor* + TX, *Sclerotinia minor* (SARRITOR^{®}) + TX, *Scytalidium* spp. + TX, *Scytalidium uredinicola* + TX, *Spodoptera exigua nuclear polyhedrosis virus* (Spod-X^{®} + TX, Spexit^{®}) + TX, *Serratia marcescens* + TX, *Serratia plymuthica* + TX, *Serratia* spp. + TX, *Sordaria fimicola* + TX, *Spodoptera littoralis nucleopolyhedrovirus* (Littovir^{®}) + TX, *Sporobolomyces roseus* + TX, *Stenotrophomonas maltophilia* + TX, *Streptomyces ahygroscopicus* + TX, *Streptomyces albaduncus* + TX, *Streptomyces exfoliates* + TX, *Streptomyces galbus* + TX, *Streptomyces griseoplanus* + TX, *Streptomyces griseoviridis* (Mycostop^{®}) + TX, *Streptomyces lydicus* (Actinovate^{®}) + TX, *Streptomyces lydicus* WYEC-108 (ActinoGrow^{®}) + TX, *Streptomyces violaceus* + TX, *Tilletiopsis minor* + TX, *Tilletiopsis* spp. + TX, *Trichoderma asperellum* (T34 Biocontrol^{®}) + TX, *Trichoderma gamsii* (Tenet^{®}) + TX, *Trichoderma atroviride* (Plantmate^{®}) + TX, *Trichoderma hamatum* TH 382 + TX, *Trichoderma harzianum rifai* (Mycosta^{®}) + TX, *Trichoderma harzianum* T-22 (Trianum-P^{®} + TX, PlantShield HC^{®} + TX, Rootshield^{®} + TX, Trianum-G^{®}) + TX, *Trichoderma harzianum* T-39 (Trichodex^{®}) + TX, *Trichoderma inhamatum* + TX, *Trichoderma koningii* + TX, *Trichoderma* spp. LC 52 (Sentinels) + TX, *Trichoderma lignorum* + TX, *Trichoderma longibrachiatum* + TX, *Trichoderma polysporum* (Binab T^{®}) + TX, *Trichoderma taxi* + TX, *Trichoderma virens* + TX, *Trichoderma virens* (formerly Gliocladium virens GL-21) (SoilGuard^{®}) + TX, *Trichoderma viride* + TX, *Trichoderma viride* strain ICC 080 (Remedier^{®}) + TX, *Trichosporon pullulans* + TX, *Trichosporon* spp. + TX, *Trichothecium* spp. + TX, *Trichothecium roseum* + TX, *Typhula phacorrhiza* strain 94670 + TX, *Typhula phacorrhiza* strain 94671 + TX, *Ulocladium atrum* + TX, *Ulocladium oudemansii* (Botry-Zen^{®}) + TX, *Ustilago maydis* + TX, various bacteria and supplementary micronutrients (Natural II^{®}) + TX, various fungi (Millennium Microbes^{®}) + TX, *Verticillium chlamydosporium* + TX, *Verticillium lecanii* (Mycotal^{®} + TX, Vertalec^{®}) + TX, Vip3Aa20 (VIPtera^{®}) + TX, *Virgibaclillus marismortui* + TX, *Xanthomonas campestris pv. Poae* (Camperico^{®}) + TX, *Xenorhabdus bovienii* + TX, *Xenorhabdus nematophilus;* and
Plant extracts including: pine oil (Retenol^{®}) + TX, azadirachtin (Plasma Neem Oil^{®} + TX, AzaGuard^{®} + TX, MeemAzal^{®} + TX, Molt-X^{®} + TX, Botanical IGR (Neemazad^{®} + TX, Neemix^{®}) + TX, canola oil (Lilly Miller Vegol^{®}) + TX, *Chenopodium ambrosioides near ambrosioides* (Requiem^{®}) + TX, *Chrysanthemum* extract (Crisant^{®}) + TX, extract of neem oil (Trilogy^{®}) + TX, essentials oils of *Labiatae* (Botania^{®}) + TX, extracts of clove rosemary peppermint and thyme oil (Garden insect killer^{®}) + TX, Glycinebetaine (Greenstim^{®}) + TX, garlic + TX, lemongrass oil (GreenMatch^{®}) + TX, neem oil + TX, *Nepeta cataria* (Catnip oil) + TX, *Nepeta catarina* + TX, nicotine + TX, oregano oil (MossBuster^{®}) + TX, *Pedaliaceae* oil (Nematon^{®}) + TX, pyrethrum + TX, *Quillaja saponaria* (NemaQ^{®}) + TX, *Reynoutria sachalinensis* (Regalia^{®} + TX, Sakalia^{®}) + TX, rotenone (Eco Roten^{®}) + TX, *Rutaceae* plant extract (Soleo^{®}) + TX, soybean oil (Ortho ecosense^{®}) + TX, tea tree oil (Timorex Gold^{®}) + TX, thymus oil + TX, AGNIQUE^{®} MMF + TX, BugOil^{®} + TX, mixture of rosemary sesame pepermint thyme and cinnamon extracts (EF 300^{®}) + TX, mixture of clove rosemary and peppermint extract (EF 400^{®}) + TX, mixture of clove pepermint garlic oil and mint (Soil Shot^{®}) + TX, kaolin (Screen^{®}) + TX, storage glucam of brown algae (Laminarin^{®}); and
pheromones including: blackheaded fireworm pheromone (3M Sprayable Blackheaded Fireworm Pheromone^{®}) + TX, Codling Moth Pheromone (Paramount dispenser-(CM)/ Isomate C-Plus^{®}) + TX, Grape Berry Moth Pheromone (3M MEC-GBM Sprayable Pheromone^{®}) + TX, Leafroller pheromone (3M MEC - LR Sprayable Pheromone^{®}) + TX, Muscamone (Snip7 Fly Bait^{®} + TX, Starbar Premium Fly Bait^{®}) + TX, Oriental Fruit Moth Pheromone (3M oriental fruit moth sprayable pheromones) + TX, Peachtree Borer Pheromone (Isomate-P^{®}) + TX, Tomato Pinworm Pheromone (3M Sprayable pheromone^{®}) + TX, Entostat powder (extract from palm tree) (Exosex CM^{®}) + TX, (E + TX,Z + TX,Z)-3 + TX,8 + TX,11 Tetradecatrienyl acetate + TX, (Z + TX,Z + TX,E)-7 + TX,11 + TX,13-Hexadecatrienal + TX, (E + TX,Z)-7 + TX,9-Dodecadien-1-yl acetate + TX, 2-Methyl-1-butanol + TX, Calcium acetate + TX, Scenturion^{®} + TX, Biolure^{®} + TX, Check-Mate^{®} + TX, Lavandulyl senecioate; and
Macrobials including: *Aphelinus abdominalis* + TX, *Aphidius ervi* (Aphelinus-System^{®}) + TX, *Acerophagus papaya* + TX, *Adalia bipunctata* (Adalia-System^{®}) + TX, *Adalia bipunctata* (Adaline^{®}) + TX, *Adalia bipunctata* (Aphidalia^{®}) + TX, *Ageniaspis citricola* + TX, *Ageniaspis fuscicollis* + TX, *Amblyseius andersoni* (Anderline^{®} + TX, Andersoni-System^{®}) + TX, *Amblyseius californicus* (Amblyline^{®} + TX, Spical^{®}) + TX, *Amblyseius cucumeris* (Thripex^{®} + TX, Bugline cucumeris^{®}) + TX, *Amblyseius fallacis* (Fallacis^{®}) + TX, *Amblyseius swirskii* (Bugline swirskii^{®} + TX, Swirskii-Mite^{®}) + TX, *Amblyseius womersleyi* (WomerMite^{®}) + TX, *Amitus hesperidum* + TX, *Anagrus atomus* + TX, *Anagyrus fusciventris* + TX, *Anagyrus kamali* + TX, *Anagyrus loecki* + TX, *Anagyrus pseudococci* (Citripar^{®}) + TX, *Anicetus benefices* + TX, *Anisopteromalus calandrae* + TX, *Anthocoris nemoralis* (Anthocoris-System^{®}) + TX, *Aphelinus abdominalis* (Apheline^{®} + TX, Aphiline^{®}) + TX, *Aphelinus asychis* + TX, *Aphidius colemani* (Aphipar^{®}) + TX, *Aphidius ervi* (Ervipar^{®}) + TX, *Aphidius gifuensis +* TX, *Aphidius matricariae* (Aphipar-M^{®}) + TX, *Aphidoletes aphidimyza* (Aphidend^{®}) + TX, *Aphidoletes aphidimyza* (Aphidoline^{®}) + TX, *Aphytis lingnanensis* + TX, *Aphytis melinus* + TX, *Aprostocetus hagenowii* + TX, *Atheta coriaria* (Staphyline^{®}) + TX, *Bombus* spp. + TX, *Bombus terrestris* (Natupol Beehive^{®}) + TX, *Bombus terrestris* (Beeline^{®} + TX, Tripol^{®}) + TX, *Cephalonomia stephanoderis* + TX, *Chilocorus nigritus* + TX, *Chrysoperla carnea* (Chrysoline^{®}) + TX, *Chrysoperla carnea* (Chrysopa^{®}) + TX, *Chrysoperla rufilabris* + TX, *Cirrospilus ingenuus* + TX, *Cirrospilus quadristriatus* + TX, *Citrostichus phyllocnistoides* + TX, *Closterocerus chamaeleon* + TX, *Closterocerus* spp. + TX, *Coccidoxenoides perminutus* (Pianopar^{®}) + TX, *Coccophagus cowperi* + TX, *Coccophagus lycimnia* + TX, *Cotesia flavipes* + TX, *Cotesia plutellae* + TX, *Cryptolaemus montrouzieri* (Cryptobug^{®} + TX, Cryptoline^{®}) + TX, *Cybocephalus nipponicus* + TX, *Dacnusa sibirica* + TX, *Dacnusa sibirica* (Minusa^{®}) + TX, *Diglyphus isaea* (Diminex^{®}) + TX, *Delphastus catalinae* (Delphastus^{®}) + TX, *Delphastus pusillus* + TX, *Diachasmimorpha krausii* + TX, *Diachasmimorpha longicaudata* + TX, *Diaparsis jucunda* + TX, *Diaphorencyrtus aligarhensis* + TX, *Diglyphus isaea* + TX, *Diglyphus isaea* (Miglyphus^{®} + TX, Digline^{®}) + TX, *Dacnusa sibirica* (DacDigline^{®} + TX, Minex^{®}) + TX, *Diversinervus* spp. + TX, *Encarsia citrina* + TX, *Encarsia formosa* (Encarsia max^{®} + TX, Encarline^{®} + TX, En-Strip^{®}) + TX, *Eretmocerus eremicus* (Enermix^{®}) + TX, *Encarsia guadeloupae* + TX, *Encarsia haitiensis* + TX, *Episyrphus balteatus* (Syrphidend^{®}) + TX, *Eretmoceris siphonini* + TX, *Eretmocerus californicus* + TX, *Eretmocerus eremicus* (Ercal^{®} + TX, Eretline e^{®}) + TX, *Eretmocerus eremicus* (Bemimix^{®}) + TX, *Eretmocerus hayati* + TX, *Eretmocerus mundus* (Bemipar^{®} + TX, Eretline m^{®}) + TX, *Eretmocerus siphonini* + TX, *Exochomus quadripustulatus* + TX, *Feltiella acarisuga* (Spidend^{®}) + TX, *Feltiella acarisuga* (Feltiline^{®}) + TX, *Fopius arisanus* + TX, *Fopius ceratitivorus* + TX, Formononetin (Wirless Beehome^{®}) + TX, *Franklinothrips vespiformis* (Vespop^{®}) + TX, *Galendromus occidentalis* + TX, *Goniozus legneri* + TX, *Habrobracon hebetor* + TX, *Harmonia axyridis* (HarmoBeetle^{®}) + TX, *Heterorhabditis* spp. (Lawn Patrol^{®}) + TX, *Heterorhabditis bacteriophora* (NemaShield HB^{®} + TX, Nemaseek^{®} + TX, Terranem-Nam^{®} + TX, Terranem^{®} + TX, Larvanem^{®} + TX, B-Green^{®} + TX, NemAttack ^{®} + TX, Nematop^{®}) + TX, *Heterorhabditis megidis* (Nemasys H^{®} + TX, BioNem H^{®} + TX, Exhibitline hm^{®} + TX, Larvanem-M^{®}) + TX, *Hippodamia convergens* + TX, *Hypoaspis aculeifer* (Aculeifer-System^{®} + TX, Entomite-A^{®}) + TX, *Hypoaspis miles* (Hypoline m^{®} + TX, Entomite-M^{®}) + TX, *Lbalia leucospoides* + TX, *Lecanoideus floccissimus* + TX, *Lemophagus errabundus* + TX, *Leptomastidea abnormis* + TX, *Leptomastix dactylopii* (Leptopar^{®}) + TX, *Leptomastix epona* + TX, *Lindorus lophanthae* + TX, *Lipolexis oregmae* + TX, *Lucilia caesar* (Natufly^{®}) + TX, *Lysiphlebus testaceipes* + TX, *Macrolophus caliginosus* (Mirical-N^{®} + TX, Macroline c^{®} + TX, Mirical^{®}) + TX, *Mesoseiulus longipes* + TX, *Metaphycus flavus* + TX, *Metaphycus lounsburyi* + TX, *Micromus angulatus* (Milacewing^{®}) + TX, *Microterys flavus* + TX, *Muscidifurax raptorellus* and *Spalangia cameroni* (Biopar^{®}) + TX, *Neodryinus typhlocybae* + TX, *Neoseiulus californicus* + TX, *Neoseiulus cucumeris* (THRYPEX^{®}) + TX, *Neoseiulus fallacis* + TX, *Nesideocoris tenuis* (NesidioBug^{®} + TX, Nesibug^{®}) + TX, *Ophyra aenescens* (Biofly^{®}) + TX, *Orius insidiosus* (Thripor-I^{®} + TX, Online i^{®}) + TX, *Orius laevigatus* (Thripor-L^{®} + TX, Online I^{®}) + TX, *Onus majusculus* (Oriline m^{®}) + TX, *Orius strigicollis* (Thripor-S^{®}) + TX, *Pauesia juniperorum* + TX, *Pediobius foveolatus* + TX, *Phasmarhabditis hermaphrodita* (Nemaslug^{®}) + TX, *Phymastichus coffea* + TX, *Phytoseiulus macropilus* + TX, *Phytoseiulus persimilis* (Spidex^{®} + TX, Phytoline p^{®}) + TX, *Podisus maculiventris* (Podisus^{®}) + TX, *Pseudacteon curvatus* + TX, *Pseudacteon obtusus* + TX, *Pseudacteon tricuspis* + TX, *Pseudaphycus maculipennis* + TX, *Pseudleptomastix mexicana* + TX, *Psyllaephagus pilosus* + TX, *Psy(talia concolor* (complex) + TX, *Quadrastichus* spp. + TX, *Rhyzobius lophanthae* + TX, *Rodolia cardinalis* + TX, *Rumina decollate* + TX, *Semielacher petiolatus* + TX, *Sitobion avenae* (Ervibank^{®}) + TX, *Steinernema carpocapsae* (Nematac C^{®} + TX, Millenium^{®} + TX, BioNem C^{®} + TX, NemAttack^{®} + TX, Nemastar^{®} + TX, Capsanem^{®}) + TX, *Steinernema feltiae* (NemaShield^{®} + TX, Nemasys F^{®} + TX, BioNem F^{®} + TX, Steinernema-System^{®} + TX, NemAttack^{®} + TX, Nemaplus^{®} + TX, Exhibitline st^{®} + TX, Scia-rid^{®} + TX, Entonem^{®}) + TX, *Steinernema kraussei* (Nemasys L^{®} + TX, BioNem L^{®} + TX, Exhibitline srb^{®}) + TX, *Steinernema riobrave* (BioVector^{®} + TX, BioVektor^{®}) + TX, *Steinernema scapterisci* (Nematac S^{®}) + TX, *Steinernema* spp. + TX, *Steinernematid* spp. (Guardian Nematodes^{®}) + TX, *Stethorus punctillum* (Stethorus^{®}) + TX, *Tamarixia radiate* + TX, *Tetrastichus setifer* + TX, *Thripobius semiluteus* + TX, *Torymus sinensis* + TX, *Trichogramma brassicae* (Tricholine b^{®}) + TX, *Trichogramma brassicae* (Tricho-Strip^{®}) + TX, *Trichogramma evanescens* + TX, *Trichogramma minutum* + TX, *Trichogramma ostriniae* + TX, *Trichogramma platneri* + TX, *Trichogramma pretiosum* + TX, *Xanthopimpla stemmator;* and
other biologicals including: abscisic acid + TX, bioSea^{®} + TX, *Chondrostereum purpureum* (Chontrol Paste^{®}) + TX, *Colletotrichum gloeosporioides* (Collego^{®}) + TX, Copper Octanoate (Cueva^{®}) + TX, Delta traps (Trapline d^{®}) + TX, Erwinia amylovora (Harpin) (ProAct^{®} + TX, Ni-HIBIT Gold CST^{®}) + TX, Ferri-phosphate (Ferramol^{®}) + TX, Funnel traps (Trapline y^{®}) + TX, Gallex^{®} + TX, Grower's Secret^{®} + TX, Homo-brassonolide + TX, Iron Phosphate (Lilly Miller Worry Free Ferramol Slug & Snail Bait^{®}) + TX, MCP hail trap (Trapline f^{®}) + TX, *Microctonus hyperodae* + TX, *Mycoleptodiscus terrestris* (Des-X^{®}) + TX, BioGain^{®} + TX, Aminomite^{®} + TX, Zenox^{®} + TX, Pheromone trap (Thripline ams^{®}) + TX, potassium bicarbonate (MilStop^{®}) + TX, potassium salts of fatty acids (Sanova^{®}) + TX, potassium silicate solution (Sil-Matrix^{®}) + TX, potassium iodide + potassiumthiocyanate (Enzicur^{®}) + TX, SuffOil-X^{®} + TX, Spider venom + TX, *Nosema locustae* (Semaspore Organic Grasshopper Control^{®}) + TX, Sticky traps (Trapline YF^{®} + TX, Rebell Amarillo^{®}) + TX and Traps (Takitrapline y + b^{®}) + TX,
or a biologically active compound or agent selected from: Brofluthrinate + TX, Diflovidazine + TX, Flometoquin + TX, Fluhexafon + TX, Plutella xylostella Granulosis virus + TX, Cydia pomonella Granulosis virus + TX, Imicyafos + TX, Heliothis virescens Nucleopolyhedrovirus + TX, Heliothis punctigera Nucleopolyhedrovirus + TX, Helicoverpa zea Nucleopolyhedrovirus + TX, Spodoptera frugiperda Nucleopolyhedrovirus + TX, Plutella xylostella Nucleopolyhedrovirus + TX, p-cymene + TX, Pyflubumide + TX, Pyrafluprole + TX, QRD 420 + TX, QRD 452 + TX, QRD 460 + TX, Terpenoid blends + TX, Terpenoids + TX, Tetraniliprole + TX, and α-terpinene + TX;
or an active substance referenced by a code + TX, such as code AE 1887196 (BSC-BX60309) + TX, code NNI-0745 GR + TX, code IKI-3106 + TX, code JT-L001 + TX, code ZNQ-08056 + TX, code IPPA152201 + TX, code HNPC-A9908 (CAS: [660411-21-2]) + TX, code HNPC-A2005 (CAS: [860028-12-2]) + TX, code JS118 + TX, code ZJ0967 + TX, code ZJ2242 + TX, code JS7119 (CAS: [929545-74-4]) + TX, code SN-1172 + TX, code HNPC-A9835 + TX, code HNPC-A9955 + TX, code HNPC-A3061 + TX, code Chuanhua 89-1 + TX, code IPP-10 + TX, code ZJ3265 + TX, code JS9117 + TX, code ZJ3757 + TX, code ZJ4042 + TX, code ZJ4014 + TX, code ITM-121 + TX, code DPX-RAB55 (DKI-2301) + TX, code NA-89 + TX, code MIE-1209 + TX, code MCI-8007 + TX, code BCS-CL73507 + TX, code S-1871 + TX, code DPX-RDS63 + TX, Quinofumelin + TX, mefentrifluconazol + TX, fenpicoxamid + TX, fluindapyr + TX, flufenpyrrolidone + TX, inpyrfluxam + TX or indiflumetpyr + TX, isoflucypram + TX, isocycloseram + TX, pyrapropoyne + TX, florylpicoxamid + TX, metyltetraprole + TX, ipflufenoquin + TX, pyridachlometyl + TX or chlopyridiflu + TX, tetrachlorantraniliprole + TX, tetrachloraniliprole + TX, Tetflupyrolimet + TX, Triflufenpyrrolidone + TX, Tyclopyrazoflor + TX, flupyrimin + TX or pyrifluramide + TX, benzpyrimoxan + TX, beflubutamid-M + TX, Benzosufyl + TX or oxazosulfyl + TX, cyetpyrafen + TX, etpyrafen + TX or, acynonapyr + TX or pyrinonafen + TX, oxotrione + TX, bixlozone + TX or clofendizone + TX or dicloroxizone + TX, cyclopyranil + TX or pyrazocyclonil + TX or cyclopyrazonil + TX , alpha-bromadiolone + TX, code AKD-1193 + TX, Oxathiapiprolin + TX, Fluopyram + TX, Penflufen+ TX, Fluoxopyrosad+ TX, fluoxapiprolin + TX, dimesulfazet + TX, cyclobutrifluram + TX, flubeneteram + TX, flupentiofenox + TX and Flupyradifurone + TX.

The references in brackets behind the active ingredients, e.g. *[3878-19-1]* refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula I selected from Tables A-1 to A-81 and Tables B-1 to B-54 and Table P with active ingredients described above comprises a compound selected from Tables A-1 to A-81 and Tables B-1 to B-54 and Table P and an active ingredient as described above preferably in a mixing ratio of from 100:1 to 1 :6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound of formula I selected from Tables A-1 to A-81 and Tables B-1 to B-54 and Table P and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I selected from Tables A-1 to A-81 and Tables B-1 to B-54 Table P and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of the invention and compositions thereof are also be suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound of formula I. The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula (I). Further, it is hereby made available, a composition comprising a plant propagation material treated with a compound of formula (I).

Seed treatment comprises all suitable seed treatment techniques known in the art, such as seed dressing, seed coating, seed dusting, seed soaking and seed pelleting. The seed treatment application of the compound formula (I) can be carried out by any known methods, such as spraying or by dusting the seeds before sowing or during the sowing/planting of the seeds.

### Biological Examples:

The Examples which follow serve to illustrate the invention. Certain compounds of the invention can be distinguished from known compounds by virtue of greater efficacy at low application rates, which can be verified by the person skilled in the art using the experimental procedures outlined in the Examples, using lower application rates if necessary, for example 50 ppm, 12.5 ppm, 6 ppm, 3 ppm, 1.5 ppm, 0.8 ppm or 0.2 ppm.

### Example B1: Activity against Bemisia tabaci (Cotton white fly)

Cotton leaf discs were placed on agar in 24-well microtiter plates and sprayed with aqueous test solutions prepared from 10'000 ppm DMSO stock solutions. After drying the leaf discs were infested with adult white flies. The samples were checked for mortality 6 days after incubation.

The following compounds resulted in at least 80% mortality at an application rate of 200 ppm: P1, P2, P9, P11, P13, P14 and P16.

### Example B2: Activity against Diabrotica balteata (Corn root worm)

Maize sprouts placed onto an agar layer in 24-well microtiter plates were treated with aqueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.

The following compounds gave an effect of at least 80% in at least one of the two categories (mortality or growth inhibition) at an application rate of 200 ppm:
P1, P2, P5, P9, P11, P13, P14, P16, P18 and P20.

### Example B3: Activity against Euschistus heros (Neotropical Brown Stink Buq)

Soybean leaves on agar in 24-well microtiter plates were sprayed with aqueous test solutions prepared from 10'000 ppm DMSO stock solutions. After drying the leaves were infested with N2 nymphs. The samples were assessed for mortality and growth inhibition in comparison to untreated samples 5 days after infestation.

The following compounds gave an effect of at least 80% in at least one of the two categories (mortality or growth inhibition) at an application rate of 200 ppm:
P1, P2, P5, P11, P13, P14, P15, P16, P18 and P20.

### Example B4: Activity against Plutella xylostella (Diamond back moth)

24-well microtiter plates with artificial diet were treated with aqueous test solutions prepared from 10'000 ppm DMSO stock solutions by pipetting. After drying, Plutella eggs were pipetted through a plastic stencil onto a gel blotting paper and the plate was closed with it. The samples were assessed for mortality and growth inhibition in comparison to untreated samples 8 days after infestation.

The following compounds gave an effect of at least 80% in at least one of the two categories (mortality or growth inhibition) at an application rate of 200 ppm:
P1, P2, P5, P9, P11, P13, P14, P16, P18 and P20.

### Example B5: Activity against Mvzus persicae (Green peach aphid) Feeding/Contact activity

Sunflower leaf discs were placed onto agar in a 24-well microtiter plate and sprayed with aqueous test solutions prepared from 10'000 ppm DMSO stock solutions. After drying, the leaf discs were infested with an aphid population of mixed ages. The samples were assessed for mortality 6 days after infestation.

The following compounds resulted in at least 80% mortality at an application rate of 200 ppm:
P1, P2, P5, P9, P11, P14, P15, P16, P18 and P20.

### Example B6: Activity against Mvzus persicae (Green peach aphid) Systemic activity

Roots of pea seedlings infested with an aphid population of mixed ages were placed directly into aqueous test solutions prepared from 10'000 DMSO stock solutions. The samples were assessed for mortality 6 days after placing seedlings into test solutions.

The following compounds resulted in at least 80% mortality at a test rate of 24 ppm:
P1, P2, P5, P9, P11, P13, P14, P15, P16, P18 and P20.

### Example B7: Activity against Spodoptera littoralis (Eqyptian cotton leaf worm)

Cotton leaf discs were placed onto agar in 24-well microtiter plates and sprayed with aqueous test solutions prepared from 10'000 ppm DMSO stock solutions. After drying the leaf discs were infested with five L1 larvae. The samples were assessed for mortality, anti-feeding effect, and growth inhibition in comparison to untreated samples 3 days after infestation. Control of Spodoptera littoralis by a test sample is given when at least one of the categories mortality, anti-feedant effect, and growth inhibition is higher than the untreated sample.

The following compounds resulted in at least 80% control at an application rate of 200 ppm:
P2, P11, P13, P14, P16, P18 and P20.

## Claims

1. A compound of formula (I) 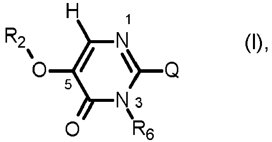 wherein
R₂ is C₁-C₆haloalkyl, C₂-C₆haloalkenyl or C₁-C₄haloalkylsulfonyl;
R₆ is C₁-C₆alkyl;
Q is a radical selected from the group consisting of formula Qa and Qb 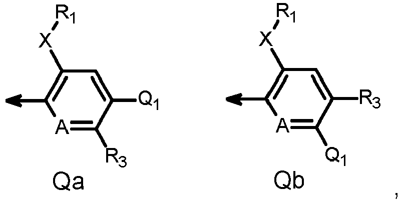
wherein the arrow denotes the point of attachment to the pyrimidinone ring;
and wherein A represents CH or N;
X is S, SO, SO₂ or SO(NH);
R₁ is C₁-C₄alkyl or C₃-C₆cycloalkyl-C₁-C₄alkyl;
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆cyanoalkoxy, C₁-C₆haloalkoxy, -N(R₄)₂, -N(R₄)COR₅, -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono- or polysubstituted by substituents selected from the group consisting of halogen, cyano, C₁-C₄alkyl, C₁-C₄haloalkyl, C₁-C₄alkoxy, C₁-C₄haloalkoxy, C₁-C₄alkylsulfanyl, C₁-C₄alkylsulfinyl and C₁-C₄alkylsulfonyl; and said ring system can contain 1, 2 or 3 ring heteroatoms selected from the group consisting of nitrogen, oxygen and sulphur, where said ring system may not contain more than one ring oxygen atom and not more than one ring sulfur atom; or
Q₁ is a five-membered aromatic ring system linked via a ring nitrogen atom to the ring which contains the substituent A, said ring system is unsubstituted or is mono- or polysubstituted by substituents selected from the group consisting of halogen, cyano, C₁-C₄alkyl, C₁-C₄haloalkyl, C₁-C₄alkoxy, C₁-C₄haloalkoxy, C₁-C₄alkylsulfanyl, C₁-C₄alkylsulfinyl and C₁-C₄alkylsulfonyl; and said ring system contains 1, 2 or 3 ring heteroatoms selected from the group consisting of nitrogen, oxygen and sulphur, where said ring system contains at least one ring nitrogen atom and may not contain more than one ring oxygen atom and not more than one ring sulfur atom;
R₃ is hydrogen, halogen or C₁-C₄alkyl;
R₄ is independently hydrogen, C₁-C₄alkyl or C₃-C₆cycloalkyl;
R₅ is C₁-C₆alkyl, C₁-C₆haloalkyl or C₃-C₆cycloalkyl;
or an agrochemically acceptable salt, stereoisomer, enantiomer, tautomer or N-oxide of a compound of formula I.

2. A compound of formula I according to claim 1, wherein
R₂ is C₁-C₆haloalkyl, preferably -CH₂CF₂CHF₂ or -CH₂CF₂CF₃;
R₆ is C₁-C₃alkyl, preferably methyl;
Q is a radical selected from the group consisting of formula Qa and Qb, wherein
A is N; and
Q₁ is hydrogen, halogen, C₃-C₆cycloalkyl, C₃-C₆cycloalkyl monosubstituted by cyano, C₁-C₆cyanoalkyl, - N(R₄)₂, -N(R₄)COR₅ or -N(R₄)CON(R₄)₂, all in which R₄ is independently either hydrogen or C₁-C₄alkyl (preferably hydrogen or methyl) and R₅ is C₁-C₆alkyl or C₃-C₆cycloalkyl (preferably methyl or cyclopropyl), 2-pyridyloxy, or 1,2,4-triazol-1-yl; preferably Q₁ is hydrogen, chlorine, bromine, cyclopropyl, 1-cyanocyclopropyl, 1-cyano-1-methyl-ethyl, -NH(CH₃), -N(CH₃)COCH₃, -N(CH₃)CO(cyclopropyl), - N(CH₃)CONH(CH₃), 2-pyridyloxy or 1,2,4-triazol-1-yl.

3. A compound of formula I according to claim 1, wherein
R₂ is C₁-C₆haloalkyl, preferably -CH₂CF₂CHF₂ or -CH₂CF₂CF₃;
R₆ is methyl;
Q is a radical selected from the group consisting of formula Qa and Qb, wherein
A is N; and
Q₁ is hydrogen, bromine, cyclopropyl, 1-cyanocyclopropyl, 1-cyano-1-methyl-ethyl, 2-pyridyloxy or-N(CH₃)COCH₃ when Q is Qa; or
Q₁ is hydrogen, chlorine, -NH(CH₃), -N(CH₃)COCH₃, -N(CH₃)CO(cyclopropyl), -N(CH₃)CONH(CH₃) or
1,2,4-triazol-1-yl, when Q is Qb.

4. A compound of formula I according to claim 1, represented by the compounds of formula (I-2) 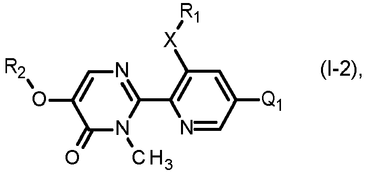
wherein X, R₁ and R₂ are as defined under formula I in claim 1, and wherein
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the pyridyl ring substituted by X-R₁, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the pyridyl ring substituted by X-R₁, said ring system is unsubstituted or is mono-substituted by substituents selected from the group consisting of halogen, cyano and C₁-C₄haloalkyl; and said ring system contains 2 or 3 ring nitrogen atoms;
each R₄ is independently preferably hydrogen or C₁-C₄alkyl; and
R₅ is preferably C₁-C₆alkyl or C₃-C₆cycloalkyl.

5. A compound of formula I according to claim 1, represented by the compounds of formula (I-3) 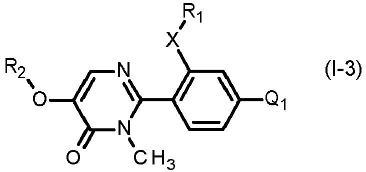
wherein X, R₁ and R₂ are as defined under formula I in claim 1, and wherein
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the phenyl ring substituted by X-R₁, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the phenyl ring substituted by X-R₁, said ring system is unsubstituted or is mono-substituted by substituents selected from the group consisting of halogen, cyano and C₁-C₄haloalkyl; and said ring system contains 2 or 3 ring nitrogen atoms;
each R₄ is independently preferably hydrogen or C₁-C₄alkyl; and
R₅ is preferably C₁-C₆alkyl or C₃-C₆cycloalkyl.

6. A compound of formula I according to claim 1, represented by the compounds of formula (I-4) 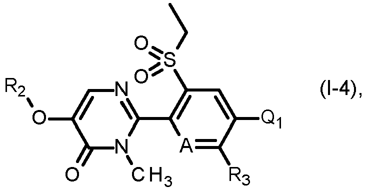 wherein
A is CH or N, preferably N;
R₂ is C₁-C₆haloalkyl, preferably -CH₂CF₂CHF₂ or -CH₂CF₂CF₃;
R₃ is hydrogen or C₁-C₄alkyl, preferably hydrogen or methyl;
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the ring which contains the substituent A, said ring system is unsubstituted or is mono-substituted by substituents selected from the group consisting of halogen, cyano and C₁-C₄haloalkyl; and said ring system contains 2 or 3 ring nitrogen atoms;
each R₄ is independently hydrogen or C₁-C₄alkyl, preferably hydrogen or methyl; and
R₅ is C₁-C₆alkyl or C₃-C₆cycloalkyl, preferably methyl or cyclopropyl.

7. A compound of formula I according to claim 1, represented by the compounds of formula (I-6) 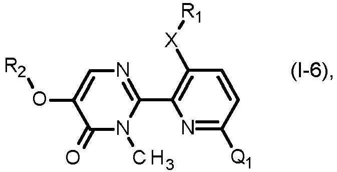
wherein X, R₁ and R₂ are as defined under formula I in claim 1, and wherein
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the pyridyl ring substituted by X-R₁, said ring system is unsubstituted or is mono-substituted by substituents selected from the group consisting of halogen, cyano and C₁-C₄haloalkyl; and said ring system can contain 1 or 2 nitrogen atoms; or
Q₁ is a five-membered aromatic ring system linked via a ring nitrogen atom to the pyridyl ring substituted by X-R₁, said ring system is unsubstituted or is mono-substituted by substituents selected from the group consisting of halogen, cyano and C₁-C₄haloalkyl; and said ring system contains 2 or 3 nitrogen atoms;
R₄ is independently preferably hydrogen or C₁-C₄alkyl; and
R₅ is preferably C₁-C₆alkyl or C₃-C₆cycloalkyl.

8. A compound of formula I according to claim 1, represented by the compounds of formula (I-7) 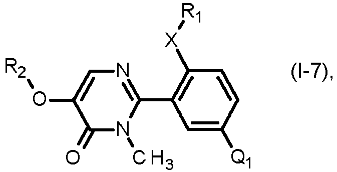
wherein X, R₁ and R₂ under formula I in claim 1, and wherein
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the phenyl ring substituted by X-R₁, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the phenyl ring substituted by X-R₁, said ring system is unsubstituted or is mono-substituted by substituents selected from the group consisting of halogen, cyano and C₁-C₄haloalkyl; and said ring system contains 2 or 3 ring nitrogen atoms;
R₄ is independently preferably hydrogen or C₁-C₄alkyl; and
R₅ is preferably C₁-C₆alkyl or C₃-C₆cycloalkyl.

9. A compound of formula I according to claim 1, represented by the compounds of formula (I-8) 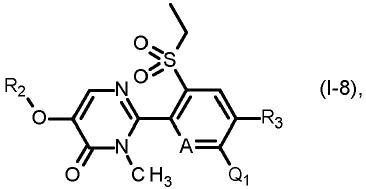 wherein
A is CH or N, preferably N;
R₂ is C₁-C₆haloalkyl, preferably -CH₂CF₂CHF₂ or -CH₂CF₂CF₃;
R₃ is hydrogen or C₁-C₄alkyl, preferably hydrogen or methyl;
Q₁ is hydrogen, halogen, C₁-C₆haloalkyl, C₃-C₆cycloalkyl, C₃-C₆cycloalkyl monosubstituted by cyano, C₁-C₆cyanoalkyl, C₁-C₆haloalkoxy, -N(R₄)₂, -N(R₄)COR₅, or -N(R₄)CON(R₄)₂, (oxazolidin-2-one)-3-yl or 2-pyridyloxy; or
Q₁ is a five- to six-membered aromatic ring system linked via a ring carbon atom to the ring which contains the substituent A, said ring system is unsubstituted or is mono-substituted by substituents selected from the group consisting of halogen, cyano and C₁-C₄haloalkyl; and said ring system can contain 1 or 2 ring nitrogen atoms; or
Q₁ is a five-membered aromatic ring system linked via a ring nitrogen atom to the ring which contains the substituent A, said ring system can be mono-substituted by substituents selected from the group consisting of halogen, cyano or C₁-C₄haloalkyl; and said ring system contains 2 or 3 ring nitrogen atoms;
R₄ is independently hydrogen or C₁-C₄alkyl, preferably hydrogen or methyl; and
R₅ is C₁-C₆alkyl or C₃-C₆cycloalkyl, preferably methyl or cyclopropyl.

10. A compound of formula I as claimed in any one of claims 1 - 7, wherein R₂ is -CH₂CF₂CHF₂ or-CH₂CF₂CF₃.

11. A compound of formula I as claimed in any one of claims 1 - 7, wherein
Q₁ is hydrogen, halogen, trifluoromethyl, cyclopropyl, cyanocyclopropyl, cyanoisopropyl, trifluoroethoxy, -N(R₄)₂, -N(R₄)COR₅, or -N(R₄)CON(R₄)₂, in each of which R₄ is independently either hydrogen or methyl and R₅ is either methyl or cyclopropyl, or Q₁ is (oxazolidin-2-one)-3-yl, 2-pyridyloxy, N-linked pyrazolyl which can be mono-substituted by chloro, cyano or trifluoromethyl; or Q₁ is N-linked triazolyl or C-linked pyrimidinyl.

12. A compound of formula I as claimed in any one of claims 1 - 7, wherein
Q₁ is hydrogen, chlorine, bromine, trifluoromethyl, cyclopropyl, 1-cyanocyclopropyl, 1-cyano-1-methylethyl, 2,2,2-trifluoroethoxy, -NH(CH₃), -N(CH₃)COCH₃, -N(CH₃)CO(cyclopropyl), -N(H)CONH(CH₃), - N(CH₃)CONH(CH₃), (oxazolidin-2-one)-3-yl, 2-pyridyloxy, pyrazol-1-yl, 3-chloro-pyrazol-1-yl, 3-cyano-pyrazol-1-yl, 3-trifluoromethyl-pyrazol-1-yl, 1,2,4-triazol-1-yl or pyrimidin-2-yl.

13. A compound of formula I as claimed in claims 1, 6 or 9, wherein
A is N; and
Q₁ is hydrogen, chlorine, bromine, cyclopropyl, 1-cyanocyclopropyl, 1-cyano-1-methyl-ethyl, -NH(CH₃), - N(CH₃)COCH₃, -N(CH₃)CO(cyclopropyl), -N(CH₃)CONH(CH₃), 2-pyridyloxy or 1,2,4-triazol-1-yl.

14. A compound of formula I as claimed in claim 1, selected from the group consisting of:
2-(3-ethylsulfanyl-2-pyridyl)-3-methyl-5-(2,2,3,3,3-pentafluoropropoxy)pyrimidin-4-one (Compound P1);
2-(3-ethylsulfonyl-2-pyridyl)-3-methyl-5-(2,2,3,3,3-pentafluoropropoxy)pyrimidin-4-one (Compound P2);
2-(5-bromo-3-ethylsulfanyl-2-pyridyl)-3-methyl-5-(2,2,3,3,3-pentafluoropropoxy)pyrimidin-4-one (Compound P3);
N-[5-ethylsulfanyl-6-[1-methyl-6-oxo-5-(2,2,3,3,3-pentafluoropropoxy)pyrimidin-2-yl]-3-pyridyl]-N-methylacetamide (Compound P4);
N-[5-ethylsulfonyl-6-[1-methyl-6-oxo-5-(2,2,3,3,3-pentafluoropropoxy)pyrimidin-2-yl]-3-pyridyl]-N-methylacetamide (Compound P5);
2-(6-chloro-3-ethylsulfanyl-2-pyridyl)-3-methyl-5-(2,2,3,3,3-pentafluoropropoxy)pyrimidin-4-one (Compound P6);
2-(6-chloro-3-ethylsulfonyl-2-pyridyl)-3-methyl-5-(2,2,3,3,3-pentafluoropropoxy)pyrimidin-4-one (Compound P7);
2-[3-ethylsulfonyl-6-(methylamino)-2-pyridyl]-3-methyl-5-(2,2,3,3,3-pentafluoropropoxy)pyrimidin-4-one (Compound P8);
N-[5-ethylsulfonyl-6-[1-methyl-6-oxo-5-(2,2,3,3,3-pentafluoropropoxy)pyrimidin-2-yl]-2-pyridyl]-N-methylacetamide (Compound P9);
1-[5-ethylsulfanyl-6-[1-methyl-6-oxo-5-(2,2,3,3,3-pentafluoropropoxy)pyrimidin-2-yl]-3-pyridyl]cyclopropanecarbonitrile (Compound P10);
1-[5-ethylsu Ifonyl-6-[1-methyl-6-oxo-5-(2,2,3,3, 3-pentaflu oropropoxy) pyrimid in-2-yl]-3-pyridyl]cyclopropanecarbonitrile (Compound P11);
2-[5-ethylsulfanyl-6-[1-methyl-6-oxo-5-(2,2,3,3,3-pentafluoropropoxy)pyrimidin-2-yl]-3-pyridyl]-2-methylpropanenitrile (Compound P12);
2-[5-ethylsulfonyl-6-[1-methyl-6-oxo-5-(2,2,3,3,3-pentafluoropropoxy)pyrimidin-2-yl]-3-pyridyl]-2-methylpropanenitrile (Compound P13);
2-[3-ethylsulfonyl-6-(1,2,4-triazol-1-yl)-2-pyridyl]-3-methyl-5-(2,2,3,3,3-pentafluoropropoxy)pyrimidin-4-one (Compound P14);
1-[5-ethylsu Ifonyl-6-[1-methyl-6-oxo-5-(2,2,3,3, 3-pentaflu oropropoxy) pyrimid in-2-yl]-2-pyridyl]-1 ,3-dimethyl-urea (Compound P15);
N-[5-ethylsulfonyl-6-[1-methyl-6-oxo-5-(2,2,3,3,3-pentafluoropropoxy)pyrimidin-2-yl]-2-pyridyl]-N-methyl-cyclopropanecarboxamide (Compound P16);
2-(5-cyclopropyl-3-ethylsulfanyl-2-pyridyl)-3-methyl-5-(2,2,3,3,3-pentafluoropropoxy)pyrimidin-4-one (Compound P17);
2-(5-cyclopropyl-3-ethylsulfonyl-2-pyridyl)-3-methyl-5-(2,2,3,3,3-pentafluoropropoxy)pyrimidin-4-one (Compound P18);
2-[3-ethylsulfanyl-5-(2-pyridyloxy)-2-pyridyl]-3-methyl-5-(2,2,3,3,3-pentafluoropropoxy)pyrimidin-4-one (Compound P19); and
2-[3-ethylsulfonyl-5-(2-pyridyloxy)-2-pyridyl]-3-methyl-5-(2,2,3,3,3-pentafluoropropoxy)pyrimidin-4-one (Compound P20).

15. A composition comprising an insecticidally, acaricidally, nematicidally or molluscicidally effective amount of a compound of formula (I), or an agrochemically acceptable salt, stereoisomer, enantiomer, tautomer or N-oxide thereof, as defined in any of claims 1 - 14 and, optionally, an auxiliary or diluent.

16. A method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I), or an agrochemically acceptable salt, stereoisomer, enantiomer, tautomer or N-oxide thereof, as defined in any of claims 1 - 14 or a composition as defined claim 15; with the exception of a method applied to the human or animal body.

17. A method for the protection of plant propagation material from the attack by insects, acarines, nematodes or molluscs, which comprises treating the propagation material or the site, where the propagation material is planted, with a composition according to claim 15.

18. A compound of formula V 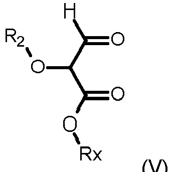
or a tautomeric form thereof, wherein
R₂ is C₁-C₆fluoroalkyl, and Rx is C₁-C₆alkyl.

## Patentansprüche

1. Verbindung der Formel (I) 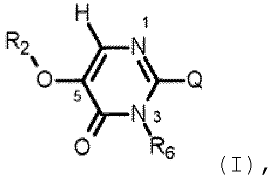 wobei
R₂ für C₁-C₆-Halogenalkyl, C₂-C₆-Halogenalkenyl oder C₁-C₄-Halogenalkylsulfonyl steht;
R₆ für C₁-C₆-Alkyl steht;
Q für einen Rest steht, der aus der Gruppe bestehend aus Formel Qa und Qb ausgewählt ist: 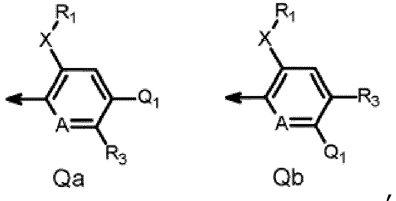
wobei der Pfeil den Verknüpfungspunkt mit dem Pyrimidinonring anzeigt;
und wobei A für CH oder N steht;
X für S, SO, SO₂ oder SO(NH) steht;
R₁ für C₁-C₄-Alkyl oder für C₃-C₆-Cycloalkyl-C₁-C₄-alkyl steht;
Q₁ für Wasserstoff, Halogen, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Cyanoalkoxy, C₁-C₆-Halogenalkoxy, -N(R₄)₂, -N(R₄)COR₅, -N(R₄)CON(R₄)₂, (Oxazolidin-2-on)-3-yl oder 2-Pyridyloxy steht oder
Q₁ für ein fünf- bis sechsgliedriges aromatisches Ringsystem steht, das über ein Ringkohlenstoffatom an den Ring, der den Substituenten A enthält, gebunden ist, wobei das Ringsystem unsubstituiert oder ein- oder mehrfach durch Substituenten, die aus der Gruppe bestehend aus Halogen, Cyano, C₁-C₄-Alkyl, C₁-C₄-Halogenalkyl, C₁-C₄-Alkoxy, C₁-C₄-Halogenalkoxy, C₁-C₄-Alkylsulfanyl, C₁-C₄-Alkylsulfinyl und C₁-C₄-Alkylsulfonyl ausgewählt sind, substituiert ist und das Ringsystem 1, 2 oder 3 Ringheteroatome, die aus der Gruppe bestehend aus Stickstoff, Sauerstoff und Schwefel ausgewählt sind, enthalten kann, wobei das Ringsystem nicht mehr als ein Ringsauerstoffatom und nicht mehr als ein Ringschwefelatom enthalten kann; oder
Q₁ für ein fünfgliedriges aromatisches Ringsystem steht, das über ein Ringstickstoffatom an den Ring, der den Substituenten A enthält, gebunden ist, wobei das Ringsystem unsubstituiert oder ein- oder mehrfach durch Substituenten, die aus der Gruppe bestehend aus Halogen, Cyano, C₁-C₄-Alkyl, C₁-C₄-Halogenalkyl, C₁-C₄-Alkoxy, C₁-C₄-Halogenalkoxy, C₁-C₄-Alkylsulfanyl, C₁-C₄-Alkylsulfinyl und C₁-C₄-Alkylsulfonyl ausgewählt sind, substituiert ist und das Ringsystem 1, 2 oder 3 Ringheteroatome, die aus der Gruppe bestehend aus Stickstoff, Sauerstoff und Schwefel ausgewählt sind, enthält, wobei das Ringsystem mindestens ein Ringstickstoffatom enthält und nicht mehr als ein Ringsauerstoffatom und nicht mehr als ein Ringschwefelatom enthalten kann;
R₃ für Wasserstoff, Halogen oder C₁-C₄-Alkyl steht;
R₄ jeweils unabhängig für Wasserstoff, C₁-C₄-Alkyl oder C₃-C₆-Cycloalkyl steht;
R₅ für C₁-C₆-Alkyl, C₁-C₆-Halogenalkyl oder C₃-C₆-Cycloalkyl steht;
oder ein agrochemisch unbedenkliches Salz, Stereoisomer, Enantiomer, Tautomer oder N-Oxid einer Verbindung der Formel I.

2. Verbindung der Formel I nach Anspruch 1, wobei
R₂ für C₁-C₆-Halogenalkyl, vorzugsweise -CH₂CF₂CHF₂ oder - CH₂CF₂CF₃, steht;
R₆ für C₁-C₃-Alkyl, vorzugsweise Methyl, steht;
Q für einen Rest steht, der aus der Gruppe bestehend aus Formel Qa und Qb ausgewählt ist, wobei
A für N steht und
Q₁ für Wasserstoff, Halogen, C₃-C₆-Cycloalkyl, C₃-C₆-Cycloalkyl, das einfach durch Cyano substituiert ist, C₁-C₆-Cyanoalkyl, -N(R₄)₂, -N(R₄)COR₅ oder -N(R₄)CON(R₄)₂ steht, wobei in allen diesen Gruppen R₄ unabhängig für Wasserstoff oder C₁-C₄-Alkyl (vorzugsweise Wasserstoff oder Methyl) steht und R₅ für C₁-C₆-Alkyl oder C₃-C₆-Cycloalkyl (vorzugsweise Methyl oder Cyclopropyl) steht, 2-Pyridyloxy oder 1,2,4-Triazol-1-yl steht; vorzugsweise Q₁ für Wasserstoff, Chlor, Brom, Cyclopropyl, 1-Cyanocyclopropyl, 1-Cyano-1-methylethyl, -NH(CH₃), - N(CH₃)COCH₃, -N(CH₃)CO(Cyclopropyl), -N(CH₃)CONH(CH₃), 2-Pyridyloxy oder 1,2,4-Triazol-1-yl steht.

3. Verbindung der Formel I nach Anspruch 1, wobei
R₂ für C₁-C₆-Halogenalkyl, vorzugsweise -CH₂CF₂CHF₂ oder - CH₂CF₂CF₃, steht;
R₆ für Methyl steht;
Q für einen Rest steht, der aus der Gruppe bestehend aus Formel Qa und Qb ausgewählt ist, wobei
A für N steht und
Q₁ für Wasserstoff, Brom, Cyclopropyl, 1-Cyanocyclopropyl, 1-Cyano-1-methylethyl, 2-Pyridyloxy oder -N(CH₃)COCH₃ steht, wenn Q für Qa steht; oder
Q₁ für Wasserstoff, Chlor, -NH(CH₃), -N(CH₃)COCH₃, - N (CH₃)CO(Cyclopropyl), -N(CH₃)CONH (CH₃)oder 1,2,4-Triazol-1-yl steht, wenn Q für Qb steht.

4. Verbindung der Formel I nach Anspruch 1, wiedergegeben durch die Verbindungen der Formel (1-2) 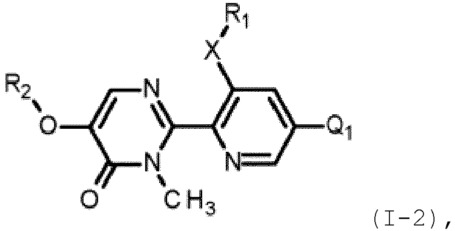
wobei X, R₁ und R₂ wie unter Formel I in Anspruch 1 definiert sind und wobei
Q₁ für Wasserstoff, Halogen, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Halogenalkoxy, -N(R₄)₂, -N(R₄)COR₅, -N(R₄)CON (R₄)₂, (Oxazolidin-2-on) -3-yl oder 2-Pyridyloxy steht oder
Q₁ für ein fünf- bis sechsgliedriges aromatisches Ringsystem steht, das über ein Ringkohlenstoffatom an den Pyridylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 1 oder 2 Ringstickstoffatome enthalten kann; oder
Q₁ für ein fünfgliedriges aromatisches Ringsystem steht, das über ein Ringstickstoffatom an den Pyridylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 2 oder 3 Ringstickstoffatome enthält;
R⁴ vorzugsweise jeweils unabhängig für Wasserstoff oder C₁-C₄-Alkyl steht und
R₅ vorzugsweise für C₁-C₆-Alkyl oder C₃-C₆-Cycloalkyl steht.

5. Verbindung der Formel I nach Anspruch 1, wiedergegeben durch die Verbindungen der Formel (1-3) 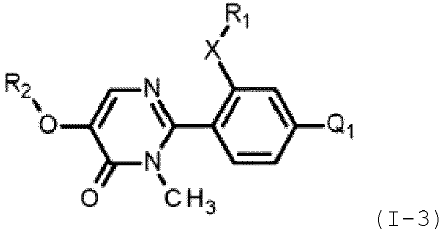
wobei X, R₁ und R₂ wie unter Formel I in Anspruch 1 definiert sind und wobei
Q₁ für Wasserstoff, Halogen, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Halogenalkoxy, -N(R₄)₂, -N(R₄)COR₅, -N(R₄)CON (R₄)₂, (Oxazolidin-2-on) -3-yl oder 2-Pyridyloxy steht oder
Q₁ für ein fünf- bis sechsgliedriges aromatisches Ringsystem steht, das über ein Ringkohlenstoffatom an den Phenylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 1 oder 2 Ringstickstoffatome enthalten kann; oder
Q₁ für ein fünfgliedriges aromatisches Ringsystem steht, das über ein Ringstickstoffatom an den Phenylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 2 oder 3 Ringstickstoffatome enthält;
R⁴ vorzugsweise jeweils unabhängig für Wasserstoff oder C₁-C₄-Alkyl steht und
R₅ vorzugsweise für C₁-C₆-Alkyl oder C₃-C₆-Cycloalkyl steht.

6. Verbindung der Formel I nach Anspruch 1, wiedergegeben durch die Verbindungen der Formel (1-4) 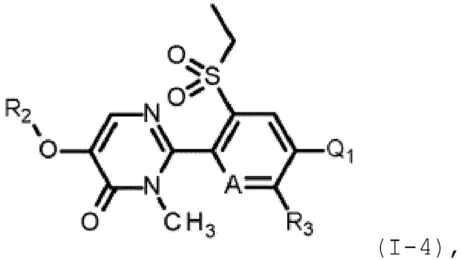 wobei
A für CH oder N, vorzugsweise N, steht;
R₂ für C₁-C₆-Halogenalkyl, vorzugsweise -CH₂CF₂CHF₂ oder - CH₂CF₂CF₃, steht;
R₃ für Wasserstoff oder C₁-C₄-Alkyl, vorzugsweise Wasserstoff oder Methyl, steht;
Q₁ für Wasserstoff, Halogen, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Halogenalkoxy, -N(R₄)₂, -N(R₄)COR₅, -N(R₄)CON(R₄)₂, (Oxazolidin-2-on)-3-yl oder 2-Pyridyloxy steht oder
Q₁ für ein fünf- bis sechsgliedriges aromatisches Ringsystem steht, das über ein Ringkohlenstoffatom an den Ring, der den Substituenten A enthält, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 1 oder 2 Ringstickstoffatome enthalten kann; oder
Q₁ für ein fünfgliedriges aromatisches Ringsystem steht, das über ein Ringstickstoffatom an den Ring, der den Substituenten A enthält, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 2 oder 3 Ringstickstoffatome enthält;
R⁴ jeweils unabhängig für Wasserstoff oder C₁-C₄-Alkyl, vorzugsweise Wasserstoff oder Methyl, steht und
R₅ für C₁-C₆-Alkyl oder C₃-C₆-Cycloalkyl, vorzugsweise Methyl oder Cyclopropyl, steht.

7. Verbindung der Formel I nach Anspruch 1, wiedergegeben durch die Verbindungen der Formel (1-6) 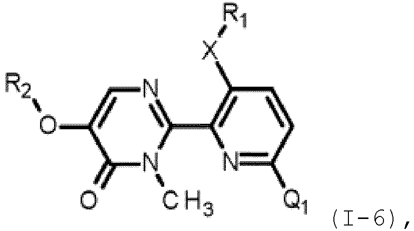
wobei X, R₁ und R₂ wie unter Formel I in Anspruch 1 definiert sind und wobei
Q₁ für Wasserstoff, Halogen, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Halogenalkoxy, -N(R₄)₂, -N(R₄)COR₅, -N(R₄)CON (R₄)₂, (Oxazolidin-2-on) -3-yl oder 2-Pyridyloxy steht oder
Q₁ für ein fünf- bis sechsgliedriges aromatisches Ringsystem steht, das über ein Ringkohlenstoffatom an den Pyridylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 1 oder 2 Stickstoffatome enthalten kann; oder
Q₁ für ein fünfgliedriges aromatisches Ringsystem steht, das über ein Ringstickstoffatom an den Pyridylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 2 oder 3 Stickstoffatome enthält;
R⁴ vorzugsweise jeweils unabhängig für Wasserstoff oder C₁-C₄-Alkyl steht und
R₅ vorzugsweise für C₁-C₆-Alkyl oder C₃-C₆-Cycloalkyl steht.

8. Verbindung der Formel I nach Anspruch 1, wiedergegeben durch die Verbindungen der Formel (1-7) 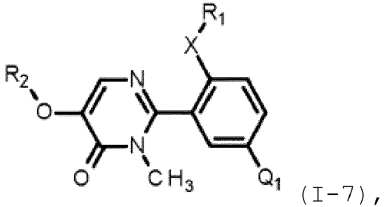
wobei X, R₁ und R₂ unter Formel I in Anspruch 1 und wobei Q₁ für Wasserstoff, Halogen, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Halogenalkoxy, -N(R₄)₂, -N(R₄)COR₅, -N(R₄)CON(R₄)₂, (Oxazolidin-2-on) -3-yl oder 2-Pyridyloxy steht oder
Q₁ für ein fünf- bis sechsgliedriges aromatisches Ringsystem steht, das über ein Ringkohlenstoffatom an den Phenylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 1 oder 2 Ringstickstoffatome enthalten kann; oder
Q₁ für ein fünfgliedriges aromatisches Ringsystem steht, das über ein Ringstickstoffatom an den Phenylring, der durch X-R₁ substituiert ist, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 2 oder 3 Ringstickstoffatome enthält;
R⁴ vorzugsweise jeweils unabhängig für Wasserstoff oder C₁-C₄-Alkyl steht und
R₅ vorzugsweise für C₁-C₆-Alkyl oder C₃-C₆-Cycloalkyl steht.

9. Verbindung der Formel I nach Anspruch 1, wiedergegeben durch die Verbindungen der Formel (1-8) 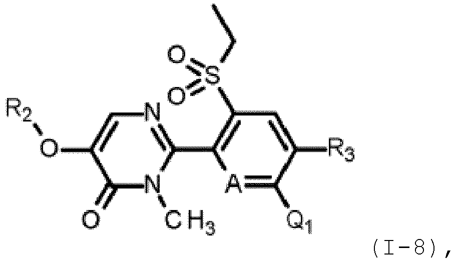 wobei
A für CH oder N, vorzugsweise N, steht;
R₂ für C₁-C₆-Halogenalkyl, vorzugsweise -CH₂CF₂CHF₂ oder - CH₂CF₂CF₃, steht;
R₃ für Wasserstoff oder C₁-C₄-Alkyl, vorzugsweise Wasserstoff oder Methyl, steht;
Q₁ für Wasserstoff, Halogen, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Halogenalkoxy, -N(R₄)₂, -N(R₄)COR₅ oder -N(R₄)CON(R₄)₂, (Oxazolidin-2-on)-3-yl oder 2-Pyridyloxy steht; oder
Q₁ für ein fünf- bis sechsgliedriges aromatisches Ringsystem steht, das über ein Ringkohlenstoffatom an den Ring, der den Substituenten A enthält, gebunden ist, wobei das Ringsystem unsubstituiert oder einfach durch Substituenten, die aus der Gruppe bestehend aus Halogen, Cyano und C₁-C₄-Halogenalkyl ausgewählt sind, substituiert ist und das Ringsystem 1 oder 2 Ringstickstoffatome enthalten kann; oder
Q₁ für ein fünfgliedriges aromatisches Ringsystem steht, das über ein Ringstickstoffatom an den Ring, der den Substituenten A enthält, wobei das Ringsystem einfach durch Substituenten, die aus der Gruppe bestehend aus Halogen, Cyano und C₁-C₄-Halogenalkyl ausgewählt sind, substituiert ist und das Ringsystem 2 oder 3 Ringstickstoffatome enthält;
R⁴ jeweils unabhängig für Wasserstoff oder C₁-C₄-Alkyl, vorzugsweise Wasserstoff oder Methyl, steht und
R₅ für C₁-C₆-Alkyl oder C₃-C₆-Cycloalkyl, vorzugsweise Methyl oder Cyclopropyl, steht.

10. Verbindung der Formel I nach einem der Ansprüche 1-7, wobei R₂ für -CH₂CF₂CHF₂ oder -CH₂CF₂CF₃ steht.

11. Verbindung der Formel I nach einem der Ansprüche 1-7, wobei
Q₁ für Wasserstoff, Halogen, Trifluormethyl, Cyclopropyl, Cyanocyclopropyl, Cyanoisopropyl, Trifluorethoxy, - N(R₄)₂, -N(R₄)COR₅ oder -N(R₄)CON(R₄)₂ steht, wobei R₄ jeweils unabhängig für Wasserstoff oder Methyl steht und R₅ für Methyl oder Cyclopropyl steht, oder Q₁ für (Oxazolidin-2-on)-3-yl, 2-Pyridyloxy, N-verknüpftes Pyrazolyl, das einfach durch Chlor, Cyano oder Trifluormethyl substituiert sein kann, steht oder Q₁ für N-verknüpftes Triazolyl oder C-verknüpftes Pyrimidinyl steht.

12. Verbindung der Formel I nach einem der Ansprüche 1-7, wobei
Q₁ für Wasserstoff, Chlor, Brom, Trifluormethyl, Cyclopropyl, 1-Cyanocyclopropyl, 1-Cyano-1-methylethyl, 2,2,2-Trifluorethoxy, -NH(CH₃), -N(CH₃)COCH₃, - N(CH₃)CO(Cyclopropyl), -N(H)CONH(CH₃), -N(CH₃)CONH(CH₃), (Oxazolidin-2-on)-3-yl, 2-Pyridyloxy, Pyrazol-1-yl, 3-Chlorpyrazol-1-yl, 3-Cyanopyrazol-1-yl, 3-Trifluormethylpyrazol-1-yl, 1,2,4-Triazol-1-yl oder Pyrimidin-2-yl steht.

13. Verbindung der Formel I nach den Ansprüchen 1, 6 oder 9, wobei
A für N steht und
Q₁ für Wasserstoff, Chlor, Brom, Cyclopropyl, 1-Cyanocyclopropyl, 1-Cyano-1-methylethyl, -NH(CH₃), - N(CH₃)COCH₃, -N(CH₃)CO(Cyclopropyl), -N(CH₃)CONH(CH₃), 2-Pyridyloxy oder 1,2,4-Triazol-1-yl steht.

14. Verbindung der Formel I nach Anspruch 1, ausgewählt aus der Gruppe bestehend aus:
2-(3-Ethylsulfanyl-2-pyridyl)-3-methyl-5-(2,2,3,3,3-pentafluorpropoxy)pyrimidin-4-on (Verbindung P1);
2-(3-Ethylsulfonyl-2-pyridyl)-3-methyl-5-(2,2,3,3,3-pentafluorpropoxy)pyrimidin-4-on (Verbindung P2);
2-(5-Brom-3-ethylsulfanyl-2-pyridyl)-3-methyl-5-(2,2,3,3,3-pentafluorpropoxy)pyrimidin-4-on (Verbindung P3);
N-[5-Ethylsulfanyl-6-[1-methyl-6-oxo-5-(2,2,3,3,3-pentafluorpropoxy)pyrimidin-2-yl]-3-pyridyl]-N-methylacetamid (Verbindung P4);
N-[5-Ethylsulfonyl-6-[1-methyl-6-oxo-5-(2,2,3,3,3-pentafluorpropoxy)pyrimidin-2-yl]-3-pyridyl]-N-methylacetamid (Verbindung P5);
2-(6-Chlor-3-ethylsulfanyl-2-pyridyl)-3-methyl-5-(2,2,3,3,3-pentafluorpropoxy)pyrimidin-4-on (Verbindung P6) ;
2-(6-Chlor-3-ethylsulfonyl-2-pyridyl)-3-methyl-5-(2,2,3,3,3-pentafluorpropoxy)pyrimidin-4-on (Verbindung P7) ;
2-[3-Ethylsulfonyl-6-(methylamino)-2-pyridyl]-3-methyl-5-(2,2,3,3,3-pentafluorpropoxy)pyrimidin-4-on (Verbindung P8);
N-[5-Ethylsulfonyl-6-[1-methyl-6-oxo-5-(2,2,3,3,3-pentafluorpropoxy)pyrimidin-2-yl]-2-pyridyl]-N-methylacetamid (Verbindung P9);
1-[5-Ethylsulfanyl-6-[1-methyl-6-oxo-5-(2,2,3,3,3-pentafluorpropoxy)pyrimidin-2-yl]-3-pyridyl]cyclopropancarbonitril (Verbindung P10);
1-[5-Ethylsulfonyl-6-[1-methyl-6-oxo-5-(2,2,3,3,3-pentafluorpropoxy)pyrimidin-2-yl]-3-pyridyl]cyclopropancarbonitril (Verbindung P11);
2-[5-ethylsulfanyl-6-[1-methyl-6-oxo-5-(2,2,3,3,3-pentafluorpropoxy)pyrimidin-2-yl]-3-pyridyl]-2-methylpropannitril (Verbindung P12);
2-[5-Ethylsulfonyl-6-[1-methyl-6-oxo-5-(2,2,3,3,3-pentafluorpropoxy)pyrimidin-2-yl]-3-pyridyl]-2-methylpropannitril (Verbindung P13);
2-[3-Ethylsulfonyl-6-(1,2,4-triazol-1-yl)-2-pyridyl]-3-methyl-5-(2,2,3,3,3-pentafluorpropoxy)pyrimidin-4-on (Verbindung P14);
1-[5-Ethylsulfonyl-6-[1-methyl-6-oxo-5-(2,2,3,3,3-pentafluorpropoxy)pyrimidin-2-yl]-2-pyridyl]-1,3-dimethylharnstoff (Verbindung P15);
N-[5-Ethylsulfonyl-6-[1-methyl-6-oxo-5-(2,2,3,3,3-pentafluorpropoxy)pyrimidin-2-yl]-2-pyridyl]-N-methyl-cyclopropancarboxamid (Verbindung P16);
2-(5-Cyclopropyl-3-ethylsulfanyl-2-pyridyl)-3-methyl-5-(2,2,3,3,3-pentafluorpropoxy)pyrimidin-4-on (Verbindung P17);
2-(5-Cyclopropyl-3-ethylsulfonyl-2-pyridyl)-3-methyl-5-(2,2,3,3,3-pentafluorpropoxy)pyrimidin-4-on (Verbindung P18) ;
2-[3-Ethylsulfanyl-5-(2-pyridyloxy)-2-pyridyl]-3-methyl-5-(2,2,3,3,3-pentafluorpropoxy)pyrimidin-4-on (Verbindung P19) und
2-[3-Ethylsulfonyl-5-(2-pyridyloxy)-2-pyridyl]-3-methyl-5-(2,2,3,3,3-pentafluorpropoxy)pyrimidin-4-on (Verbindung P20).

15. Zusammensetzung, umfassend eine insektizid, akarizid, nematizid oder molluskizid wirksame Menge einer Verbindung der Formel (I) oder eines agrochemisch unbedenklichen Salzes, Stereoisomers, Enantiomers, Tautomers oder N-Oxids davon gemäß einem der Ansprüche 1-14 und gegebenenfalls einen Hilfsstoff oder ein Verdünnungsmittel.

16. Verfahren zur Bekämpfung und Kontrolle von Insekten, Acarina, Nematoden oder Mollusken, bei dem man eine insektizid, akarizid, nematizid oder molluskizid wirksame Menge einer Verbindung der Formel (I) oder eines agrochemisch unbedenklichen Salzes, Stereoisomers, Enantiomers, Tautomers oder N-Oxids davon gemäß einem der Ansprüche 1-14 oder eine Zusammensetzung gemäß Anspruch 15 auf einen Schädling, einen Standort eines Schädlings oder eine Pflanze, die gegenüber Befall durch einen Schädling empfindlich ist, ausbringt; mit Ausnahme eines auf den menschlichen oder tierischen Körper angewendeten Verfahrens.

17. Verfahren zum Schutz von Pflanzenfortpflanzungsmaterial gegen Befall durch Insekten, Acarina, Nematoden oder Mollusken, bei dem man das Fortpflanzungsmaterial oder die Stelle, an der das Fortpflanzungsmaterial angepflanzt ist, mit einer Zusammensetzung nach Anspruch 15 behandelt.

18. Verbindung der Formel V 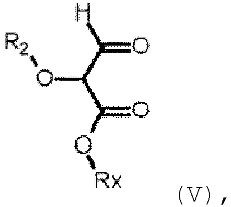
oder eine tautomere Form davon, wobei
R₂ für C₁-C₆-Fluoralkyl steht und Rx für C₁-C₆-Alkyl steht.

## Revendications

1. Composé de formule (I) 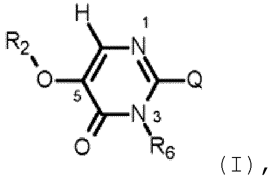
R₂ étant C₁-C₆halogénoalkyle, C₂-C₆halogénoalcényle ou C₁-C₄halogénoalkylsulfonyle ;
R₆ étant C₁-C₆alkyle ;
Q étant un radical choisi dans le groupe constitué par la formule Qa et Qb 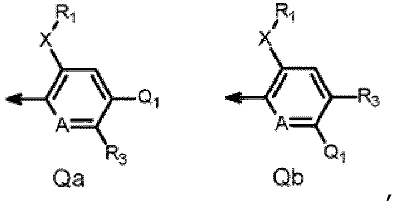
la flèche désignant le point de fixation au cycle pyrimidinone ;
et A représentant CH ou N ;
X étant S, SO, SO₂ ou SO(NH) ;
R₁ étant C₁-C₄alkyle ou C₃-C₆cycloalkyl-C₁-C₄alkyle ;
Q₁ étant hydrogène, halogène, C₁-C₆halogénoalkyle, C₃-C₆cycloalkyle, C₃-C₆cycloalkyle monosubstitué par cyano, C₁-C₆cyanoalkyle, C₁-C₆cyanoalcoxy, C₁-C₆halogénoalcoxy, - N(R₄)₂, -N(R₄)COR₅, -N(R₄)CON(R₄)₂, (oxazolidin-2-one)-3-yle ou 2-pyridinyloxy ; ou
Q₁ étant un système cyclique aromatique à cinq à six chaînons lié via un atome de carbone de cycle au cycle qui contient le substituant A, ledit système cyclique étant non substitué ou étant monosubstitué ou polysubstitué par des substituants choisis dans le groupe constitué par halogène, cyano, C₁-C₄alkyle, C₁-C₄halogénoalkyle, C₁-C₄alcoxy, C₁-C₄halogénoalcoxy, C₁-C₄alkylsulfanyle, C₁-C₄alkylsulfinyle et C₁-C₄alkylsulfonyle ; et ledit système cyclique pouvant contenir 1, 2 ou 3 hétéroatomes de cycle choisis dans le groupe constitué par azote, oxygène et soufre, où ledit système cyclique ne peut pas contenir plus d'un atome d'oxygène de cycle et pas plus d'un atome de soufre de cycle ; ou
Q₁ étant un système cyclique aromatique à cinq chaînons lié via un atome d'azote de cycle au cycle qui contient le substituant A, ledit système cyclique étant non substitué ou étant monosubstitué ou polysubstitué par des substituants choisis dans le groupe constitué par halogène, cyano, C₁-C₄alkyle, C₁-C₄halogénoalkyle, C₁-C₄alcoxy, C₁-C₄halogénoalcoxy, C₁-C₄alkylsulfanyle, C₁-C₄alkylsulfinyle et C₁-C₄alkylsulfonyle ; et ledit système cyclique contenant 1, 2 ou 3 hétéroatomes de cycle choisis dans le groupe constitué par azote, oxygène et soufre, où ledit système cyclique contient au moins un atome d'azote de cycle et ne peut pas contenir plus d'un atome d'oxygène de cycle et pas plus d'un atome de soufre de cycle ;
R₃ étant hydrogène, halogène ou C₁-C₄alkyle ;
R₄ étant indépendamment hydrogène, C₁-C₄alkyle ou C₃-C₆cycloalkyle ;
R₅ étant C₁-C₆alkyle, C₁-C₆halogénoalkyle ou C₃-C₆cycloalkyle ;
ou sel, stéréoisomère, énantiomère, forme tautomère ou N-oxyde acceptable sur le plan agrochimique d'un composé de formule I.

2. Composé de formule I selon la revendication 1,
R₂ étant C₁-C₆halogénoalkyle, préférablement -CH₂CF₂CHF₂ ou -CH₂CF₂CF₃ ;
R₆ étant C₁-C₃alkyle, préférablement méthyle ;
Q étant un radical choisi dans le groupe constitué par la formule Qa et Qb,
A étant N ; et
Q₁ étant hydrogène, halogène, C₃-C₆cycloalkyle, C₃-C₆cycloalkyle monosubstitué par cyano, C₁-C₆cyanoalkyle, -N(R₄)₂, -N(R₄)COR₅ ou -N(R₄)CON(R₄)₂, dans tous lesquels R₄ est indépendamment soit hydrogène, soit C₁-C₄alkyle (préférablement hydrogène ou méthyle) et R₅ est C₁-C₆alkyle ou C₃-C₆cycloalkyle (préférablement méthyle ou cyclopropyle), 2-pyridinyloxy, ou 1,2,4-triazol-1-yle ;
préférablement Q₁ étant hydrogène, chlore, brome, cyclopropyle, 1-cyanocyclopropyle, 1-cyano-1-méthyl-éthyle, -NH(CH₃), -N(CH₃)COCH₃, -N(CH₃)CO(cyclopropyle), -N(CH₃)CONH(CH₃), 2-pyridinyloxy ou 1,2,4-triazol-1-yle.

3. Composé de formule I selon la revendication 1,
R₂ étant C₁-C₆halogénoalkyle, préférablement -CH₂CF₂CHF₂ ou -CH₂CF₂CF₃ ;
R₆ étant méthyle ;
Q étant un radical choisi dans le groupe constitué par la formule Qa et Qb,
A étant N ; et
Q₁ étant hydrogène, brome, cyclopropyle, 1-cyanocyclopropyle, 1-cyano-1-méthyl-éthyle, 2-pyridinyloxy ou -N(CH₃)COCH₃ lorsque Q est Qa ; ou
Q₁ étant hydrogène, chlore, -NH(CH₃), -N(CH₃)COCH₃, - N(CH₃)CO (cyclopropyle), -N(CH₃)CONH(CH₃) ou 1,2,4-triazol-1-yle, lorsque Q est Qb.

4. Composé de formule I selon la revendication 1, représenté par les composés de formule (1-2) 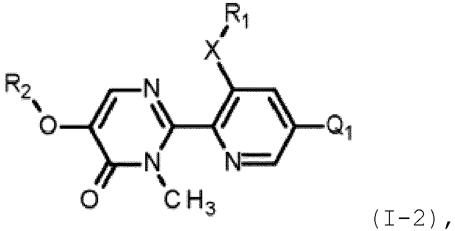
X, R₁ et R₂ étant tels que définis dans la formule I dans la revendication 1, et,
Q₁ étant hydrogène, halogène, C₁-C₆halogénoalkyle, C₃-C₆cycloalkyle, C₃-C₆cycloalkyle monosubstitué par cyano, C₁-C₆cyanoalkyle, C₁-C₆halogénoalcoxy, -N(R₄)₂, -N(R₄)COR₅, ou -N(R₄)CON(R₄)₂, (oxazolidin-2-one)-3-yle ou 2-pyridinyloxy ; ou
Q₁ étant un système cyclique aromatique à cinq à six chaînons lié via un atome de carbone de cycle au cycle pyridinyle substitué par X-R₁, ledit système cyclique étant non substitué ou étant monosubstitué par des substituants choisis dans le groupe constitué par halogène, cyano et C₁-C₄halogénoalkyle ; et ledit système cyclique pouvant contenir 1 ou 2 atomes d'azote de cycle ; ou
Q₁ étant un système cyclique aromatique à cinq chaînons lié via un atome d'azote de cycle au cycle pyridinyle substitué par X-R₁, ledit système cyclique étant non substitué ou étant monosubstitué par des substituants choisis dans le groupe constitué par halogène, cyano et C₁-C₄halogénoalkyle ; et ledit système cyclique contenant 2 ou 3 atomes d'azote de cycle ;
chaque R₄ étant indépendamment préférablement hydrogène ou C₁-C₄alkyle ; et
R₅ étant préférablement C₁-C₆alkyle ou C₃-C₆cycloalkyle.

5. Composé de formule I selon la revendication 1, représenté par les composés de formule (1-3) 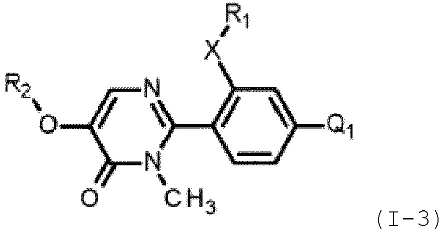
X, R₁ et R₂ étant tels que définis dans la formule I dans la revendication 1, et,
Q₁ étant hydrogène, halogène, C₁-C₆halogénoalkyle, C₃-C₆cycloalkyle, C₃-C₆cycloalkyle monosubstitué par cyano, C₁-C₆cyanoalkyle, C₁-C₆halogénoalcoxy, -N(R₄)₂, -N(R₄)COR₅, ou -N(R₄)CON(R₄)₂, (oxazolidin-2-one)-3-yle ou 2-pyridinyloxy ; ou
Q₁ étant un système cyclique aromatique à cinq à six chaînons lié via un atome de carbone de cycle au cycle phényle substitué par X-R₁, ledit système cyclique étant non substitué ou étant monosubstitué par des substituants choisis dans le groupe constitué par halogène, cyano et C₁-C₄halogénoalkyle ; et ledit système cyclique pouvant contenir 1 ou 2 atomes d'azote de cycle ; ou
Q₁ étant un système cyclique aromatique à cinq chaînons lié via un atome d'azote de cycle au cycle phényle substitué par X-R₁, ledit système cyclique étant non substitué ou étant monosubstitué par des substituants choisis dans le groupe constitué par halogène, cyano et C₁-C₄halogénoalkyle ; et ledit système cyclique contenant 2 ou 3 atomes d'azote de cycle ;
chaque R₄ étant indépendamment préférablement hydrogène ou C₁-C₄alkyle ; et
R₅ étant préférablement C₁-C₆alkyle ou C₃-C₆cycloalkyle.

6. Composé de formule I selon la revendication 1, représenté par les composés de formule (1-4) 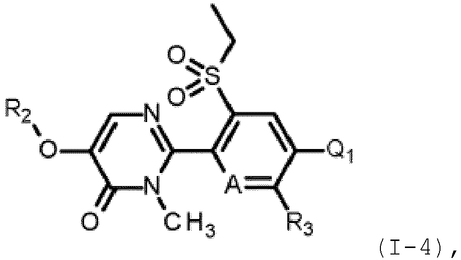
A étant CH ou N, préférablement N ;
R₂ étant C₁-C₆halogénoalkyle, préférablement -CH₂CF₂CHF₂ ou -CH₂CF₂CF₃ ;
R₃ étant hydrogène ou C₁-C₄alkyle, préférablement hydrogène ou méthyle ;
Q₁ étant hydrogène, halogène, C₁-C₆halogénoalkyle, C₃-C₆cycloalkyle, C₃-C₆cycloalkyle monosubstitué par cyano, C₁-C₆cyanoalkyle, C₁-C₆halogénoalcoxy, -N(R₄)₂, -N(R₄)COR₅, ou -N(R₄)CON(R₄)₂, (oxazolidin-2-one)-3-yle ou 2-pyridinyloxy ; ou
Q₁ étant un système cyclique aromatique à cinq à six chaînons lié via un atome de carbone de cycle au cycle qui contient le substituant A, ledit système cyclique étant non substitué ou étant monosubstitué par des substituants choisis dans le groupe constitué par halogène, cyano et C₁-C₄halogénoalkyle ; et ledit système cyclique pouvant contenir 1 ou 2 atomes d'azote de cycle ; ou
Q₁ étant un système cyclique aromatique à cinq chaînons lié via un atome d'azote de cycle au cycle qui contient le substituant A, ledit système cyclique étant non substitué ou étant monosubstitué par des substituants choisis dans le groupe constitué par halogène, cyano et C₁-C₄halogénoalkyle ; et ledit système cyclique contenant 2 ou 3 atomes d'azote de cycle ;
chaque R₄ étant indépendamment hydrogène ou C₁-C₄alkyle, préférablement hydrogène ou méthyle ; et
R₅ étant C₁-C₆alkyle ou C₃-C₆cycloalkyle, préférablement méthyle ou cyclopropyle.

7. Composé de formule I selon la revendication 1, représenté par les composés de formule (1-6) 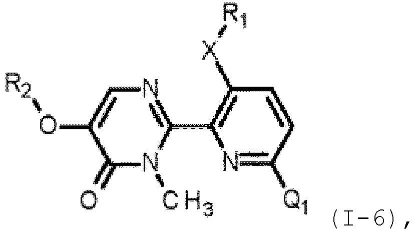
X, R₁ et R₂ étant tels que définis dans la formule I dans la revendication 1, et,
Q₁ étant hydrogène, halogène, C₁-C₆halogénoalkyle, C₃-C₆cycloalkyle, C₃-C₆cycloalkyle monosubstitué par cyano, C₁-C₆cyanoalkyle, C₁-C₆halogénoalcoxy, -N(R₄)₂, -N(R₄)COR₅, ou -N(R₄)CON(R₄)₂, (oxazolidin-2-one)-3-yle ou 2-pyridinyloxy ; ou
Q₁ étant un système cyclique aromatique à cinq à six chaînons lié via un atome de carbone de cycle au cycle pyridinyle substitué par X-R₁, ledit système cyclique étant non substitué ou étant monosubstitué par des substituants choisis dans le groupe constitué par halogène, cyano et C₁-C₄halogénoalkyle ; et ledit système cyclique pouvant contenir 1 ou 2 atomes d'azote ; ou
Q₁ étant un système cyclique aromatique à cinq chaînons lié via un atome d'azote de cycle au cycle pyridinyle substitué par X-R₁, ledit système cyclique étant non substitué ou étant monosubstitué par des substituants choisis dans le groupe constitué par halogène, cyano et C₁-C₄halogénoalkyle ; et ledit système cyclique contenant 2 ou 3 atomes d'azote ;
R₄ étant indépendamment préférablement hydrogène ou C₁-C₄alkyle ; et
R₅ étant préférablement C₁-C₆alkyle ou C₃-C₆cycloalkyle.

8. Composé de formule I selon la revendication 1, représenté par les composés de formule (1-7) 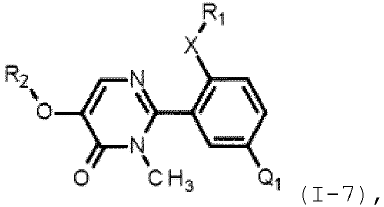
X, R₁ et R₂ dans la formule I dans la revendication 1, et,
Q₁ étant hydrogène, halogène, C₁-C₆halogénoalkyle, C₃-C₆cycloalkyle, C₃-C₆cycloalkyle monosubstitué par cyano, C₁-C₆cyanoalkyle, C₁-C₆halogénoalcoxy, -N(R₄)₂, -N(R₄)COR₅, ou -N(R₄)CON(R₄)₂, (oxazolidin-2-one)-3-yle ou 2-pyridinyloxy ; ou
Q₁ étant un système cyclique aromatique à cinq à six chaînons lié via un atome de carbone de cycle au cycle phényle substitué par X-R₁, ledit système cyclique étant non substitué ou étant monosubstitué par des substituants choisis dans le groupe constitué par halogène, cyano et C₁-C₄halogénoalkyle ; et ledit système cyclique pouvant contenir 1 ou 2 atomes d'azote de cycle ; ou
Q₁ étant un système cyclique aromatique à cinq chaînons lié via un atome d'azote de cycle au cycle phényle substitué par X-R₁, ledit système cyclique étant non substitué ou étant monosubstitué par des substituants choisis dans le groupe constitué par halogène, cyano et C₁-C₄halogénoalkyle ; et ledit système cyclique contenant 2 ou 3 atomes d'azote de cycle ;
R₄ étant indépendamment préférablement hydrogène ou C₁-C₄alkyle ; et
R₅ étant préférablement C₁-C₆alkyle ou C₃-C₆cycloalkyle.

9. Composé de formule I selon la revendication 1, représenté par les composés de formule (1-8) 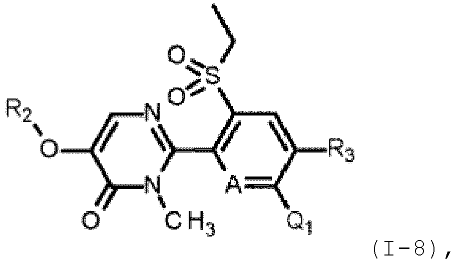
A étant CH ou N, préférablement N ;
R₂ étant C₁-C₆halogénoalkyle, préférablement -CH₂CF₂CHF₂ ou -CH₂CF₂CF₃ ;
R₃ étant hydrogène ou C₁-C₄alkyle, préférablement hydrogène ou méthyle ;
Q₁ étant hydrogène, halogène, C₁-C₆halogénoalkyle, C₃-C₆cycloalkyle, C₃-C₆cycloalkyle monosubstitué par cyano, C₁-C₆cyanoalkyle, C₁-C₆halogénoalcoxy, -N(R₄)₂, -N(R₄)COR₅, ou -N(R₄)CON(R₄)₂, (oxazolidin-2-one)-3-yle ou 2-pyridinyloxy ; ou
Q₁ étant un système cyclique aromatique à cinq à six chaînons lié via un atome de carbone de cycle au cycle qui contient le substituant A, ledit système cyclique étant non substitué ou étant monosubstitué par des substituants choisis dans le groupe constitué par halogène, cyano et C₁-C₄halogénoalkyle ; et ledit système cyclique pouvant contenir 1 ou 2 atomes d'azote de cycle ; ou
Q₁ étant un système cyclique aromatique à cinq chaînons lié via un atome d'azote de cycle au cycle qui contient le substituant A, ledit système cyclique pouvant être monosubstitué par des substituants choisis dans le groupe constitué par halogène, cyano ou C₁-C₄halogénoalkyle ; et ledit système cyclique contenant 2 ou 3 atomes d'azote de cycle ;
R₄ étant indépendamment hydrogène ou C₁-C₄alkyle, préférablement hydrogène ou méthyle ; et
R₅ étant C₁-C₆alkyle ou C₃-C₆cycloalkyle, préférablement méthyle ou cyclopropyle.

10. Composé de formule I selon l'une quelconque des revendications 1 à 7, R₂ étant -CH₂CF₂CHF₂ ou -CH₂CF₂CF₃.

11. Composé de formule I selon l'une quelconque des revendications 1 à 7,
Q₁ étant hydrogène, halogène, trifluorométhyle, cyclopropyle, cyanocyclopropyle, cyanoisopropyle, trifluoroéthoxy, -N(R₄)₂, -N(R₄)COR₅, ou -N(R₄)CON(R₄)₂, dans chacun desquels R₄ est indépendamment soit hydrogène, soit méthyle et R₃ est soit méthyle, soit cyclopropyle, ou Q₁ étant (oxazolidin-2-one)-3-yle, 2-pyridinyloxy, pyrazolyle N-lié qui peut être monosubstitué par chloro, cyano ou trifluorométhyle ; ou Q₁ étant triazolyle N-lié ou pyrimidinyle C-lié.

12. Composé de formule I selon l'une quelconque des revendications 1 à 7,
Q₁ étant hydrogène, chlore, brome, trifluorométhyle, cyclopropyle, 1-cyanocyclopropyle, 1-cyano-1-méthyl-éthyle, 2,2,2-trifluoroéthoxy, -NH(CH₃), -N(CH₃)COCH₃,-N(CH₃)CO(cyclopropyle), -N(H)CONH(CH₃), -N(CH₃)CONH(CH₃), (oxazolidin-2-one)-3-yle, 2-pyridinyloxy, pyrazol-1-yle, 3-chloro-pyrazol-1-yle, 3-cyano-pyrazol-1-yle, 3-trifluorométhyl-pyrazol-1-yle, 1,2,4-triazol-1-yle ou pyrimidin-2-yle.

13. Composé de formule I selon les revendications 1, 6 ou 9,
A étant N ; et
Q₁ étant hydrogène, chlore, brome, cyclopropyle, 1-cyanocyclopropyle, 1-cyano-1-méthyl-éthyle, -NH(CH₃),-N(CH₃)COCH₃, -N(CH₃)CO(cyclopropyle), -N(CH₃)CONH(CH₃), 2-pyridinyloxy ou 1,2,4-triazol-1-yle.

14. Composé de formule I selon la revendication 1, choisi dans le groupe constitué par :
2-(3-éthylsulfanyl-2-pyridinyl)-3-méthyl-5-(2,2,3,3,3-pentafluoropropoxy)pyrimidin-4-one (Composé P1) ;
2-(3-éthylsulfonyl-2-pyridinyl)-3-méthyl-5-(2,2,3,3,3-pentafluoropropoxy)pyrimidin-4-one (Composé P2) ;
2-(5-bromo-3-éthylsulfanyl-2-pyridinyl)-3-méthyl-5-(2,2,3,3,3-pentafluoropropoxy)pyrimidin-4-one (Composé P3) ;
N-[5-éthylsulfanyl-6-[1-méthyl-6-oxo-5-(2,2,3,3,3-pentafluoropropoxy)pyrimidin-2-yl]-3-pyridinyl]-N-méthyl-acétamide (Composé P4) ;
N-[5-éthylsulfonyl-6-[1-méthyl-6-oxo-5-(2,2,3,3,3-pentafluoropropoxy)pyrimidin-2-yl]-3-pyridinyl]-N-méthyl-acétamide (Composé P5) ;
2-(6-chloro-3-éthylsulfanyl-2-pyridinyl)-3-méthyl-5-(2,2,3,3,3-pentafluoropropoxy)pyrimidin-4-one (Composé P6) ;
2-(6-chloro-3-éthylsulfonyl-2-pyridinyl)-3-méthyl-5-(2,2,3,3,3-pentafluoropropoxy)pyrimidin-4-one (Composé P7) ;
2-[3-éthylsulfonyl-6-(méthylamino)-2-pyridinyl]-3-méthyl-5-(2,2,3,3,3-pentafluoropropoxy)pyrimidin-4-one (Composé P8) ;
N-[5-éthylsulfonyl-6-[1-méthyl-6-oxo-5-(2,2,3,3,3-pentafluoropropoxy)pyrimidin-2-yl]-2-pyridinyl]-N-méthyl-acétamide (Composé P9) ;
1-[5-éthylsulfanyl-6-[1-méthyl-6-oxo-5-(2,2,3,3,3-pentafluoropropoxy)pyrimidin-2-yl]-3-pyridinyl]cyclopropanecarbonitrile (Composé P10) ;
1-[5-éthylsulfonyl-6-[1-méthyl-6-oxo-5-(2,2,3,3,3-pentafluoropropoxy)pyrimidin-2-yl]-3-pyridinyl]cyclopropanecarbonitrile (Composé P11) ;
2-[5-éthylsulfanyl-6-[1-méthyl-6-oxo-5-(2,2,3,3,3-pentafluoropropoxy)pyrimidin-2-yl]-3-pyridinyl]-2-méthyl-propanenitrile (Composé P12) ;
2-[5-éthylsulfonyl-6-[1-méthyl-6-oxo-5-(2,2,3,3,3-pentafluoropropoxy)pyrimidin-2-yl]-3-pyridinyl]-2-méthyl-propanenitrile (Composé P13) ;
2-[3-éthylsulfonyl-6-(1,2,4-triazol-1-yl)-2-pyridinyl]-3-méthyl-5-(2,2,3,3,3-pentafluoropropoxy)pyrimidin-4-one (Composé P14) ;
1-[5-éthylsulfonyl-6-[1-méthyl-6-oxo-5-(2,2,3,3,3-pentafluoropropoxy)pyrimidin-2-yl]-2-pyridinyl]-1,3-diméthyl-urée (Composé P15) ;
N-[5-éthylsulfonyl-6-[1-méthyl-6-oxo-5-(2,2,3,3,3-pentafluoropropoxy)pyrimidin-2-yl]-2-pyridinyl]-N-méthyl-cyclopropanecarboxamide (Composé P16) ;
2-(5-cyclopropyl-3-éthylsulfanyl-2-pyridinyl)-3-méthyl-5-(2,2,3,3,3-pentafluoropropoxy)pyrimidin-4-one (Composé P17) ;
2-(5-cyclopropyl-3-éthylsulfonyl-2-pyridinyl)-3-méthyl-5-(2,2,3,3,3-pentafluoropropoxy)pyrimidin-4-one (Composé P18) ;
2-[3-éthylsulfanyl-5-(2-pyridinyloxy)-2-pyridinyl]-3-méthyl-5-(2,2,3,3,3-pentafluoropropoxy)pyrimidin-4-one (Composé P19) ; et
2-[3-éthylsulfonyl-5-(2-pyridinyloxy)-2-pyridinyl]-3-méthyl-5-(2,2,3,3,3-pentafluoropropoxy)pyrimidin-4-one (Composé P20).

15. Composition comprenant une quantité efficace sur le plan insecticide, sur le plan acaricide, sur le plan nématicide, ou sur le plan molluscicide d'un composé de formule (I), ou d'un sel, d'un stéréoisomère, d'un énantiomère, d'une forme tautomère ou d'un N-oxyde acceptable sur le plan agrochimique correspondant(e), tel(le) que défini(e) dans l'une quelconque des revendications 1 à 14 et, éventuellement, un auxiliaire ou diluant.

16. Procédé de lutte contre des insectes, des acariens, des nématodes ou des mollusques, et de régulation de ceux-ci, qui comprend une application sur un organisme nuisible, sur un site d'un organisme nuisible, ou sur un végétal susceptible d'être attaqué par un organisme nuisible, d'une quantité efficace sur le plan insecticide, sur le plan acaricide, sur le plan nématicide, ou sur le plan molluscicide d'un composé de formule (I), ou d'un sel, d'un stéréoisomère, d'un énantiomère, d'une forme tautomère ou d'un N-oxyde acceptable sur le plan agrochimique correspondant(e), tel(le) que défini(e) dans l'une quelconque des revendications 1 à 14 ou d'une composition telle que définie dans la revendication 15 ; à l'exception d'un procédé appliqué au corps humain ou animal.

17. Procédé pour la protection d'un matériel de propagation végétale contre l'attaque par des insectes, des acariens, des nématodes ou des mollusques, qui comprend un traitement du matériel de propagation ou du site où le matériel de propagation est planté, avec une composition selon la revendication 15.

18. Composé de formule V 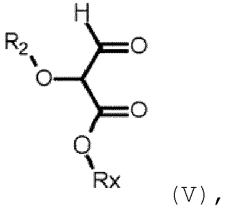
ou forme tautomère correspondante,
R₂ étant C₁-C₆fluoroalkyle, et Rx étant C₁-C₆alkyle.
